(19) Europäisches Patentamt
European Patent Office
Office européen des brevets

(11) Publication number: 0 400 660 A1

(12) # EUROPEAN PATENT APPLICATION

(21) Application number: 90110397.8

(22) Date of filing: 31.05.90

(51) Int. Cl.5: C07D 401/04, C07D 405/04, C07D 401/14, C07D 401/10, C07D 401/12

(30) Priority: 31.05.89 JP 136208/89

(43) Date of publication of application: 05.12.90 Bulletin 90/49

(84) Designated Contracting States: CH DE FR GB IT LI

(71) Applicant: FUJIREBIO INC.
6-7, Shimoochiai 4-chome Shinjuku-ku
Tokyo 161(JP)

(72) Inventor: Ikawa, Hiroshi
26-10, 1-chome, Miyasaka, Setagaya-Ku
Tokyo(JP)
Inventor: Kadoiri, Akiyoshi
1458-5-303 Hazama-cho
Hachioji-shi, Tokyo(JP)
Inventor: Kobayashi, Nobuo
1228, Kotta
Tama-shi, Tokyo(JP)
Inventor: Konagai, Yasuko
13-10 2-chome, Kamitakaido, Suginami-ku
Tokyo(JP)
Inventor: Sekine, Yasuo
23-7, 2-chome, Fujizuka, Kouhoku-ku
Yokohama-shi, Kanagawa-ken(JP)

(74) Representative: Marx, Lothar, Dr. et al
Patentanwälte Schwabe, Sandmair, Marx
Stuntzstrasse 16 Postfach 86 02 45
D-8000 München 80(DE)

(54) **1,4-dihydropyridine derivatives.**

(57) 1,4-Dihydropyridine derivatives of formula (I):

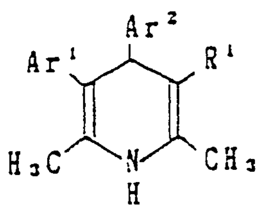

( I )

wherein $Ar^1$ and $Ar^2$ each represent an unsubstituted or substituted aromatic hydrocarbon or aromatic heterocyclic group; and $R^1$ represents $-CO_2R^2$, $-SO_2R^3$, $-COR^4$, $-CON(R^5)_2$, -CN or $-NO_2$ in which $R^2$ is hydrogen, a straight chain, branched chain or cyclic saturated hydrocarbon group, which may have a substituent or a straight chain, branched chain or cyclic unsaturated hydrocarbon group having 2 to 10 carbon atoms, which may have a substituent, $R^3$ is an alkyl group having 1 to 4 carbon atoms, $R^4$ is an alkyl group having 1 to 4 carbon atoms or a phenyl group; and $R^5$ is an alkyl group having 1 to 4 carbon atoms.

EP 0 400 660 A1

# 1,4-DIHYDROPYRIDINE DERIVATIVES

## BACKGROUND OF THE INVENTION

The present invention relates to 1,4-dihydropyridine derivatives having superior vasodilative and platelet aggregation inhibiting activities.

Some of 1,4-dihydropyridine-3,5-dicarboxylate compounds have vasodilative effects based on the calcium entry blocking action and therefore are employed as coronary vasodilators, ameliorants of cerebral circulation, and hypotensive drugs.

Representative examples of such compounds are 1,4-dihyro-2,6-dimethyl-4-(2-nitrophenyl)pyridine-3,5-dicarbo xylic acid dimethyl ester (generally referred to as "NIFEDIPINE"; U.S. Patent No. 3,644,627) and 1,4-dihydro-2,6-dimethyl-4-(3-nitrophenyl)pyridine-3,5-dicarboxylic acid-3-[2-(N-benzyl-N-methylamino)-ethyl]ester-5-methyl ester hydrochloride (generally referred to as "NICARDIPINE"; Japanese Patent Publication 55-45075).

In addition, 1,4-dihydropyridine compounds having both vasodilative based on the calcium entry blocking action and platelet aggregation inhibitory activities, are now being developed as disclosed, for example, in Japanese Laid-Open Patent Applications Nos. 56-140989, 60-215684, 61-10576, 61-5076, 61-197578, 60-226876, 61-47477, 61-212581, and 62-187468.

1,4-dihydropyridine compounds such as NIFEDIPINE and NICARDIPINE have high vasolidative activity based on the calcium entry blocking action, but are not satisfactory as remedy for atherosclerosis. Therefore, researches are being made for obtaining 1,4-dihydropyridine derivatives having not only vasodilating activity, but also platelet aggregation inhibitory activity, capable of inhibiting platelet aggregation and preventing and curing atherosclerosis, which can be used as remedies for circulatory system. However, satisfactory remedies having the above-mentioned activities have not been found yet.

## SUMMARY OF THE INVENTION

It is therefore an object of the present invention to provide medicines capable of preventing and curing diseases of circulatory systems, which are particularly useful, for example, as hypotensive drugs, vasodilators, ameliorants of cerebral circulation, and anti-thrombotic drugs.

This object of the present invention is achieved by 1,4-dihydropyridine derivatives of formula (I):

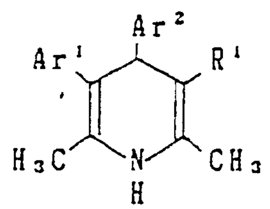

........ ( I )

wherein $Ar^1$ and $Ar^2$ each represent an aromatic hydrocarbon group which may have a substituent, or an aromatic heterocyclic group which may have a substituent; $R^1$ represents a group selected from the group consisting of $-CO_2R^2$, $-SO_2R^3$, $-COR^4$, $-CON(R^5)_2$, -CN or $-NO_2$; $R^2$ represents (i) hydrogen, (ii) a straight chain, branched chain or cyclic saturated hydrocarbon group having 2 to 10 carbon atoms, which may have a substituent selected from the group consisting of an alkyl group having 1 to 4 carbon atoms, an alkoxyl group having 1 to 4 carbon atoms, a phenoxy group which may have a substituent, a phenylthio group which may have a substituent, a substituted amino group, a cyclic amino group which may have a substituent, a phenyl group which may have a substituent, an aromatic heterocyclic group which may have a substituent, and a trihalomethyl group, or (iii) a straight chain, branched chain or cyclic unsaturated hydrocarbon group having 2 to 10 carbon atoms, which may have a substituent selected from the group consisting of an alkyl group having 1 to 4 carbon atoms, an alkoxyl group having 1 to 4 carbon atoms, a phenoxy group which may have a substituent, a phenylthio group which may have a substituent, a substituted amino group, a cyclic amino group which may have a substituent, a phenyl group which may have a substituent, an aromatic heterocyclic group which may have a substituent, and a trihalomethyl group; $R^3$ represents an alkyl group having 1 to 4 carbon atoms; $R^4$ represens an alkyl group having 1 to 4 carbon

atoms or a phenyl group; and $R^5$ represents an alkyl group having 1 to 4 carbon atoms.

The present invention is based on the discovery that the above 1,4-dihydropyridine derivatives have superior vasodilative based on the calcium entry blocking action and platelet aggregation inhibiting activities.

## DETAILED DESCRIPTION OF THE PREFERRED EMBODIMENTS

In the 1,4-dihydropyridine derivatives having the above general formula (I), $Ar^1$ and $Ar^2$ may be an aromatic hydrocarbon group, which may have a substituent. Examples of the aromatic hydrocarbon group represented by either $Ar^1$ or $Ar^2$ are phenyl group and napthyl group, which may have a substituent.

$Ar^1$ and $Ar^2$ may also be an aromatic heterocyclic group, which may have a substituent. Examples of the aromatic heterocyclic group represented by $Ar^1$ or $Ar^2$ include pyridyl group, quinolyl group, isoquinolyl group, furyl group, thienyl group, benzoxazolyl group, benzothiazolyl group, pyridazinyl group, pyrazinyl group, pyrimidinyl group, indolyl group, benzoxadiazolyl group, benzothiadiazolyl group, which may have a substituent.

Examples of the substituent of the aromatic hydrocarbon group and the aromatic heterocyclic carbon group represented by either $Ar^1$ or $Ar^2$ include a halogen, cyano group, nitro group, trifluoromethyl group, trichloromethyl group, azide group, amide group, an alkyl group having 1 to 4 carbon atoms, an alkoxyl group having 1 to 4 carbon atoms, a benzoyl group, an alkylthio group having 1 to 4 carbon atoms, phenylthio group, phenoxy group, a lower alkoxycarbonyl group, a lower acyl group, benzyloxy group, hydroxy group, and cinnamyloxy group.

Specific examples of the group represented by either $Ar^1$ or $Ar^2$ include phenyl group, 2-nitrophenyl group, 3-nitro-phenyl group, 4-nitrophenyl group, 2-cyanophenyl group, 2-bromophenyl group, 3-bromophenyl group, 4-bromo phenyl group, 2-chlorophenyl group, 3-chlorophenyl group, 4-chloro-phenyl group, 2-iodophenyl group, 3-iodophenyl group, 4-iodophenyl group, 3-cyanophenyl group, 4-cyanophenyl group, 2,3-dichlorophenyl group, 2,6-dichlorophenyl group, 3,5-dichlorophenyl group, 2-furyl group, furyl group, thienyl group, 3-thienyl group, 1-naphthyl group, naphthyl group, 3-azidophenyl group, 2-trifluoromethylphenyl group, 3-trifluoromethylphenyl group, 4-trifluoromethylphenyl group, 2-methylphenyl group, 3-methylphenyl group, 4-methylphenyl group, 2-hydroxyphenyl group, 3-hydroxyphenyl group, 4-hydroxyphenyl group, 2-benzyloxyphenyl group, 3-benzyloxyphenyl group, 2-cinnamyloxyphenyl group, 3-cinnamyloxyphenyl group, 3-benzoylphenyl group, 2-methoxyphenyl group, 3-methoxyphenyl group, 4-methoxyphenyl group, 4-methylthiophenyl group, 3-trichloromethylphenyl group, 2-pyridyl group, pyridyl group, 4-pyridyl group, quinolyl group, 3-quinolyl group, 4-quinolyl group, 1-isoquinolyl group, quinoxyalyl group, 2-chloropyridyl group, 6-chloropyridyl group, 2-methylpyridyl group, 6-methylpyridyl group, 2-methoxypyridyl group, 6-methoxypyridyl group, 5-bromopyridyl group, 2-amionpyridyl group, 2-mercaptopyridyl group, pyridazinyl group, 4-pyridazinyl group, pyrazinyl group, pyrimidinyl group, 5-pyrimidyl group, 7-benzoxazolyl group, 7-benzothiazolyl group, 3-benzoxadiazolyl group, 3-benzothiazolyl group, 2-indolyl group, 3-indolyl group, and 5-indolyl group.

Further in the formula (I), $R^1$ is $-CO_2R^2$, $-SO_2R^3$, $-COR^4$, $-CON(R^5)_2$, -CN or $-NO_2$.

$R^2$ in $-CO_2R^2$ represents (i) hydrogen, (ii) a straight chain, branched chain or cyclic saturated hydrocarbon group having 2 to 10 carbon atoms, which may have a substituent selected from the group consisting of an alkyl group having 1 to 4 carbon atoms, an alkoxyl group having 1 to 4 carbon atoms, a phenoxy group which may have a substituent, a phenylthio group which may have a substituent, a substituted amino group, a cyclic amino group which may have a substituent, a phenyl group which may have a substituent, an aromatic heterocyclic group which may have a substituent, and a trihalomethyl group, or (iii) a straight chain, branched chain or cyclic unsaturated hydrocarbon group having 2 to 10 carbon atoms, which may have a substituent selected from the group consisting of an alkyl group having 1 to 4 carbon atoms, an alkoxyl group having 1 to 4 carbon atoms, a phenoxy group which may have a substituent, a phenylthio group which may have a substituent, a substituted amino group, a cyclic amino group which may have a substituent, a phenyl group which may have a substituent, an aromatic heterocyclic group which may have a substituent, and a trihalomethyl group.

Examples of the straight chain or branched saturated hydrocarbon gruop represented by $R^2$ include methyl group, ethyl group, n-propyl group, n-butyl group, n-pentyl group, n-hexyl group, n-heptyl group, n-octyl group, n-nonyl group, n-decyl group, isoproyl group, and isobutyl group, which may have a substituent.

Examples of the cyclic hydrocarbon group represented by $R^2$ include cyclopentyl group and cyclohexyl group, which may have a substituent.

Examples of the unsaturated hydrocarbon group represented by $R^2$ include propenyl group, 2-butenyl group, 3-butenyl group, 2-pentenyl group, 2,4-hexadienyl group, 2,4-hexadiynyl group, hexa-4-en-2-yne, which may have a substituent.

Examples of the substituent of $R^2$ includean alkoxyl group having 1 to 6 carbon atoms, such as methoxy group, ethoxy group and propoxy group; an unsubstituted or substituted phenoxy group; an unsubstituted or substituted phenylthio group; a substituted amino group, such as dimethyl amino group, diethyl amino group, and N-benzyl-N-methylamino group; cyclic amino group, such as an unsubstituted or substituted piperazinyl group and piperidinyl group; an unsubstituted or substituted phenyl group; an unsubstituted or substituted aromatic heterocyclic group, such as pyridyl group, quinolyl group, isoquinolyl group, furyl group, thienyl group, benzoxazolyl group, benzothiazolyl group, pyridazinyl group, pyrazinyl group, pyrimidinyl group, indolyl group, benzoxadiazolyl group, and benzothiadiazolyl group; and a trihalomethyl group.

Specific examples of $R^2$ include hydrogen, methyl group, ethyl group, n-propyl group, n-butyl group, n-pentyl group, n-hexyl group, n-heptyl group, n-octyl group, n-nonyl group, n-decyl group, isopropyl group, isobutyl group, cyclopentyl group, cyclohexyl group, propenyl group, 2-propyne-1-yl group, (E)-2-butene-1-yl group, (E)-3-butene-1-yl group, (E)-2-pentene-1-yl group, (2E,4E)-2,4-hexadienyl group, 2,4-hexadiynyl group, (E)-hexa-4-en-2-yne group, (E)-3-phenyl-2-propene-1-yl group, (Z)-3-phenyl-2-propene-1-yl group, 3-phenyl-2-propyne-1-yl group, (2E,4E)-5-phenyl-2,4-pentadiene-1-yl group, 5-phenyl-penta-2,4-diyne-1-yl group, (E)-5-phenyl-penta-2-en-4-yne-1-yl group, (E)-3-[3-(1-imidazolylmethyl)phenyl]-2-propene-1-yl group, (E)-3-[2-(1-imidazolylmethyl)phenyl]-2-propene-1-yl group, (E)-3-[4-(1-imidazolylmethyl)phenyl]-2-propene-1-yl group, (Z)-3-[4-(1-imidazolylmethyl)phenyl]-2-propene-1-yl group, (E)-3-[6-(1-imidazolylmethyl)pyridine-2-yl]-2-propene-1-yl group, (E)-3-[5-(1-imidazolylmethyl)furan-2-yl]-2-propene-1-yl group, (E)-3-[5-(1-imidazolylmethyl)thiophene-2-yl]-2- propene-1-yl group, (E)-3-phenyl-1-methyl-2-propene-1-yl group, (E)-1-fluoro-3-phenyl-2-propene-1-yl group, 2-methoxyethyl group, 3-methoxypropyl group, 3-ethoxypropyl group, 2-phenoxyethyl group, 2-phenylthioethyl group, 2-(N-methylamino)ethyl group, 2-(N,N-dimethylamino)ethyl group, 2-(N-methyl-N-phenylamino)ethyl group, 2-(N,N-diethylamino)ethyl group, 2-(N-benzyl-N-methylamino)ethyl group, 2-(1-piperazinyl)ethyl group, 4-(1-piperazinyl)butyl group, 6-(1-piperazinyl)hexyl group, 2-(4-piperidinyl)ethyl group, 2-(4-phenylpiperazine-1-yl)ethyl group, 3-(4-phenylpiperazine-1-yl)propyl group, 4-(4-phenyl-piperazine-1-yl)butyl group, 6-(4-phenylpiperazine-1-yl)hexyl group, 2-(4-phenylpiperidine-1-yl)ethyl group, 3-(4-phenylpiperidine-1-yl)propyl group, 4-(4-phenylpiperidine-1-yl)butyl group, 3-(4-phenyliperidine-1-yl)butyl group, 6-(4-phenylpiperidine-1-yl)exyl group, 2-[4-(diphenylmethyl)-piperazine-1-yl]ethyl group, 3-[4-(diphenylmethyl)-piperazine-1-yl)propyl group, 4-[4-(diphenylmethyl)-piperazine-1-yl)]butyl group, 6-[4-(diphenylmethyl)-piperazine-1-yl)]hexyl group, 2-morpholinoethyl group, N-benzylpyrrolidine-3-yl group, N-benzylpiperidine-3-yl group, 2-(1,2,3,4-tetrahydro isoquinoline-2-yl)ethyl group, 2,2,2-trifluoroethyl group, 2-(3,7-dihydro-3,7-dimethyl-1H-purine-2,6-dione-1-yl)ethyl group, and 2-(1,2,3,6-tetrahydro-1,3-dimethyl-2,6-dioxo-7H-purine-7-yl)ethyl group.

$R^3$ in $-SO_2R^3$ is an alkyl group having 1 to 4 carbon atoms. Specific examples of the alkyl group represented by $R^3$ include methyl group, ethyl group, n-propyl group, and isopropyl group.

$R^4$ in $-COR^4$ is a phenyl group or an alkyl group having 1 to 4 carbon atoms. Specific examples of the alkyl group represented by $R^4$ include methyl group, ethyl group, n-propyl group, and isopropyl group.

$R^5$ in $-CON(R^5)_2$ is an alkyl group having 1 to 4 carbon atoms. Specific examples of the alkyl group represented by $R^5$ is selected from the group consisting of methyl group, ethyl group, n-propyl group, and isopropyl group.

Specific examples of the 1,4-diphydropyridine derivatives having formula (I) according to the present invention are as follows:

methyl 1,4-dihydro-2,6-dimethyl-4-(3-nitrophenyl)-5-(2-pyridyl)pyridine-3-carboxylate,
methyl 1,4-dihydro-2,6-dimethyl-4-(3-nitrophenyl)-5-pyridylpyridine-3-carboxylate,
methyl 1,4-dihydro-2,6-dimethyl-4-(3-nitrophenyl)-5-(4-pyridyl)pyridine-3-carboxylate,
methyl 1,4-dihydro-2,6-dimethyl-4-(3-nitrophenyl)-5- pyrazinylpyridine-3-carboxylate,
methyl 1,4-dihydro-2,6-dimethyl-4-(3-nitrophenyl)-5-pyridazinylpyridine-3-carboxylate,
methyl 1,4-dihydro-2,6-dimethyl-4-(3-nitrophenyl)-5-(4-pyridazinyl)pyridine-3-carboxylate,
methyl 1,4-dihydro-2,6-dimethyl-4-(3-nitrophenyl)-5-pyrimidylpyridine-3-carboxylate,
methyl 1,4-dihydro-2,6-dimethyl-4-(3-nitrophenyl)-5-(5-pyrimidinyl) pyridine-3-carboxylate,
methyl 1,4-dihydro-2,6-dimethyl-4-(3-nitrophenyl)-5-quinolylpyridine-3-carboxylate,
methyl 1,4-dihydro-2,6-dimethyl-4-(3-nitrophenyl)-5-(3-quinolyl)pyridine-3-carboxylate,
methyl 1,4-dihydro-2,6-dimethyl-4-(3-nitrophenyl)-5-(4-quinolyl)pyridine-3-carboxylate,
methyl 1,4-dihydro-2,6-dimethyl-5-(1-isoquinolyl)-4-(4-nitrophenyl)pyridine-3-carboxylate,
methyl 1,4-dihydro-2,6-dimethyl-4-(3-nitrophenyl)-5-quinoxalylpyridine-3-carboxylate,

methyl 1,4-dihydro-2,6-dimethyl-5-(2-methoxyphenyl)-4-(3-nitrophenyl)pyridine-3-carboxylate,
methyl 1,4-dihydro-2,6-dimethyl-5-(2-fluorophenyl)-4-(3-nitrophenyl)pyridine-3-carboxylate,
methyl 1,4-dihydro-2,6-dimethyl-4-(3-nitrophenyl)-5-thienylpyridine-3-carboxylate,
methyl 1,4-dihydro-2,6-dimethyl-5-(2-furyl)-4-(3-nitrophenyl)pyridine-3-carboxylate,
methyl 1,4-dihydro-2,6-dimethyl-5-(2-methylpyridyl)-4-(3-nitrophenyl)pyridine-3-carboxylate,
methyl 1,4-dihydro-2,6-dimethyl-5-(6-methylpyridyl)-4-(3-nitrophenyl)pyridine-3-carboxylate,
methyl 1,4-dihydro-2,6-dimethyl-5-(2-methoxypyridyl)-4-(3-nitrophenyl)pyridine-3-carboxylate,
methyl 1,4-dihydro-2,6-dimethyl-5-(6-methoxypyridyl)-4-(3-nitrophenyl)pyridine-3-carboxylate,
methyl 1,4-dihydro-2,6-dimethyl-5-(2-mercaptopyridyl)-4-(3-nitrophenyl pyridine-3-carboxylate,
methyl 5-(5-bromopyridyl)-1,4-dihydro-2,6-dimethyl-4-(3-nitrophenyl)pyridine-3-carboxylate,
methyl 5-(2-aminopyridyl)-1,4-dihydro-2,6-dimethyl-4-(3-nitrophenyl)pyridine-3-carboxylate,
methyl 5-(2-chloropyridyl)-1,4-dihydro-2,6-dimethyl-4-(3-nitrophenyl)pyridine-3-carboxylate,
methyl 5-(6-chloropyridyl)-1,4-dihydro-2,6-dimethyl-4-(3-nitrophenyl)pyridine-3-carboxylate,
methyl 4-(7-benzoxazolyl)-1,4-dihydro-2,6-dimethyl-5-pyridylpyridine-3-carboxylate,
methyl 4-(3-benzoxazolyl)-1,4-dihydro-2,6-dimethyl-5-pyridylpyridine-3-carboxylate,
methyl 4-(3-benzothiazolyl)-1,4-dihydro-2,6-dimethyl-5-pyridylpyridine-3-carboxylate,
methyl 1,4-dihydro-2,6-dimethyl-4-(2-indolyl)-5-pyridylpyridine-3-carboxylate,
methyl 1,4-dihydro-2,6-dimethyl-4-(3-indolyl)-5-pyridylpyridine-3-carboxylate,
methyl 1,4-dihydro-2,6-dimethyl-4-(5-indolyl)-5-pyridylpyridine-3-carboxylate,
methyl 4-(2-cyanophenyl)-1,4-dihydro-2,6-dimethyl-5-pyridylpridine-3-carboxylate
methyl 1,4-dihydro-2,6-dimethyl-5-pyridyl-4-(2-trifluorophenyl)pyridine-3-carboxylate,
methyl 1,4-dihydro-2,6-dimethyl-5-pyridyl-4-(3-trifluorophenyl)pyridine-3-carboxylate,
methyl 1,4-dihydro-2,6-dimethyl-5-pyridyl-4-(4-trifluorophenyl)pyridine-3-carboxylate,
methyl 4-(2-bromophenyl)-1,4-dihydro-2,6-dimethyl-5- pyridylpyridine-3-carboxylate,
methyl 4-(3-bromophenyl)-1,4-dihydro-2,6-dimethyl-5-pyridylpyridine-3-carboxylate,
methyl 4-(4-bromophenyl)-1,4-dihydro-2,6-dimethyl-5-pyridylpyridine-3-carboxylate,
methyl 4-(2-chlorophenyl)-1,4-dihydro-2,6-dimethyl-5-pyridylpyridine-3-carboxylate,
methyl 4-(3-chlorophenyl)-1,4-dihydro-2,6-dimethyl-5-pyridylpyridine-3-carboxylate,
methyl 4-(4-chlorophenyl)-1,4-dihydro-2,6-dimethyl-5-pyridylpyridine-3-carboxylate,
methyl 4-(2,3-dichlorophenyl)-1,4-dihydro-2,6-dimethyl-5-pyridylpyridine-3-carboxylate,
methyl 4-(2,6-dichlorophenyl)-1,4-dihydro-2,6-dimethyl-5-pyridylpyridine-3-carboxylate,
methyl 4-(3,5-dichlorophenyl)-1,4-dihydro-2,6-dimethyl-5-pyridylpyridine-3-carboxylate,
methyl 1,4-dihydro-2,6-dimethyl-4-(2-furyl)-5-pyridylpyridine-3-carboxylate,
methyl 1,4-dihydro-2,6-dimethyl-4-furyl-5-pyridylpyridine-3-carboxylate,
methyl 1,4-dihydro-2,6-dimethyl-5-pyridyl-4-thienylpyridine-3-carboxylate,
methyl 1,4-dihydro-2,6-dimethyl-5-pyridyl-4-(3-thienyl)pyridine-3-carboxylate,
methyl 1,4-dihydro-2,6-dimethylpyridine-4,5-dipyridyl-3-carboxylate,
methyl 1,4-dihydro-2,6-dimethyl-5-pyridyl-4-(4-quinolyl)pyridine-3-carboxylate,
3-cyano-1,4-dihydro-2,6-dimethyl-4-(3-nitrophenyl)-5-pyridylpyridine,
3-benzoyl-1,4-dihydro-2,6-dimethyl-4-3-nitrophenyl)-5-pyridylpyridine,
3-acetyl-1,4-dihydro-2,6-dimethyl-4-(3-nitrophenyl)-5-pyridylpyridine
methyl 4-(3-azidophenyl)-1,4-dihydro-2,6-dimethyl-5-pyridylpyridine-3-carboxylate,
methyl 1,4-dihydro-2,6-dimethyl-4-(1-naphthyl)-5-pyridylpyridine-3-carboxylate,
methyl 1,4-dihydro-2,6-dimethyl-4-naphthyl-5-pyridylpyridine-3-carboxylate,
methyl 1,4-dihydro-2,6-dimethyl-4-(2-iodophenyl)-5-pyridylpyridine-3-carboxylate,
methyl 1,4-dihydro-2,6-dimethyl-4-(3-iodophenyl)-5-pyridylpyridine-3-carboxylate,
methyl 1,4-dihydro-2,6-dimethyl-4-(4-iodophenyl)-5-pyridylpyridine-3-carboxylate,
methyl 1,4-dihydro-2,6-dimethyl-4-(2-methylphenyl)-5-pyridylpyridine-3-carboxylate,
methyl 1,4-dihydro-2,6-dimethyl-4-(3-methylphenyl)-5-pyridylpyridine-3-carboxylate,
methyl 1,4-dihydro-2,6-dimethyl-4-(4-methylphenyl)-5-pyridylpyridine-3-carboxylate,
methyl 1,4-dihydro-2,6-dimethyl-4-(2-methoxylphenyl)-5-pyridylpyridine-3-carboxylate,
methyl 1,4-dihydro-2,6-dimethyl-4-(3-methoxylphenyl)-5-pyridylpyridine-3-carboxylate,
methyl 1,4-dihydro-2,6-dimethyl-4-(4-methoxylphenyl)-5-pyridylpyridine-3-carboxylate,
methyl 1,4-dihydro-2,6-dimethyl-4-(4-methylthiophenyl)-5-pyridylpyridine-3-carboxylate,
ethyl 1,4-dihydro-2,6-dimethyl-4-(3-nitrophenyl)-5-pyridylpyridine-3-carboxylate,
isopropyl 1,4-dihydro-2,6-dimethyl-4-(3-nitrophenyl)-5-pyridylpyridine-3-carboxylate,
n-hexyl 1,4-dihydro-2,6-dimethyl-4-(3-nitrophenyl)-5- pyridylpyridine-3-carboxylate,
cyclohexyl 1,4-dihydro-2,6-dimethyl-4-(3-nitrophenyl)-5-pyridylpyridine-3-carboxylate,

2-methoxyethyl 1,4-dihydro-2,6-dimethyl-4-(3-nitrophenyl)-5-pyridylpyridine-3-carboxylate,
2-phenoxyethyl 1,4-dihydro-2,6-dimethyl-4-(3-nitrophenyl)-5-pyridylpyridine-3-carboxylate,
2-phenoxyethyl 1,4-dihydro-2,6-dimethyl-4-(3-nitrophenyl)-5-(4-pyridyl)pyridine-3-carboxylate,
2-(phenylthio)ethyl 1,4-dihydro-2,6-dimethyl-4-(3-nitrophenyl)-5-pyridylpyridine-3-carboxylate,
2-pentene-1-ol 1,4-dihydro-2,6-dimethyl-4-(3-nitrophenyl)-5-(4-pyridyl)pyridine-3-carboxylate,
2-propyne-1-yl 1,4-dihydro-2,6-dimethyl-4-(3-nitrophenyl)-5-pyridylpyridine-3-carboxylate,
(2E,4E)-2,4-hexadiene-1-yl 1,4-dihydro-2,6-dimethyl-4-(3-nitrophenyl)-5-pyridylpyridine-3-carboxylate,
2,4-hexadiyne-1-yl 1,4-dihydro-2,6-dimethyl-4-(3-nitrophenyl)-5-pyridylpyridine-3-carboxylate,
(E)-3-phenyl-2-propene-1-yl 1,4-dihydro-2,6-dimethyl-4-(3-nitrophenyl)-5-pyridylpyridine-3-carboxylate,
(E)-3-phenyl-2-propene-1-yl 1,4-dihydro-2,6-dimethyl-4-(3-nitrophenyl)-5-(4-pyridyl)pyridylpyridine-3-carboxylate,
(Z)-3-phenyl-2-propene-1-yl 1,4-dihydro-2,6-dimethyl-4-(3-nitrophenyl-5-pyridylpyridine-3-carboxylate,
(2E,4E)-5-phenyl-2,4-pentadiene-1-yl 1,4-dihydro-2,6-dimethyl-4-(3-nitrophenyl)-5-pyridylpyridine-3-carboxylate,
5-phenyl-penta-2,4-diyne-1-yl 1,4-dihydro-2,6-dimethyl-4-(3-nitrophenyl)-5-pyridylpyridine-3-carboxylate,
(E)-5-phenyl-penta-2-ene-4-yne-1-yl 1,4-dihydro-2,6-dimethyl-4-(3-nitrophenyl)-5-pyridylpyridine-3-carboxylate,
(E)-3-[4-(1-imidazolylmethyl)phenyl-2-propene-1-yl 1,4-dihydro-2,6-dimethyl-4-(3-nitrophenyl)-5-pyridylpyridine-3-carboxylate
(E)-3-[4-(1-imidazolylmethyl)phenyl-2-propene-1-yl 1,4-dihydro-2,6-dimethyl-5-(2-furyl)-4-(3-nitrophenyl)-pyridine-3-carboxylate,
(Z)-3-[4-(1-imidazolylmethyl)phenyl]-2-propene-1-yl 1,4-dihydro-2,6-dimethyl-4-(3-nitrophenyl)-5-pyridylpyridine-3-carboxylate,
(E)-3-[6-(1-imidazolylmethyl)pyridine-2-yl]-2-propene-1-yl 1,4-dihydro-2,6-dimethyl-4-(3-nitrophenyl)-5-pyridylpyridine-3-carboxylate,
(E)-3-[5-(1-imidazolylmethyl)furan-2-yl]-2-propene-1-yl 1,4-dihydro-2,6-dimethyl-4-(3-nitrophenyl)-5-pyridylpyridine-3-carboxylate,
(E)-3-[5-(1-imidazolylmethyl)thiophene-2-yl]-2-propene-1-yl 1,4-dihydro-2,6-dimethyl-4-(3-nitrophenyl)-5-pyridylpyridine-3-carboxylate,
2-(N-benzyl-N-methylamino)ethyl 1,4-dihydro-2,6-dimethyl-4-(3-nitrophenyl)-5-pyridylpyridine-3-carboxylate,
2-(N-benzyl-N-methylamino)ethyl 1,4-dihydro-2,6-dimethyl-4-(3-nitrophenyl)-5-(4-pyridyl)pyridine-3-carboxylate,
2-(4-phenylpiperazine-1-yl)ethyl 1,4-dihydro-2,6-dimethyl-4-(3-nitrophenyl)-5-pyridylpyridine-3-carboxylate,
2-(4-phenylpiperazine-1-yl)ethyl 1,4-dihydro-2,6-dimethyl-4-(3-nitrophenyl-5-(4-pyridyl)pyridine-3-carboxylate,
2-(4-phenylpiperidine-1-yl)ethyl 1,4-dihydro-2,6-dimethyl-4-(3-nitrophenyl)-5-pyridylpyridine-3-carboxylate,
2[4-diphenylmethyl)piperazine-1-yl]ethyl 1,4-dihydro-2,6-dimethyl-4-(3-nitrophenyl)-5-pyridylpyridine-3-carboxylate
2-(1,2,3,4-tetrahydroisoquinoline-2-yl)ethyl 1,4-dihydro-2,6-dimethyl-4-(3-nitrophenyl)-5-pyridylpyridine-3-carboxylate,
2,2,2-trifluoroethyl 1,4-dihydro-2,6-dimethyl-4-(3-nitrophenyl)-5-pyridylpyridine-3-carboxylate,
2-(3,7-dihydro-3,7-dimethyl-1H-purine-2,6-dione-1-yl)ethyl 1,4-dihydro-2,6-dimethyl-4-(3-nitrophenyl)-5-pyridylpyridine-3-carboxylate,
2-(1,2,3,6-tetrahydro-1,3-dimethyl-2,6-dioxo-7H-purine-7-yl)ethyl 1,4-dihydro-2,6-dimethyl-4-(3-nitrophenyl)-5-pyridylpyridine-3-carboxylate,
3-cyano-1,4-dihydro-2,6-dimethyl-4-(3-nitrophenyl)-5-(2-pyridyl)pyridine,
N,N-diethyl-1,4-dihydro-2,6-dimethyl-4-(3-nitrophenyl)-5-(2-pyridyl)pyridine-3-carboxamide,
3-benzoyl-1,4-dihydro-2,6-dimethyl-4-(3-nitrophenyl)-5-(2-pyridyl)pyridine,
3-acetyl-1,4-dihydro-2,6-dimethyl-4-(3-nitrophenyl)-5-(2-pyridyl pyridine,
1,4-dihydro-2,6-dimethyl-3-nitro-4-(3-nitrophenyl)-5-(2-pyridyl)pyridine,
methyl 1,4-dihydro-2,6-dimethyl-4-(3-nitrophenyl)-5-(2-pyridyl)pyridine-3-sulfinate, and
2,2,3-trifluoroethyl 1,4-dihydro-2,6-dimethyl-4-(3-nitrophenyl)-5-(2-pyridyl) pyridine-3-carboxylate.

1,4-dihydropryridine derivatives having formula (I) according to the present invention can be prepared by either Process 1 or Process 2 as shown below:

[Process 1]

Compounds having formula (I-1):

$CH(Ar^2)=C(Ar^1)-C(CH_3)=O$ (I-1)

are allowed to react with compounds having formula (I-2):

$CH_3-C(NH_2)=CHR^1$ (I-2)

wherein $R^1$, $Ar^1$ and $Ar^2$ are the same as those defined previously in formula (I).

Examples of the compounds having formula (I-1) are as follows:

4-(2-nitrophenyl)-3-pyridyl-3-butene-2-one,
4-(3-nitrophenyl)-3-pyridyl-3-butene-2-one,
4-(4-nitrophenyl)-3-pyridyl-3-butene-2-one,
4-(2-nitrophenyl)-3-(2-pyridyl)-3-butene-2-one,
4-(3-nitrophenyl)-3-(2-pyridyl)-3-butene-2-one,
4-(4-nitrophenyl)-3-(2-pyridyl)-3-butene-2-one,
4-(2-nitrophenyl)-3-(4-pyridyl)-3-butene-2-one,
4-(3-nitrophenyl)-3-(4-pyridyl)-3-butene-2-one,
4-(4-nitrophenyl)-3-(4-pyridyl)-3-butene-2-one,
4-(2-cyanophenyl)-3-pyridyl-3-butene-2-one,
4-(3-cyanophenyl)-3-pyridyl-3-butene-2-one,
4-(4-cyanophenyl)-3-pyridyl-3-butene-2-one,
4-(2,3-dichlorophenyl)-3-pyridyl-3-butene-2-one,
4-(3,5-dichlorophenyl)-3-pyridyl-3-butene-2-one,
4-(2,6-dichlorophenyl)-3-pyridyl-3-butene-2-one,
3-pyridyl-4-(2-trifluoromethylphenyl)-3-butene-2-one,
3-pyridyl-4-(3-trifluoromethylphenyl)-3-butene-2-one,
3-pyridyl-4-(4-trifluoromethylphenyl)-3-butene-2-one,
4-(2-furyl)-3-pyridyl-3-butene-2-one,
4-furyl-3-pyridyl-3-butene-2-one,
3-pyridyl-4-thienyl-3-butene-2-one,
3-pyridyl-4-(3-thienyl)-3-butene-2-one,
3-pyridyl-4-quinolyl-3-butene-2-one,
3-pyridyl-4-(3-quinolyl)-3-butene-2-one,
3-pyridyl-4-(4-quinolyl)-3-butene-2-one,
4-(2-methoxyphenyl)-3-(2-pyridyl)-3-butene-2-one,
4-(3-methoxyphenyl)-3-(2-pyridyl)-3-butene-2-one,
4-(4-methoxyphenyl)-3-(2-pyridyl)-3-butene-2-one,
4-(3-nitrophenyl)-3-pyrazinyl-3-butene-2-one,
4-(3-nitrophenyl)-3-pyrimidinyl-3-butene-2-one,
4-(3-nitrophenyl)-3-(5-pyrimidinyl)-3-butene-2-one,
4-(3-nitrophenyl)-3-(3-quinolyl)-3-butene-2-one,
4-(3-nitrophenyl)-3-(4-quinolyl)-3-butene-2-one,
3-(1-isoquinolyl)-4-(3-nitrophenyl)-3-butene-2-one,
4-(2-pyridyl)-3-pyridyl-3-butene-2-one,
3,4-dipyridyl-3-butene-2-one,
4-(4-pyridyl)-3-pyridyl-3-butene-2-one,
4-(3-nitrophenyl)-3-quinoxalyl-3-butene-one,
3-(2-methylpyridyl)-4-(3-nitrophenyl)-3-butene-2-one,
3-(6-methylpyridyl)-4-(3-nitrophenyl)-3-butene-2-one,
3-(2-methoxypyridyl)-4-(3-nitrophenyl)-3-butene-2-one,
3-(6-methoxypyridyl)-4-(3-nitrophenyl)-3-butene-2-one,
3-(5-bromopyridyl)-4-(3-nitrophenyl)-3-butene-2-one,
3-(2-aminopyridyl)-4-(3-nitrophenyl)-3-butene-2-one,
3-(2-mercaptopyridyl)-4-(3-nitrophenyl)-3-butene-2-one,
3-(7-benzoxazolyl)-4-(3-nitrophenyl)-3-butene-2-one,
3-(7-benzothiazolyl)-4-(3-nitrophenyl)-3-butene-2-one,
3-(7-benzoxyathiazolyl)-4-(3-nitrophenyl)-3-butene-2-one,
3-(3-benzothiadiazolyl)-4-(3-nitrophenyl)-3-butene-2-one,
3-(2-indolyl)-4-(3-nitrophenyl)-3-butene-2-one,
3-(3-indolyl)-4-(3-nitrophenyl)-3-butene-2-one,
3-(5-indolyl)-4-(3-nitrophenyl)-3-butene-2-one,

4-(3-nitrophenyl)-3-pyridazinyl-3-butene-2-one,
4-(3-nitrophenyl)-3-quinolyl-3-butene-2-one,
3-(2-furyl)-4-(3-nitrophenyl)-3-butene-2-one,
3-(2-methoxyphenyl)-4-(3-nitrophenyl)-3-butene-2-one,
3-(2-fluorophenyl)-4-(3-nitrophenyl)-3-butene-2-one,
3-(2-chloropyridyl)-4-(3-nitrophenyl)-3-butene-2-one,
3-(6-chloropyridyl)-4-(3-nitrophenyl)-3-butene-2-one,
3-(5-isoquinolyl)-4-(3-nitrophenyl)-3-butene-2-one,
4-(3-nitrophenyl)-3-thienyl-3-butene-2-one,
4-(1-naphthyl)-3-pyridyl-3-butene-2-one,
4-naphthyl-3-pyridyl-3-butene-2-one,
4-(3-azidophenyl)-3-pyridyl-3-butene-2-one,
4-(2-methylphenyl)-3-pyridyl-3-butene-2-one,
4-(3-methylphenyl)-3-pyridyl-3-butene-2-one,
4-(4-methylphenyl)-3-pyridyl-3-butene-2-one,
4-(2-chlorophenyl)-3-pyridyl-3-butene-2-one,
4-(2-bromophenyl)-3-pyridyl-3-butene-2-one,
4-(3-bromophenyl)-3-pyridyl-3-butene-2-one,
4-(4-bromophenyl)-3-pyridyl-3-butene-2-one,
4-(2-iodophenyl)-3-pyridyl-3-butene-2-one,
4-(3-iodophenyl)-3-pyridyl-3-butene-2-one,
4-(4-iodophenyl)-3-pyridyl-3-butene-2-one,
4-(1-isoquinolyl)-3-pyridyl-3-butene-2-one,
4-(5-isoquinolyl)-3-pyridyl-3-butene-2-one,
4-(2-hydroxyphenyl)-3-pyridyl-3-butene-2-one,
4-(3-hydroxyphenyl)-3-pyridyl-3-butene-2-one,
4-(4-hydroxyphenyl)-3-pyridyl-3-butene-2-one,
4-(2-benzyloxyphenyl)-3-pyridyl-3-butene-2-one,
4-(3-benzyloxyphenyl)-3-pyridyl-3-butene-2-one,
4-(4-benzyloxyphenyl)-3-pyridyl-3-butene-2-one,
4-(2-cinnamyloxyphenyl)-3-pyridyl-3-butene-2-one,
4-(3-cinnamyloxyphenyl)-3-pyridyl-3-butene-2-one,
4-(4-cinnamyloxyphenyl)-3-pyridyl-3-butene-2-one, and
4-(3-benzoylphenyl)-3-pyridyl-3-butene-2-one.

Examples of the compounds having formula (I-2) are as follows:

methyl 3-aminocrotonate,
ethyl 3-aminocrotonate,
n-propyl 3-aminocrotonate,
n-butyl 3-aminocrotonate,
n-pentyl 3-aminocrotonate,
n-hexyl 3-aminocrotonate,
n-heptyl 3-aminocrotonate,
n-octyl 3-aminocrotonate,
n-nonyl 3-aminocrotonate,
n-decyl 3-aminocrotonate,
isopropyl 3-aminocrotonate,
isobutyl 3-aminocrotonate,
cyclopentyl 3-aminocrotonate,
cyclohexyl 3-aminocrotonate,
propene-1-yl 3-aminocrotonate,
2-propyne-1-yl 3-aminocrotonate,
(E)-2-butene-1-yl 3-aminocrotonate,
(E)-3-butene-1-yl 3-aminocrotonate,
(E)-2-pentene-1-yl 3-aminocrotonate,
(2E,4E)-2,4-hexadienyl 3-aminocrotonate,
2,4-hexadienyl 3-aminocrotonate,
(E)-hexa-4-en-2-yne 3-aminocrotonate,
(E)-3-phenyl-2-propene-1-yl 3-aminocrotonate,

(Z)-3-phenyl-2-propene-1-yl 3-aminocrotonate,
3-phenyl-2-propyne-1-yl 3-aminocrotonate,
(2E,4E)-5-phenyl-2,4-pentadiene-1-yl 3-aminocrotonate,
5-phenyl-penta-2,4-diyne-1-yl 3-aminocrotonate,
(E)-5-phenyl-penta-2-ene-4-yn-1-yl 3-aminocrotonate,
(E)-3-[4-(1-imidazolylmethyl)phenyl]-2-propene-1-yl 3-aminocrotonate,
(E)-3-[3-(1-imidazolylmethyl)phenyl]-2-propene-1-yl 3-aminocrotonate,
(E)-3-[2-(1-imidazolylmethyl)phenyl]-2-propene-1-yl 3-aminocrotonate,
(Z)-3-[4-(1-imidazolylmethyl)phenyl]-2-propene-1-yl 3-aminocrotonate,
E)-3-[6-(1-imidazolylmethyl)pyridine-2-yl]-2-propene-1-yl 3-aminocrotonate,
(E)-3-[5-(1-imidazolylmethyl)furan-2-yl]-2-propene-1-yl 3-aminocrotonate,
(E)-3-[5-(1-imidazolylmethyl)thiophene-2-yl]-2-propene-1-yl 3-aminocrotonate,
(E)-3-phenyl-1-methyl-2-propene-1-yl 3-aminocrotonate,
(E)-1-fluoro-3-phenyl-2-propene-1-yl 3-aminocrotonate,
2-methoxyethyl 3-aminocrotonate,
3-methoxypropyl 3-aminocrotonate,
3-ethoxypropyl 3-aminocrotonate,
2-phenoxyethyl 3-aminocrotonate,
2-phenylthioethyl 3-aminocrotonate,
2-(N-methylamino)ethyl 3-aminocrotonate,
2-(N,N-dimethylamino)ethyl 3-aminocrotonate,
2-(N-methyl-N-phenylamino)ethyl 3-aminocrotonate,
2-(N,N-diethylamino)ethyl 3-aminocrotonate,
2-(N-benzyl-N-methylamino)ethyl 3-aminocrotonate;
2-(1-piperazinyl)ethyl 3-aminocrotonate,
4-(1-piperazinyl)butyl 3-aminocrotonate,
6-(1-piperazinyl)hexyl 3-aminocrotonate,
2-(4-phenylpiperazine-1-yl)ethyl 3-aminocrotonate,
3-(4-phenylpiperazine-1-yl)propyl 3-aminocrotonate,
4-(4-phenylpiperazine-1-yl)butyl 3-aminocrotonate,
6-(4-phenylpiperazine-1-yl)hexyl 3-aminocrotonate,
2-(4-phenylpiperidine-1-yl)ethy 3-aminocrotonate,
3-(4-phenylpiperidine-1-yl)propyl 3-aminocrotonate,
4-(4-phenylpiperidine-1-yl)butyl 3-aminocrotonate,
6-(4-phenylpiperidine-1-yl)hexyl 3-aminocrotonate,
2-[4-(diphenylmethyl)piperazine-1-yl]ethyl 3-aminocrotonate,
3-[4-(diphenylmethyl)piperazine-1-yl]propyl 3-aminocrotonate,
4-[4-(diphenylmethyl)piperazine-1-yl]butyl 3-aminocrotonate,
2-morpholinoethyl 3-aminocrotonate,
N-benzylpyrrolidine-3-yl 3-aminocrotonate,
N-benzylpiperiridine-3-yl 3-aminocrotonate,
2-(1,2,3,4-tetrahyisoquinoline-2-yl)ethyl 3-aminocrotonate,
2,2,2-trifluoroethyl 3-aminocrotonate,
2-(3,7-dihydro-3,7-dimethyl-1H-purine-2,6-dione-1-yl)ethyl 3-aminocrotonate,
2-(1,2,3,6-tetrahydro-1,3-dimethyl-2,6-dioxo-7H-purine-7-yl)ethyl 3-aminocrotonate,
3-aminocrotonitrile,
N,N-diethyl 3-amino-2-butene amide,
3-amino-1-phenyl-2-butene-1-one,
4-amino-3-pentene-2-one,
2-amino-1-nitro-1-propene, and
methyl 3-amino-3-propene sulfinate.

The reaction can be carried out without any solvents, or in an inactive solvent such as tetrahydrofuran, 1,4-dioxane, benzene, toluene, methanol, ethanol, n-propanol, isopropanol, dimethyl sulfoxide, and dimethylformamide at temperatures of 50° C to 150° C.

[Process 2]

Carboxylic acid derivatives having formula (I-3)

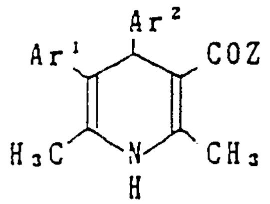

(I-3)

are allowed to react with alcohol derivatives having formula (I-4)
HO-$R^2$ (I-4)
wherein $R^2$, $Ar^1$ and $Ar^2$ are the same as those defined previously in formula (I), and Z is a hydroxyl group, a halogen, or an active ester moiety such as a methanesulfonyloxy group, a benzotriazol-1-oxy group, or a succinimide group.

The above carboxylic acid derivatives of formula (I-3) can be prepared without difficulty as will be decribed later.

Examples of the carboxylic acid derivatives of formula (I-3) are as follows:
1,4-dihydro-2,6-dimethyl-4-(3-nitrophenyl)-5-pyridylpyridine-3-carboxylic acid chloride,
1,4-dihydro-2,6-dimethyl-4-(3-nitrophenyl)-5-(2-pyridyl)pyridine-3-carboxylic acid,
1,4-dihydro-2,6-dimethyl-4-(3-nitrophenyl)-5-pyridylpyridine-3-carboxylic acid,
1,4-dihydro-2,6-dimethyl-4-(3-nitrophenyl)-5-(4-pyridyl)pyridine-3-carboxylic acid,
1,4-dihydro-2,6-dimethyl-4-(3-nitrophenyl)-5-pyrazinylpyridine-3-carboxylic acid,
1,4-dihydro-2,6-dimethyl-4-(3-nitrophenyl)-5-pyridazinylpyridine-3-carboxylic acid,
1,4-dihydro-2,6-dimethyl-4-(3-nitrophenyl)-5-(4-pyridazinyl)pyridine-3-carboxylic acid,
1,4-dihydro-2,6-dimethyl-4-(3-nitrophenyl)-5-pyrimidylpyridine-3-carboxylic acid,
1,4-dihydro-2,6-dimethyl-4-(3-nitrophenyl)-5-(5-pyrimidyl)pyridine-3-carboxylic acid,
1,4-dihydro-2,6-dimethyl-4-(3-nitrophenyl)-5-quinolylpyridine-3-carboxylic acid,
1,4-dihydro-2,6-dimethyl-4-(3-nitrophenyl)-5-(3-quinolyl)pyridine-3-carboxylic acid,
1,4-dihydro-2,6-dimethyl-4-(3-nitrophenyl)-5-(2-pyridyl)pyridine-3-carboxylic acid,
1,4-dihydro-2,6-dimethyl-4-(3-nitrophenyl)-5-(4-quinolyl)pyridine-3-carboxylic acid,
1,4-dihydro-2,6-dimethyl-5-(1-isoquinolyl)-4-(3-nitrophenyl)pyridine-3-carboxylic acid,
1,4-dihydro-2,6-dimethyl-4-(3-nitrophenyl)-5-quinoxalylpyridine-3-carboxylic acid,
1,4-dihydro-2,6-dimethyl-5-(2-methylpyridyl)-4-(3-nitrophenyl)-pyridine-3-carboxylic acid,
1,4-dihydro-2,6-dimethyl-5-(6-methylpyridyl)-4-(3-nitrophenyl)pyridine-3-carboxylic acid,
1,4-dihydro-2,6-dimethyl-5-(2-methoxypyridyl)-4-(3-nitrophenyl)pyridine-3-carboxylic acid,
1,4-dihydro-2,6-dimethyl-5-(6-methoxypyridyl)-4-(3-nitrophenyl)pyridine-3-carboxylic acid,
1,4-dihydro-2,6-dimethyl-5-(2-mercaptopyridyl)-4-(3-nitrophenyl)pyridine-3-carboxylic acid,
5-(5-bromopyridyl)-1,4-dihydro-2,6-dimethyl-4-(3-nitrophenyl)pyridine-3-carboxylic acid,
5-(2-aminopyridyl)-1,4-dihydro-2,6-dimethyl-4-(3-nitrophenyl)pyridine-3-carboxylic acid,
5-(2-chloropyridyl)-1,4-dihydro-2,6-dimethyl-4-(3-nitrophenyl)pyridine-3-carboxylic acid,
5-(6-chloropyridyl)-1,4-dihydro-2,6-dimethyl-4-(3-nitrophenyl)pyridine-3-carboxylic acid,
4-(7-benzoxazolyl)-1,4-dihydro-2,6-dimethyl-5-pyridylpyridine-3-carboxylic acid,
4-(7-benzothiazolyl)-1,4-dihydro-2,6-dimethyl-5-pyridylpyridine-3-carboxylic acid,
4-(3-benzoxazolyl)-1,4-dihydro-2,6-dimethyl-5-pyridylpyridine-3-carboxylic acid,
4-(3-benzothiazolyl)-1,4-dihydro-2,6-dimethyl-5-pyridylpyridine-3-carboxylic acid,
1,4-dihydro-2,6-dimethyl-4-(2-indolyl)-5-pyridylpyridine-3-carboxylic acid,
1,4-dihydro-2,6-dimethyl-4-(3-indolyl)-5-pyridylpyridine-3-carboxylic acid,
1,4-dihydro-2,6-dimethyl-4-(5-indolyl)-5-pyridylpyridine-3-carboxylic acid,
4-(2-cyanophenyl)-1,4-dihydro-2,6-dimethyl-5-pyridylpyridine-3-carboxylic acid,
1,4-dihydro-2,6-dimethyl-5-pyridyl-4-(2-trifluoromethylphenyl)pyridine-3-carboxylic acid,
1,4-dihydro-2,6-dimethyl-5-pyridyl-4-(3-trifluoromethylphenyl)pyridine-3-carboxylic acid,
1,4-dihydro-2,6-dimethyl-5-pyridyl-4-(4-trifluoromethylphenyl)pyridine-3-carboxylic acid,
4-(2-bromophenyl)-1,4-dihydro-2,6-dimethyl-5-pyridylpyridine-3-carboxylic acid,
4-(3-bromophenyl)-1,4-dihydro-2,6-dimethyl-5-pyridylpyridine-3-carboxylic acid,
4-(4-bromophenyl)-1,4-dihydro-2,6-dimethyl-5-pyridylpyridine-3-carboxylic acid,
4-(2-chlorophenyl)-1,4-dihydro-2,6-dimethyl-5-pyridylpyridine-3-carboxylic acid,
4-(3-chlorophenyl)-1,4-dihydro-2,6-dimethyl-5-pyridyl pyridine-3-carboxylic acid,

4-(4-chlorophenyl)-1,4-dihydro-2,6-dimethyl-5-pyridylpyridine-3-carboxylic acid,
4-(2,3-dichlorophenyl)-1,4-dihydro-2,6-dimethyl-5-pyridylpyridine-3-carboxylic acid,
4-(2,6-dichlorophenyl)-1,4-dihydro-2,6-dimethyl-5-pyridylpyridine-3-carboxylic acid,
4-(3,5-dichlorophenyl)-1,4-dihydro-2,6-dimethyl-5-pyridylpyridine-3-carboxylic acid,
1,4-dihydro-2,6-dimethyl-4-(2-furyl)-5-pyridylpyridine-3-carboxylic acid,
1,4-dihydro-2,6-dimethyl-4-furyl-5-pyridylpyridine-3-carboxylic acid,
1,4-dihydro-2,6-dimethyl-5-pyridyl-4-thienylpyridine-3-carboxylic acid,
1,4-dihydro-2,6-dimethyl-5-pyridyl-4-(3-thienyl)pyridine-3-carboxylic acid,
4-(3-azidophenyl)-1,4-dihydro-2,6-dimethyl-5-pyridylpyridine-3-carboxylic acid,
1,4-dihydro-2,6-dimethyl-4-(1-naphthyl)-5-pyridylpyridine-3-carboxylic acid,
1,4-dihydro-2,6-dimethyl-4-naphthyl-5-pyridylpyridine-3-carboxylic acid,
1,4-dihydro-2,6-dimethyl-4-(2-iodophenyl)-5-pyridylpyridine-3-carboxylic acid,
1,4-dihydro-2,6-dimethyl-4-(3-iodophenyl)-5-pyridylpyridine-3-carboxylic acid,
1,4-dihydro-2,6-dimethyl-4-(4-iodophenyl)-5-pyridylpyridine-3-carboxylic acid,
1,4-dihydro-2,6-dimethyl-4-(2-methylphenyl)-5-pyridylpyridine-3-carboxylic acid,
1,4-dihydro-2,6-dimethyl-4-(3-methylphenyl)-5-pyridylpyridine-3-carboxylic acid,
1,4-dihydro-2,6-dimethyl-4-(4-methylphenyl)-5-pyridylpyridine-3-carboxylic acid,
1,4-dihydro-2,6-dimethyl-4-(2-methoxyphenyl)-5-pyridylpyridine-3-carboxylic acid,
1,4-dihydro-2,6-dimethyl-4-(3-methoxyphenyl)-5-pyridylpyridine-3-carboxylic acid,
1,4-dihydro-2,6-dimethyl-4-(4-methoxyphenyl)-5-pyridylpyridine-3-carboxylic acid,
1,4-dihydro-2,6-dimethyl-4-(4-methylthiophenyl)-5-pyridylpyridine-3-carboxylic acid,
1,4-dihydro-2,6-dimethyl-4-(2-hydroxyphenyl)-5-pyridylpyridine-3-carboxylic acid,
1,4-dihydro-2,6-dimethyl-4-(3-hydroxyphenyl)-5-pyridylpyridine-3-carboxylic acid,
1,4-dihydro-2,6-dimethyl-4-(4-hydroxyphenyl)-5-pyridylpyridine-3-carboxylic acid,
4-(2-benzyloxyphenyl)-1,4-dihydro-2,6-dimethyl-5-pyridylpyridine-3-carboxylic acid,
4-(4-benzyloxyphenyl)-1,4-dihydro-2,6-dimethyl-5-pyridylpyridine-3-carboxylic acid,
4-(2-cinnamyloxyphenyl)-1,4-dihydro-2,6-dimethyl-5-pyridylpyridine-3-carboxylic acid,
4-(3-cinnamyloxyphenyl)-1,4-dihydro-2,6-dimethyl-5-pyridylpyridine-3-carboxylic acid,
4-(4-cinnamyloxyphenyl)-1,4-dihydro-2,6-dimethyl-5-pyridylpyridine-3-carboxylic acid, and
4-(3-benzoylphenyl)-1,4-dihydro-2,6-dimethyl-5-pyridylpyridine-3-carboxylic acid.

The alcohol derivatives having formula (I-4) can be prepared without difficulty by using compounds which are easily available industriously,

Examples of the alcohol derivatives are as follows: methyl alcohol, ethyl alcohol, n-propyl alcohol, n-butyl alcohol, n-pentyl alcohol, n-hexyl alcohol, n-heptyl alcohol, n-octyl alcohol, n-nonyl alcohol, n-decyl alcohol, isopropyl alcohol, isobutyl alcohol, cyclopentyl alcohol, cyclohexyl alcohol, 2-propyne-1-ol, (E)-2-butene-1-ol, (E)-3-butene-1-ol, (E)-2-pentene-1-ol, (2E,4E)-2,4-hexadiene-1-ol, 2,4-hexadiene-1-ol, (E)-hexa-4-ene-2-yne-1-ol, (E)-3-phenyl-2-propene-1-ol, (Z)-3-phenyl-2-propene-1-ol, 3-phenyl-2-propyne-1-ol, (2E,4E)-5-phenyl-2,4-pentadiene-1-ol, 5-phenyl-2,4-pentadiene-1-ol,
(E)-5-phenyl-penta-2-ene-4-yne-1-ol,
(E)-3-[4-(1-imidazolylmethyl)phenyl]-2-propene-1-ol,
(E)-3-[3-(1-imidazolylmethyl)phenyl]-2-propene-1-ol,
(E)-3-[2-(1-imidazolylmethyl)phenyl]-2-propene-1-ol,
(Z)-3-[4-(1-imidazolylmethyl)phenyl]-2-propene-1-ol,
(E)-3-[6-(1-imidazolylmethyl)pyridine-2-yl]-2-propene-1-ol,
(E)-3-[5-(1-imidazolylmethyl)furan-2-yl]-2-propene-1-ol,
(E)-3-[5-(1-imidazolylmethyl)thiophene-2-yl]-2-propene-1-ol,
(E)-3-phenyl-1-methyl-2-propene-1-ol,
(E)-2-fluoro-3-phenyl-2-propene-1-ol,
2-methoxyethanol, 3-methoxypropanol, 2-phenoxyethanol,
2-phenylthioethanol, 2-(N-methylamino)ethanol,
2-(N,N-dimethylamino)ethanol,
2-(N-methyl-N-phenylamino)ethanol,
2-(N,N-dimethylamino)ethanol,
2-(N-benzyl-N-methylamino)ethanol,
2-(1-piperazinyl)ethanol,
4-(1-piperazinyl)butanol,
6-(1-piperazinyl)hexanol,
2-(1-piperidinyl)ethanol,

2-(4-phenylpiperazine-1-yl)ethanol,
3-(4-phenylpiperazine-1-yl)propanol,
4-(4-phenylpiperazine-1-yl)butanol,
6-(4-phenylpiperazine-1-yl)hexanol,
2-(4-phenylpiperidine-1-yl)ethanol,
3-(4-phenylpiperidine-1-yl)propanol,
4-(4-phenylpiperidine-1-yl)butanol,
6-(4-phenylpiperidine-1-yl)hexanol,
2-[4-(diphenylmethyl)piperazine-1-yl]ethanol,
3-[4-(diphenylmethyl)piperazine-1-yl]propanol,
4-[4-(diphenylmethyl)piperazine-1-yl]butanol,
6-[4-(diphenylmethyl)piperazine-1-yl]hexanol,
2-morpholinoethanol, N-benzylpyrrolidine-3-yl-1-ol,
N-benzylpiperidine-3-ol, 2-(1,2,3,4-tetrahydroisoquinoline-2-yl)ethanol, 2,2,2-trifluoroethanol, 2-(3,7-dihydro-3,7-dimethyl-1H-purine-2,6-dione-1-yl)ethanol, and 2-(1,2,3,6-tetrahydro-1,3-dimethyl-2,6-dioxo-7H-purine-7-yl)ethanol.

It is preferable that the reaction be carried out in an inactive solvent, such as benzene, toluene, dichloromethane, chloroform, 1,2-dichloroethane, acetonitrile, and acetone, at temperatures of $0^\circ C$ to $120^\circ C$.

The compounds prepared by Process 1 and Process 2 can be easily isolated by conventional procedures.

Starding materials for preparing 1,4-dihydropyridine derivatives of the present invention can be synthesized as follows:

1. Synthesis of 4-(3-nitrophenyl)-3-pyridyl-3-butene-2-one:

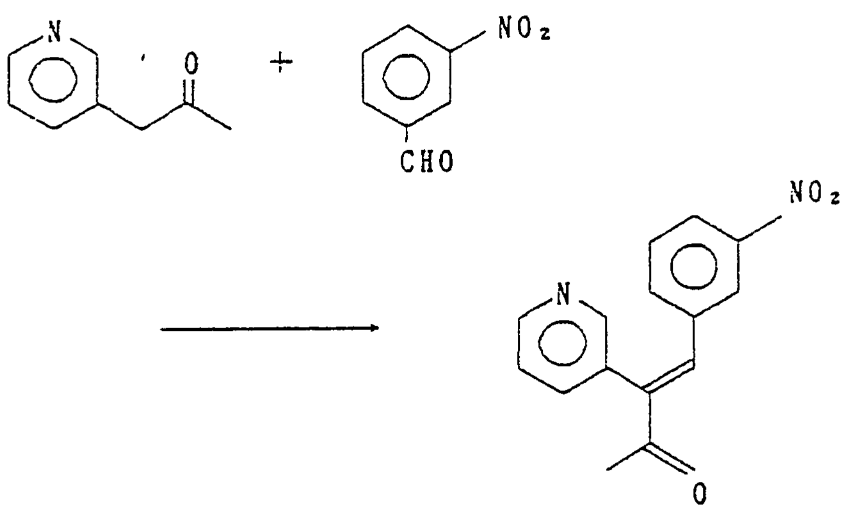

2.76 g (20 mmol) of 1-pyridyl-2-propanone and 3.066 g (20 mmol) of 3-nitrobenzaldehyde were dissolved in benzene. To this solution, 0.29 g (2.0 mmol) of piperidinium acetate was added, and the mixture was refluxed with application of heat for 6 hours for dehydration. The reaction mixture was washed with water, and dried over anhydrous sodium sulfate. The benzene contained in the reaction mixture was distilled off under reduced pressure and the remaining reaction product was chromatographed on a silica gel column, whereby the captioned compound was obtained. The yield was 4.5 g (87.9%).

NMR (δ, $CDCl_3$) 2.47(3H,s)
7.30(1H,d,J=8.4Hz)
7.38(1H,dd,J=8.4Hz,8.4Hz)
7.39(1H,dd,J=7.8Hz,5Hz)
7.56(1H,ddd,J=7.8Hz,2Hz,2Hz)

7.78(1H,s)
7.92(1H,s)
8.10(1H,d,J=8.4Hz)
8.35(1H,d,J=2Hz)
8.66(1H,dd,J=5Hz,2Hz)

2. Synthesis of 4-(3-nitrophenyl)-3-(4-pyridyl)-3-butene-2-one:

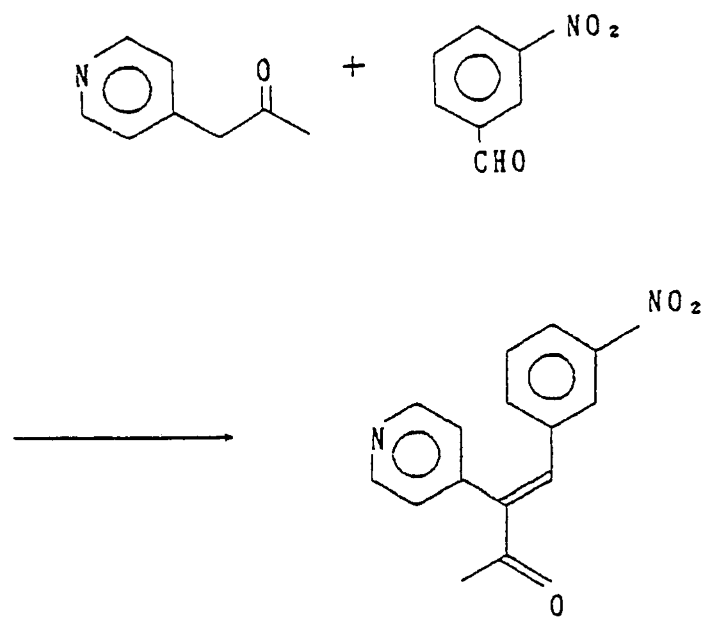

4.62 g (34 mmole) of 1-(4-pyridyl)-2-propanone and 5.14 g (34 mmol) of 3-nitrobenzaldehyde were dissolved in benzene. To this solution, 0.49 g (3.4 mmol) of piperidinium acetate was added, and the mixture was refluxed with application of heat for 6 hours for dehydration. The reaction mixture was washed with water, and dried over anhydrous sodium sulfate. The benzene contained in the reaction mixture was distilled off under reduced pressure and the remaining reaction product was chromatographed on a silica gel column, whereby the captioned compound was obtained. The yield was 7.695 g (87.3%).

NMR (δ, $CDCl_3$) 2.45(3H,s)
7.15(2H,d,J=5.4Hz)
7.26(1H,d,J=8.4Hz)
7.37(1H,dd,J=8.4Hz,8.4Hz)
7.75(1H,s)
8.01(1H,s)
8.12(1H,d,J=8.4Hz)
8.69(1H,d,J=5.4Hz)

3. Synthesis of 2-cyanoethyl 1,4-dihydro-2,6-dimethyl-4-(3-nitrophenyl)-5-pyridylpyridine-3-carboxylate:

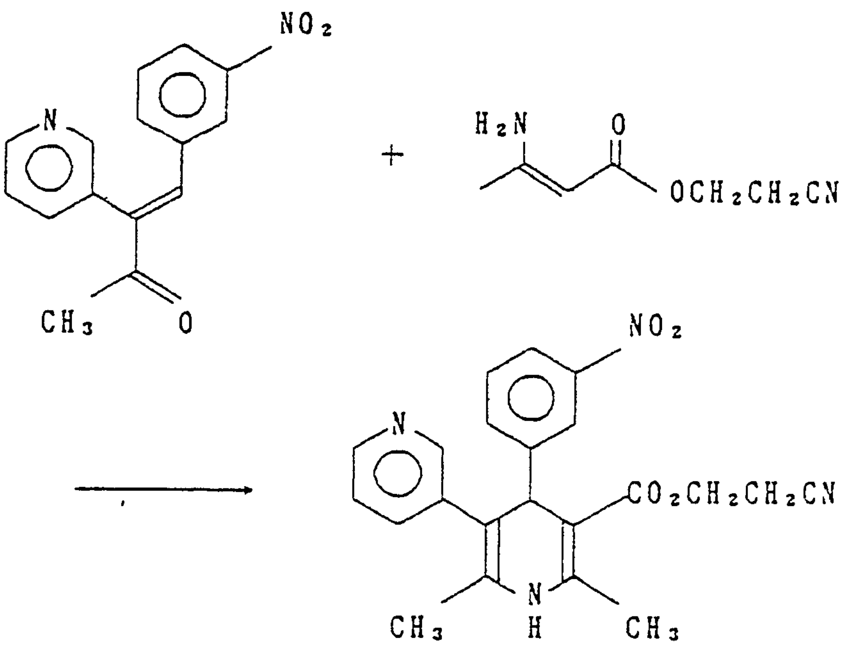

A mixuture of 5.365 g (20 mmol) of 4-(3-nitrophenyl)-3-pyridyl-3-butene-2-one, 15.417 g (100 mmol) of 2-cyanoethyl 3-aminocrotonate, 5.451 g (40 mmol) of zinc chloride, and 10 g of Molecular Shieves 4A was added to 1,4-dioxane. This reaction mixture was refluxed with application of heat in an inert atmosphere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 7.94 g (87%).

IR ($cm^{-1}$, KBr) $\nu_{CO}$1696 $\nu_{NO2}$1530,1352

$\nu_{CN}$2256

NMR ($\delta$, $CDCl_3$) 1.86(3H,s)

2.42(3H,s)

2.58-2.66(2H,m)

4.18-4.30(2H,m)

4.78(1H,s)

5.92(1H,s)

7.19(1H,dd,J=7.5Hz,4.8Hz)

7.26-7.33(1H,m)

7.37(1H,dd,J=7.8Hz,7.8Hz)

7.49(1H,d,J=7.8Hz)

8.02(1H,d,J=7.8Hz)

8.07(1H,s)

8.19(1H,d,J=2Hz)

8.43(1H,dd,J=4.8HZ,2HZ)

4. Synthesis of 1,4-dihydro-2,6-dimethyl-4-(3-nitrophenyl)-5-(4-pyridyl)pyridine-3-carboxylate:

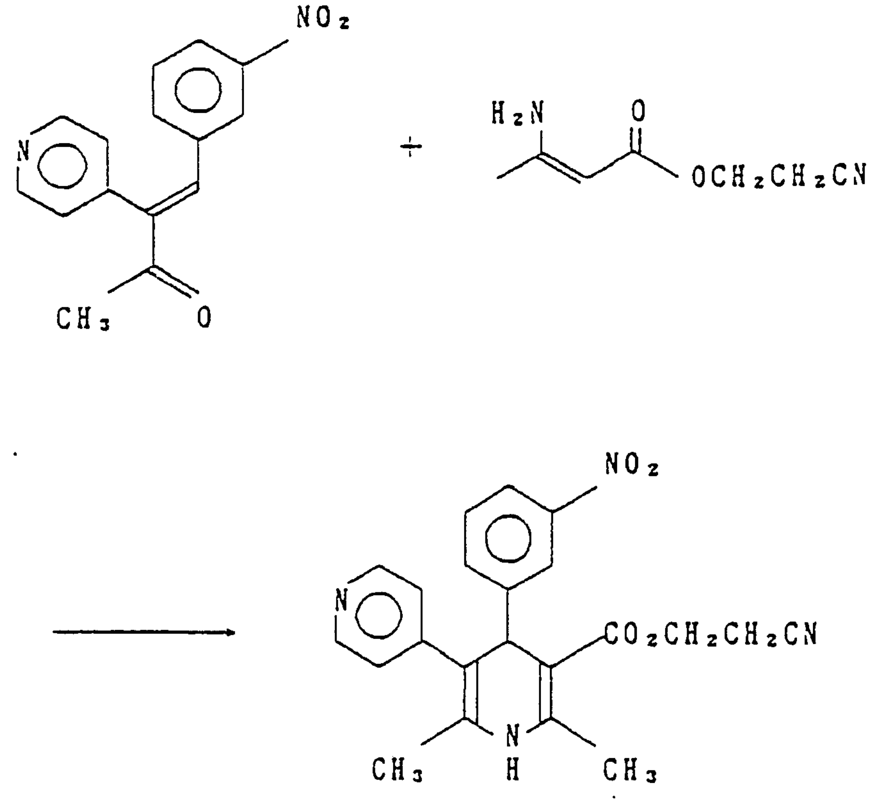

A mixuture of 5.365 g (20 mmol) of 4-(3-nitrophenyl)-3-(4-pyridyl)-3-butene-2-one, 9.250 g (60 mmol) of 2-cyanoethyl 3-aminocrotonate, 5.451 g (40 mmol) of zinc chloride, and 10 g of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 4.980 g (62%).

IR ($cm^{-1}$, KBr) $\nu_{CO}$1704 $\nu_{NO_2}$1528,1352
$\nu_{CN}$2256
NMR ($\delta$, $CDCl_3$) 1.97(3H,s)
2.43(3H,s)
2.60-2.70(2H,m)
4.20-4.33(2H,m)
4.88(1H,s)
5.78 (1H,s)
6.98(2H,d,J = 6Hz)
7.37(1H,dd,J = 8.4Hz,8.4Hz)
7.48(1H,d,J = 8.4Hz)
8.03(1H,d,J = 8.4Hz)
8.08(1H,s)
8.47(2H,d,J = 6Hz)

5. Synthesis of 1,4-dihydro-2,6-dimethyl-4-(3-nitrophenyl)-5-pyridylprydine-3-carboxylic acid:

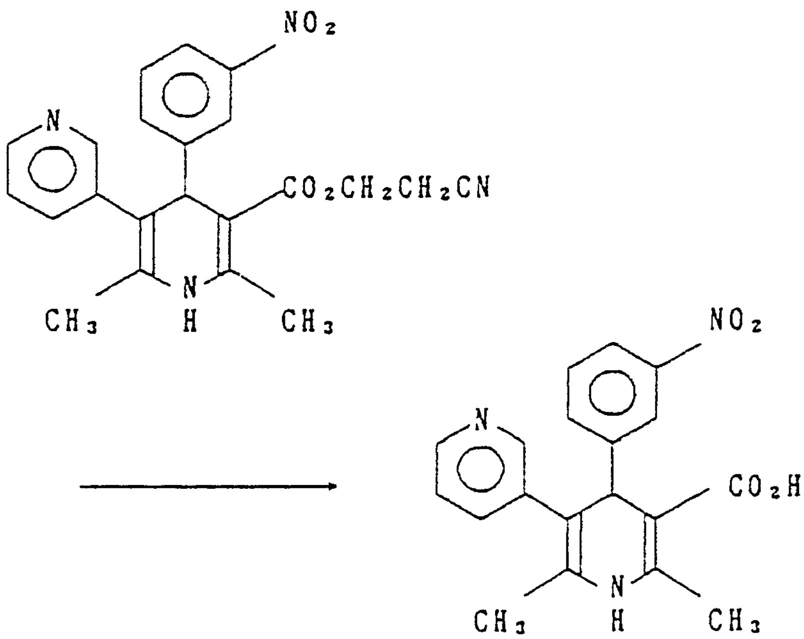

5.40 g (100 mmol) of sodium methoxide was dissolved in 40 ml of anhydrous methanol. To this solution was added, with ice-cooled, 4.04 g (10 mmol) of 1,4-dihydro-2,6- dimethyl-4-(3-nitrophenyl)-5-pyridylpyridine-3-carboxylate. The reaction mixture was stirred for 5 hours and neutralized with acetic acid. To this reaction mixture was added 400 ml of water. Crystals separated were collected, washed with water and dried under reduced pressure, whereby the captioned compound was obtained. The yield was 2.45 g (70%).

FAB Mass 352 (M+1)
NMR (δ, $(CD_3)_2CO$)
1.92(3H,s)
2.38(3H,s)
4.90(1H,s)
7.24(1H,dd,J=8Hz,5Hz)
7.46(1H,d,J=8Hz)
7.50(1H,dd,J=8Hz,8Hz)
7.66(1H,d,J=8Hz)
7.76(1H,s)
8.01(1H,d,J=8Hz)
8.09(1H,s)
8.25(1H,s)
8.36(1H,d,J=5Hz)

6. Synthesis of 1,4-dihyro-2,6-dimethyl-4-(3-nitrophenyl)-5-(4-pyridyl)pyridine-3-carboxylic acid:

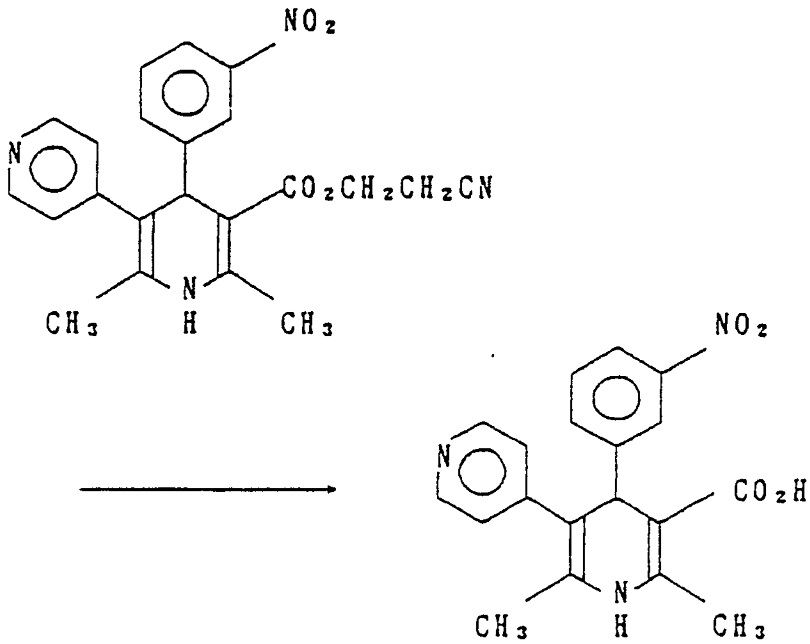

1.35 g (25 mmol) of sodium methoxide was dissolved in 20 ml of anhydrous methanol. To this solution was added, with ice-cooled, 2.02 g (5 mmol) of 1,4-dihydro-2,6-dimethyl-4-(3-nitrophenyl)-5-(4-pyridyl)-pyridine-3-carboxylate. The reaction mixture was stirred at room temperature for 5 hours and neutralized with acetic acid. To this reaction mixture was added 400 ml of water. Crystals were separated from the reaction mixture, which were collected, washed with water and dried under reduced pressure, whereby the captioned compound was obtained. The yield was 1.71 g (98%).

FAB Mass 352 (M+1)

NMR (δ, $(CD_3)_2CO$)

2.03(3H,s)

2.37(3H,s)

4.99(1H,s)

7.09(2H,d,J=6Hz)

7.50(1H,dd,J=7Hz,7Hz)

7.67(1H,d,J=7Hz)

7.83(1H,s)

8.02(1KH,d,J=7Hz)

8.11(1H,s)

8.42(2H,d,J=6Hz)

The synthesis of 1,4-dihydropyridine derivatives of the present invention will now be explained with reference to the examples shown below:

Example 1

Synthesis of methyl 1,4-dihydro-2,6-dimethyl-4-(3-nitrophenyl)-5-pyridylpridine-3-carboxylate:

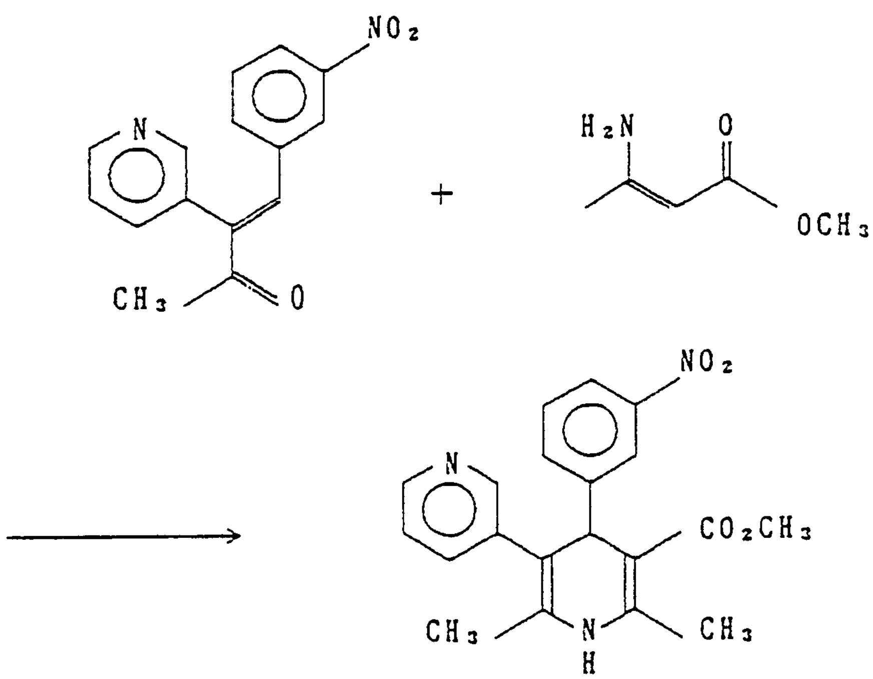

A mixuture of 268 mg (1 mmol) of 4-(3-nitrophenyl)-3-pyridyl-3-butene-2-one, 576 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 259 mg (71%).

Melting point: 148.5 to 150.6 °C

IR ($cm^{-1}$, KBr) ; $\nu$ CO 1700

$\nu$ $NO_2$ 1530, 1345

| Mass Analysis for $C_{20}H_{19}N_3O_4$ | |
|---|---|
| Calculated: | 365.13750 |
| Found: | 365.1375 |

NMR ($\delta$, $CDCl_3$); 1.87(3H,s), 2.40(3H,s), 3.62(3H,s), 4.79(1H,s), 5.60(1H,s), 7.16(1H,dd,J=8Hz,5Hz), 7.26-(1H,dt,J=8Hz,2Hz,2Hz), 7.34(1H,dd,J=8Hz,8Hz), 7.46 (1H,d,J=8Hz), 8.01,(1H,ddd,J=8Hz, 2Hz,1Hz), 8.06-(1H,t,J=2Hz), 8.22(1H,d,J=2Hz), 8.42(1H,dd,J=5Hz,2Hz)

Example 2

Synthesis of ethyl 1,4-dihydro-2,6-dimethyl-4-(3-nitrophenyl)-5-pyridylpridine-3-carboxylate:

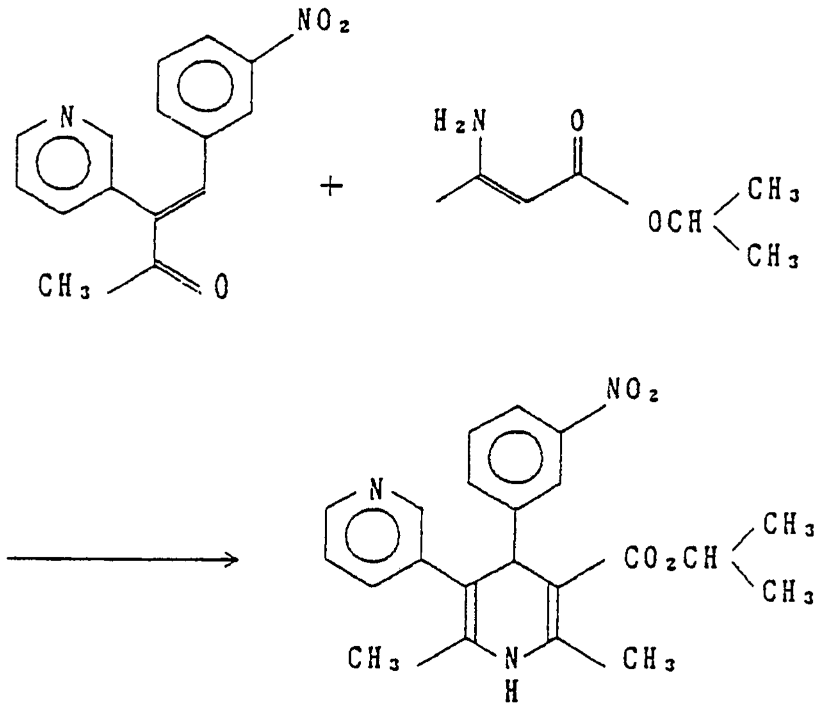

A mixuture of 268 mg (1 mmol) of 4-(3-nitrophenyl)-3-pyridyl-3-butene-2-one, 646 mg (5 mmol) of ethyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 250 mg (67%).

Melting point: oil

IR ($cm^{-1}$, KBr) ; $\nu$ CO 1695

$\nu$ $NO_2$ 1530, 1350

| Mass Analysis for $C_{21}H_{21}N_3O_4$ | |
|---|---|
| Calculated: | 379.15316 |
| Found: | 379.15322 |

NMR ($\delta$, $CDCl_3$); 1.19(3H,t,J=6Hz), 1.88(3H,s), 2.40(3H,s), 4.0~4.15(2H,m), 4.78(1H,s), 5.60(1H,s), 7.20-(1H,dd,J=7,5Hz), 7.29(1H,d,J=7Hz), 7.35 (1H,dd,J=8Hz,8Hz), 7.50 (1H,d,J=8Hz), 8.01(1H,d,J=8Hz), 8.08-(1H,s), 8.24(1H,s), 8.43(1H,d,J=5Hz)

Example 3

Synthesis of isopropyl 1,4-dihydro-2,6-dimethyl-4-(3-nitrophenyl)-5-pyridylpyridine-3-carboxylate:

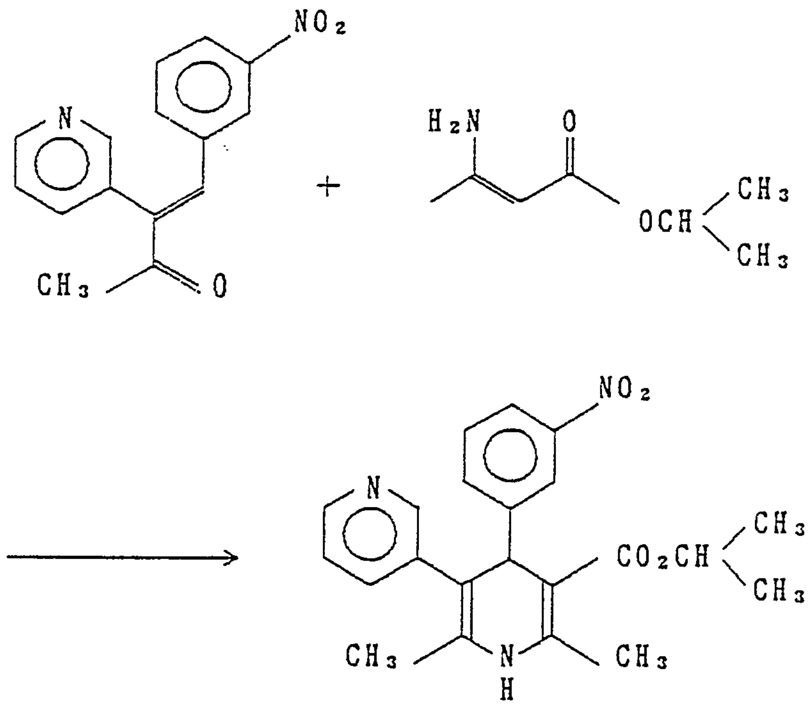

A mixuture of 268 mg (1 mmol) of 4-(3-nitrophenyl)-3-pyridyl-3-butene-2-one, 715 mg (5 mmol) of isopropyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 290 mg (74%).
Melting point: 140.1 to 141.3° C
IR ($cm^{-1}$, KBr) ; ν CO 1705
ν $NO_2$ 1530, 1350

| Mass Analysis for $C_{22}H_{23}N_3O_4$ | |
|---|---|
| Calculated:<br>Found: | 393.16882<br>393.16837 |

NMR (δ, $CDCl_3$); 1.06(3H,d,J=6Hz), 1.23(3H,d,J=6Hz), 1.88(3H,s), 2.40(3H,s), 4.77(1H,s),4.95(1H,m), 5.57 (1H,s), 7.20~7.41(3H,m), 7.50(1H,d,J=8Hz), 8.02(1H,d,J=8Hz), 8.07(1H,s), 8.27(1H,s), 8.44(1H,d,J=5Hz)

Example 4

Synthesis of n-hexyl 1,4-dihydro-2,6-dimethyl-4-(3-nitrophenyl)-5-pyridylpyridine-3-carboxylate:

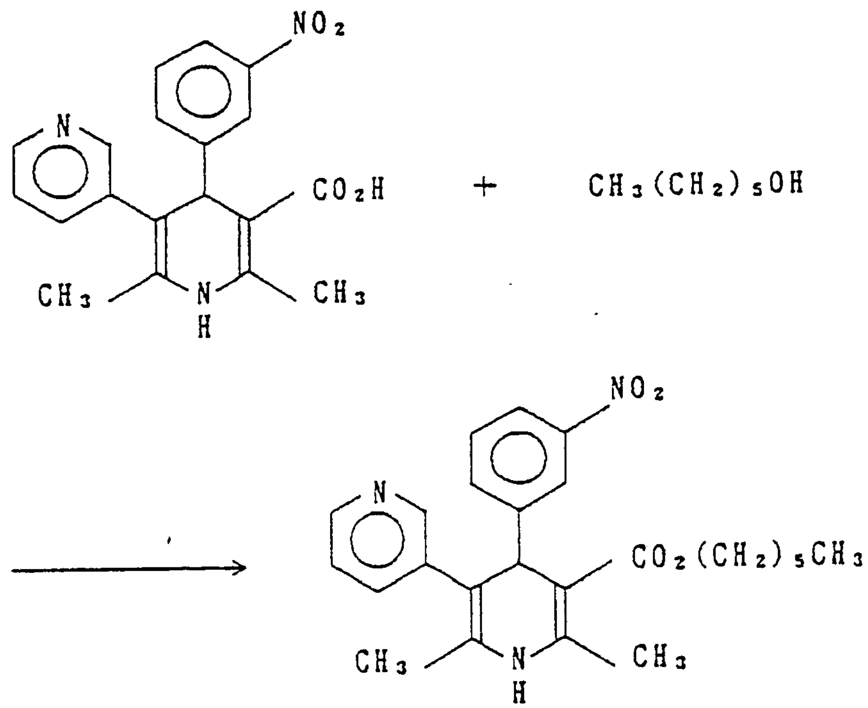

350 mg (1 mmol) of 1,4-dihydro-2,6-dimethyl-4-(3-nitrophenyl)-5-pyridylpyridine-3-carboxylic acid, 102 mg (1 mmol) of hexyl alcohol, 309 mg (1.4 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled of and the residue was chromatographed on a silica gel column, whereby the captioned compound was obtained. The yield was 389 mg (89.5%).

Melting point: 120.5 to 121.6 °C

IR ($cm^{-1}$, KBr) ; ν CO 1705

ν $NO_2$ 1525, 1350

| Mass Analysis for $C_{25}H_{29}N_3O_4$ | |
|---|---|
| Calculated: | 435.21575 |
| Found: | 435.21680 |

NMR (δ, $CDCl_3$); 0.848(3H,t,J=6Hz), 1.12~1.30(6H,m), 1.50~1.60(2H,m), 1.85(3H,s), 2.41(3H,s), 3.96-(1H,dt,J=11,7Hz), 4.04(1H,dt,J=11,7Hz), 4.77(1H,s), 5.57(1H,s), 7.18(1H,dd,J=8,5Hz), 7.28(1H,d,J=8Hz), 7.34(1H,dd,J=8Hz,8Hz), 7.46(1H,d,J=8Hz), 8.00(1H,d,J=8Hz), 8.05(1H,s), 8.23(1H,s), 8.43(1H,d,J=5Hz)

Example 5

Synthesis of cyclohexyl 1,4-dihydro-2,6-dimethyl-4-(3-nitrophenyl)-5-pyridylpyridine-3-carboxylate:

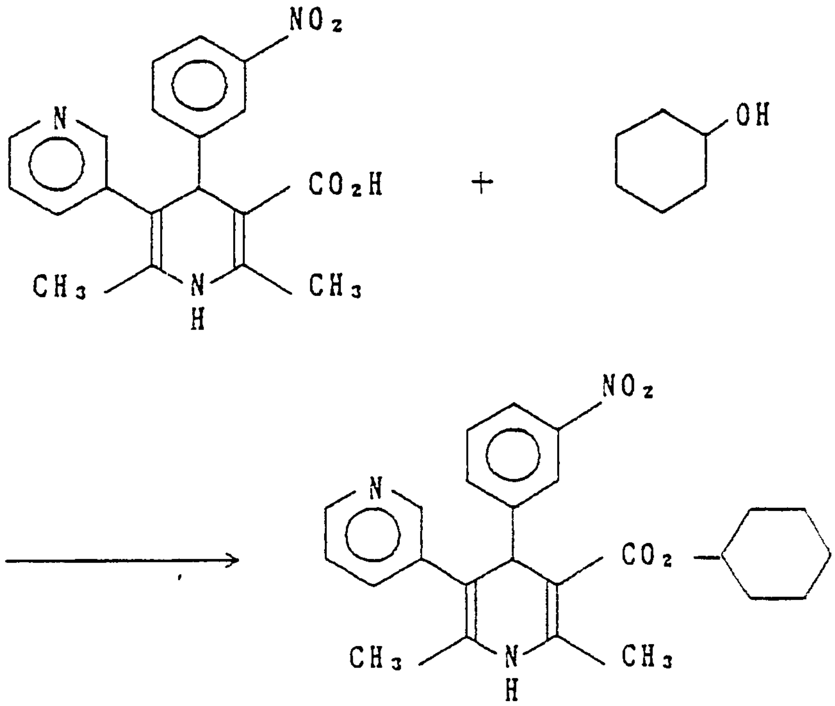

350 mg (1 mmol) of 1,4-dihydro-2,6-dimethyl-4-(3-nitrophenyl)-5-pyridylpyridine-3-carboxylic acid, 100 mg (1 mmol) of cyclohexanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 362 mg (83%).

Melting point: oil

IR ($cm^{-1}$, KBr) ; $\nu$ CO 1694

$\nu$ $NO_2$ 1532, 1350

| Mass Analysis for $C_{25}H_{27}N_3O_4$ | |
|---|---|
| Calculated:<br>Found: | 433.20011<br>433.19727 |

NMR ($\delta$, $CDCl_3$); 1.10~2.24(10H,m), 1.87(3H,s), 2.41(3H,s) , 4.64~4.76(1H,m) , 4.78.(1H,s), 5.58(1H,s), 7.23-(1H,dd,J=7.7Hz,4.9Hz), 7.34(1H,d,J=7.7Hz), 7.35(1H,dd,J=8.7Hz,8.7Hz), 7.49(1H,d,J=8.7Hz), 8.01-(1H,d,J=8.7Hz), 8.06(1H,s), 8.25(1H,d,J=1.5Hz), 8.44(1H,dd,J=4.9Hz,1.5Hz)

Example 6

Synthesis of 2-methoxyethyl 1,4-dihydro-2,6-dimethyl-4-(3-nitrophenyl)-5-pyridylpyridine-3-carboxylate:

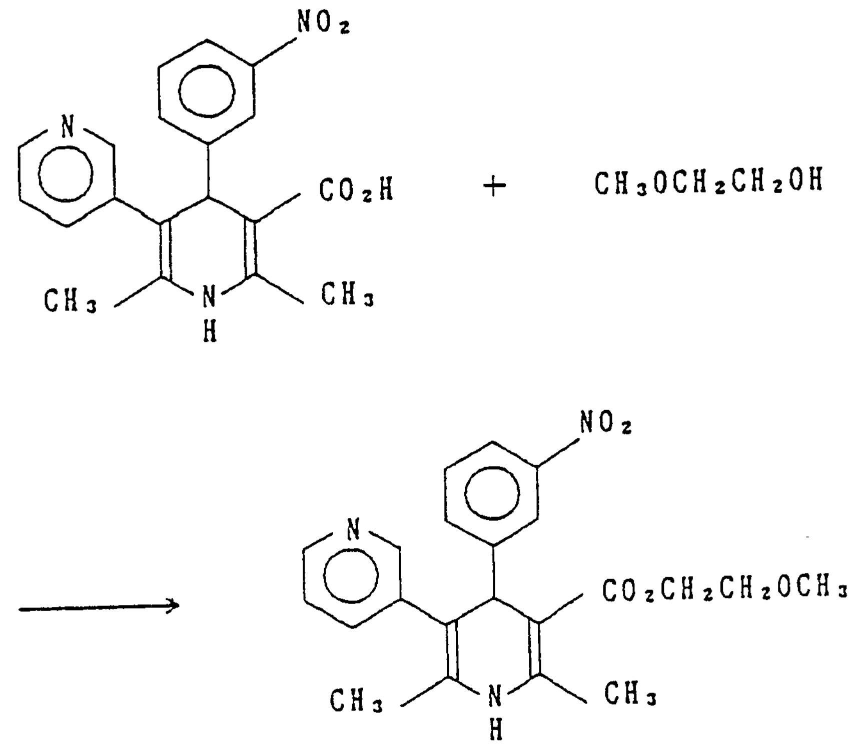


350 mg (1 mmol) of 1,4-dihydro-2,6-dimethyl-4-(3-nitrophenyl)-5-pyridylpyridine-3-carboxylic acid, 76 mg (1 mmol) of 2-methoxyethanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 384 mg (85%).

Melting point: 163.0 to 164.2 °C

IR ($cm^{-1}$, KBr) ; ν CO 1690

ν $NO_2$ 1534, 1352

| Mass Analysis for $C_{22}H_{23}N_3O_5$ | |
|---|---|
| Calculated:<br>Found: | 409.16373<br>409.16560 |

NMR (δ, $CDCl_3$); 1.89(3H,s), 2.40(3H,s), 3.32(3H,s), 3.45~3.58(2H,m), 4.08~4.25(2H,m), 4.81(1H,s), 5.65-(1H,s), 7.28(1H,dd,J=7.8Hz,5Hz), 7.36(1H,dd,J=7.8Hz,7.8Hz), 7.41(1H,d,J=7.8Hz), 7.54(1H,d,J=7.8Hz), 8.02(1H,d,J=7.8Hz), 8.08(1H,s), 8.25(1H,s), 8.44(1H,d,J=3.5Hz)

Example 7

Synthesis of 2-phenoxyethyl 1,4-dihydro-2,6-dimethyl-4-(3-nitrophenyl)-5-pyridylpyridine-3-carboxylate:

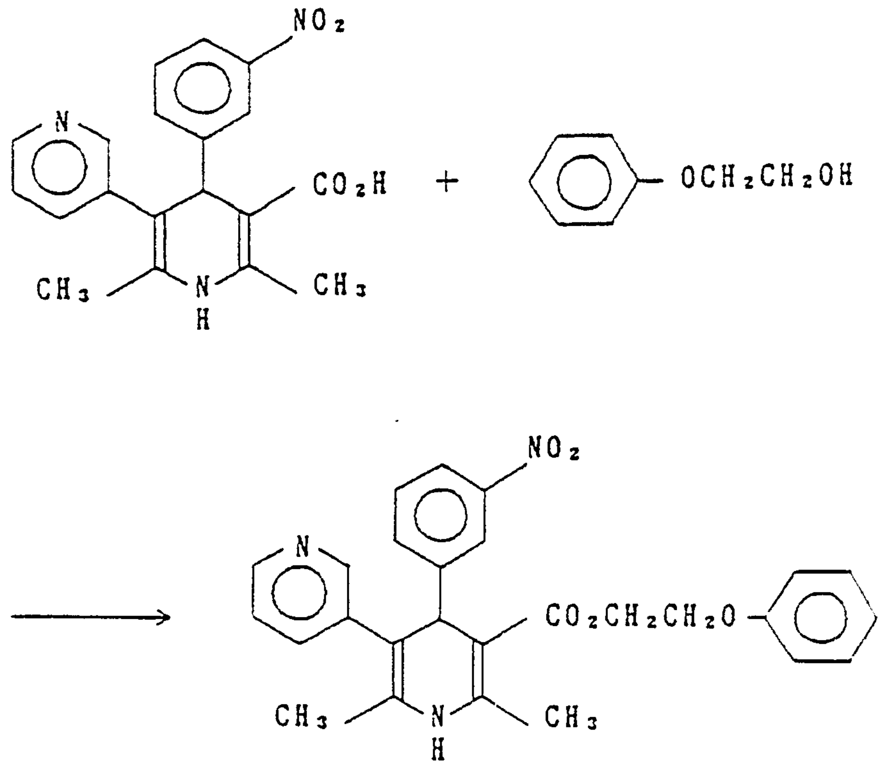

350 mg (1 mmol) of 1,4-dihydro-2,6-dimethyl-4-(3-nitrophenyl)-5-pyridylpyridine-3-carboxylic acid, 138 mg (1 mmol) of 2-phenoxyethanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 445 mg (94%).
Melting point: 69.5 to 70.8° C
IR ($cm^{-1}$, KBr) ; $\nu$ CO 1696
$\nu$ $NO_2$ 1528, 1350

| Mass Analysis for $C_{27}H_{25}N_3O_5$ | |
|---|---|
| Calculated: | 471.17938 |
| Found: | 471.17893 |

NMR ($\delta$, $CDCl_3$); 1.91(3H,s), 2.41(3H,s), 4.02~4.17(2H,m), 4.29~4.50(2H,m), 4.80(1H,s), 5.702 (1H,s), 6.84-(2H,d,J=7.8Hz), 6.96(1H,t,J=7.8Hz), 7.20(1H,t, J=8.2Hz), 7.27(2H,J=7.8Hz), 7.36(1H,dd,J=7.5Hz,5Hz), 7.43~7.51(2H,m), 7.96(1H,d,J=8.2Hz), 8.07(1H,s), 8.27(1H,d,J=2.4Hz), 8.45(1H,dd,J=5Hz,2.4Hz)

Example 8

Synthesis of 2-(phenylthio)ethyl 1,4-dihydro-2,6-dimethyl-4-(3-nitrophenyl)-5-pyridylpyridine-3-carboxylate:

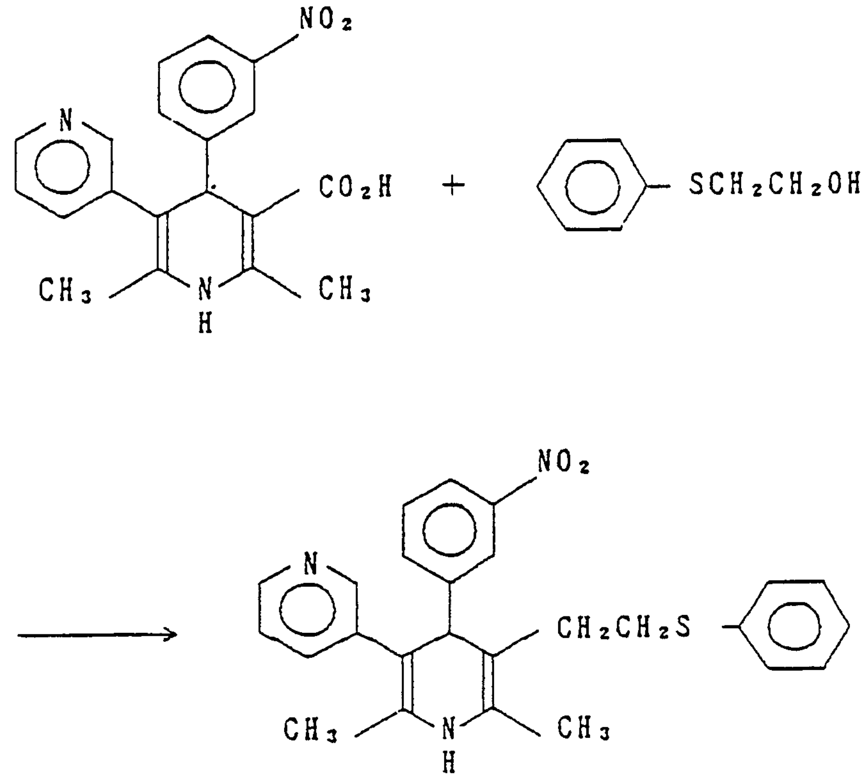

350 mg (1 mmol) of 1,4-dihydro-2,6-dimethyl-4-(3-nitrophenyl)-5-pyridylpyridine-3-carboxylic acid, 154 mg (1 mmol) of 2-(phenylthio)ethanol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 470 mg (96%).
Melting point: oil
IR ($cm^{-1}$, KBr) ; $\nu$ CO 1704
$\nu$ $NO_2$ 1528, 1348

| Mass Analysis for $C_{27}H_{25}N_3O_4S$ | |
|---|---|
| Calculated: | 487.15653 |
| Found: | 487.15736 |

NMR ($\delta$, $CDC\ell_3$); 1.89(3H,s), 2.40(3H,s), 3.70(2H,t,J = 7.2Hz), 4.13~4.27(2H,m), 4.78(1H,s), 5.69 (1H,s), 7.14~7.39(7H,m) 7.42(1H,d,J = 8.4Hz), 7.49(1H,d,J = 7.2Hz), 8.02(1H,d,J = 7.2Hz), 8.06(1H,s), 8.26-(1H,d,J = 2.4Hz), 8.46(1H,dd,J = 6Hz,2.4Hz)

Example 9

Synthesis of 2,2,2-trifluoroethyl 1,4-dihydro-2,6-dimethyl-4-(3-nitrophenyl)-5-pyridylpyridine-3-carboxylate:

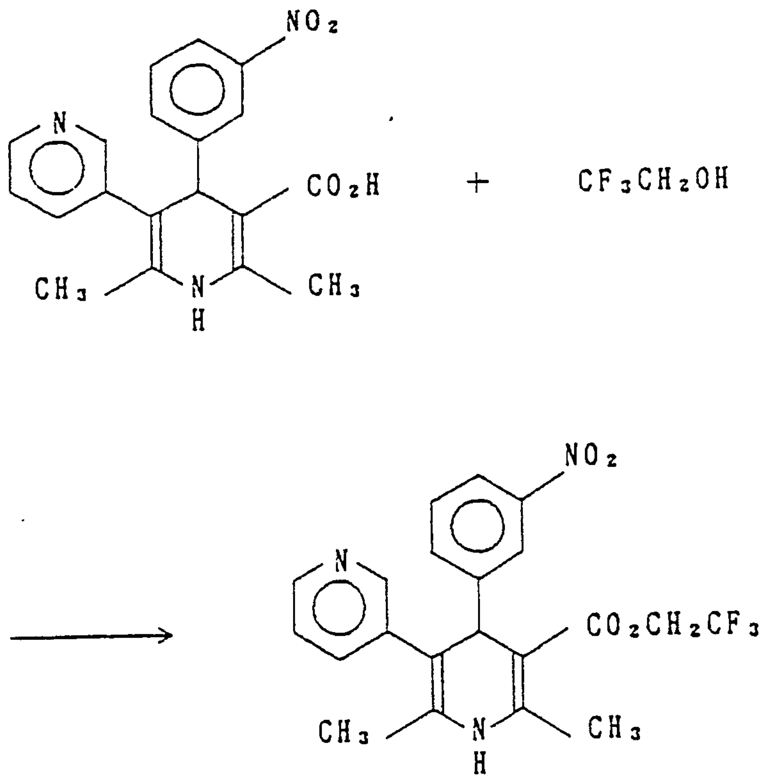


350 mg (1 mmol) of 1,4-dihydro-2,6-dimethyl-4-(3-nitrophenyl)-5-pyridylpyridine3-carboxylic acid, 100 mg (1 mmol) of 2,2,2-trifluoroethanol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 450 mg (100%).
Melting point: 152.7 to 154.3 °C
IR ($cm^{-1}$, KBr) ; ν CO 1710
ν $NO_2$ 1528, 1352

| Mass Analysis for $C_{21}H_{18}F_3N_3O_4$ | |
|---|---|
| Calculated:<br>Found: | 433.12489<br>433.12338 |

NMR (δ, $CDCl_3$); 1.88(3H,s), 2.42(3H,s), 4.27~4.50(2H,m), 4.76(1H,s), 5.84(1H,s), 7.26-(1H,dd,J=6.6Hz,4.2Hz), 7.33(1H,d,J=6.6Hz), 7.35(1H,dd,J=7.8Hz, 7.8Hz), 7.43(1H,d,J=7.8Hz), 8.03-(1H,d,J=7.8Hz), 8.07(1H,s), 8.23(1H,s), 8.45(1H,d,J=4.2Hz)

Example 10

Synthesis of 2-propene-1-yl 1,4-dihydro-2,6-dimethyl-4-(3-nitrophenyl)-5-pyridylpyridine-3-carboxylate:

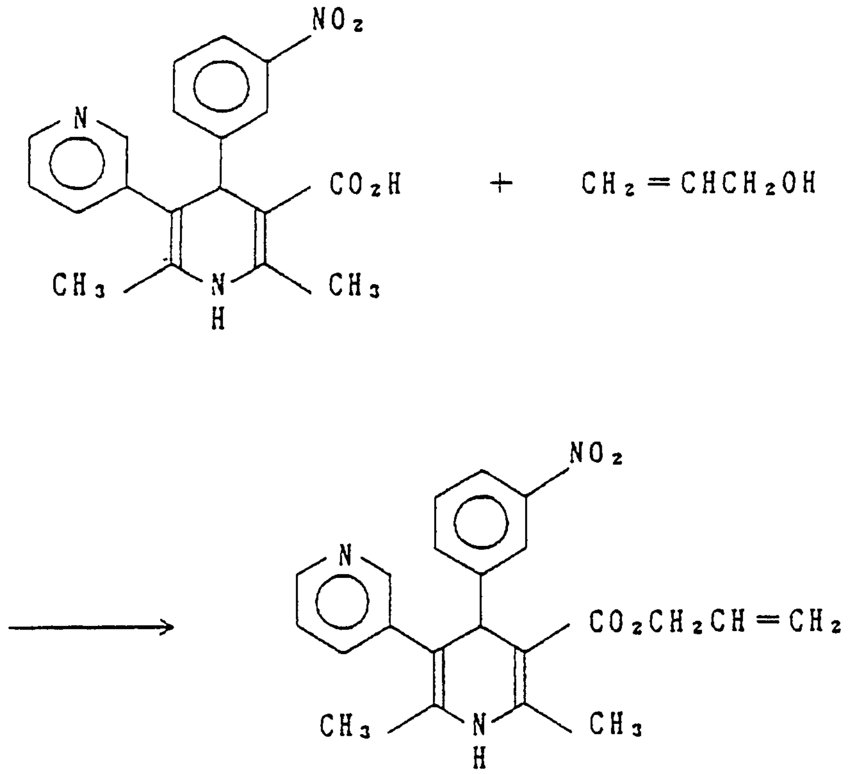

350 mg (1 mmol) of 1,4-dihydro-2,6-dimethyl-4-(3-nitrophenyl)-5-pyridylpyridine-3-carboxylic acid, 58 mg (1 mmol) of 2-propene-1-ol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for, hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 400 mg (100%).

Melting point: 141.8 to 143.6 °C

IR ($cm^{-1}$, KBr) ; ν CO 1695

ν $NO_2$ 1535, 1350

Mass Analysis for $C_{22}H_{21}N_3O_4$

Calculated: 391.15316

Found: 391.15218

NMR (δ, $CDCl_3$); 1.87(3H,s), 2.41(3H,s), 4.50(1H,dd,J=12.6Hz), 4.56(1H,dd,J=12.6Hz), 4.80(1H,s), 5.14-(1H,d,J=11Hz), 5.16(1H,d,J=19Hz), 5.63(1H,s), 5.85(1H,ddt,J=19,11,6Hz),7.16(1H,dd,J=8,5Hz), 7.25-(1H,d,J=8Hz), 7.34(1H,dd,J=8Hz,8Hz), 7.48(1H,d,J=8Hz), 8.01(1H, d,J=8Hz), 8.07(1H,s), 8.22(1H,s), 8.42-(1H,d,J=5Hz)

Example 11

Synthesis of 2-propyne-1-yl 1,4-dihydro-2,6-dimethyl-4-(3-nitrophenyl)-5-pyridylpyridine-3-carboxylate:

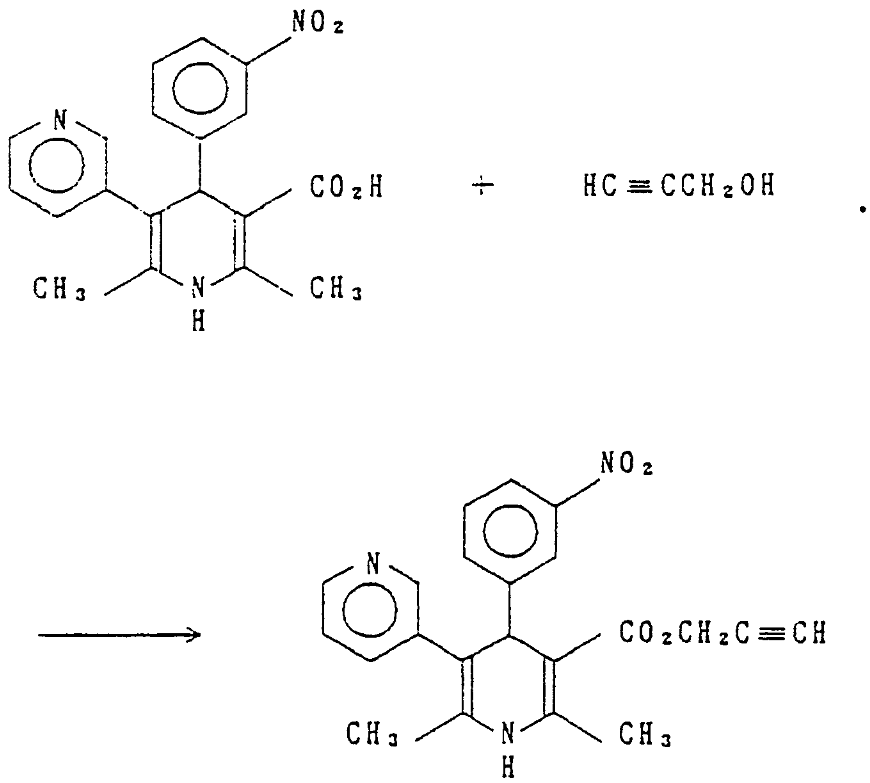

350 mg (1 mmol) of 1,4-dihydro-2,6-dimethyl-4-(3-nitrophenyl)-5-pyridylpyridine-3-carboxylic acid, 56 mg (1 mmol) of 2-propyne-1-ol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 420 mg (100%).
Melting point: 179.2 to 180.1 °C
IR ($cm^{-1}$, KBr) ; ν CO 1695
ν $NO_2$ 1530, 1350

| Mass Analysis for $C_{22}H_{19}N_3O_4$ | |
|---|---|
| Calculated: | 389.13751 |
| Found: | 389.13593 |

NMR (δ, $CDCl_3$); 1.87(3H,s), 2.39(1H,t,J=2Hz), 2.41(3H,s), 4.58(1H,dd,J=15,2Hz), 4.65(1H,dd,J=15,2Hz), 4.79(1H,s), 5.68(1H,s), 7.15(1H,dd,J=8,5Hz), 7.25(1H,dt,J=8,2Hz), 7.35(1H,dd,J=8Hz,8Hz), 7.52-(1H,d,J=8Hz), 8.01(1H,d,J=8Hz), 8.08(1H,s), 8.22(1H,s), 8.42(1H,dd,J=5,2Hz)

Example 12

Synthesis of (2E,4E)-2,4-hexadiene-1-yl 1,4-dihydro-2,6-dimethyl-4-(3-nitrophenyl)-5-pyridylpyridine-3-carboxylate:

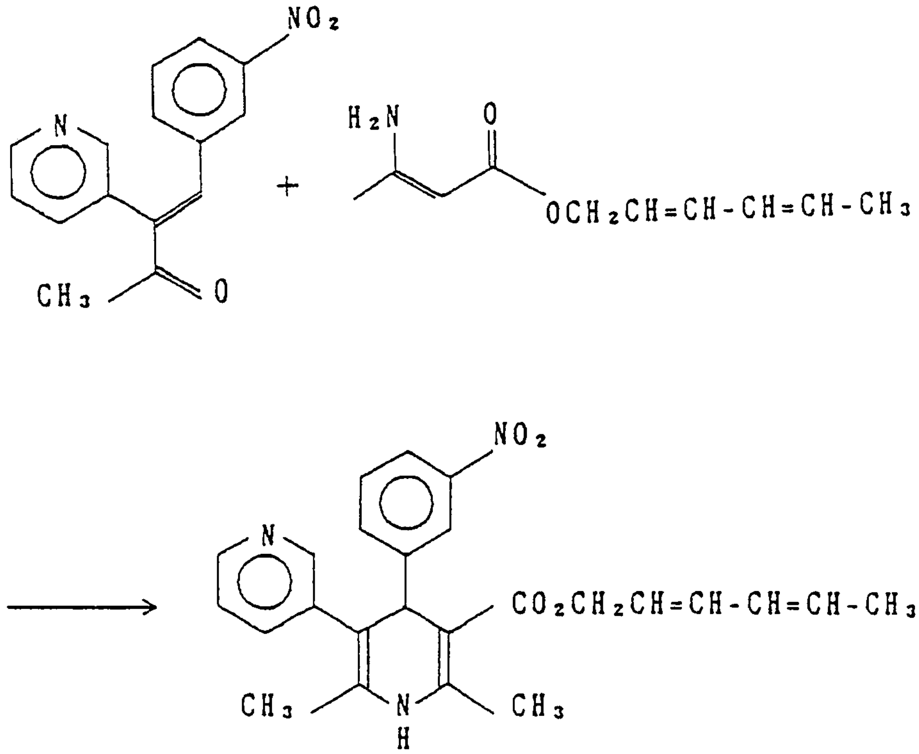

268 mg (1 mmol) of 4-(3-nitrophenyl)-3-pyridyl-3-butene-2-one, 905 mg (5 mmol) of (2E,4E)-2,4-hexadiene-1-yl 3-aminocrotonate, 273 mg (2 mmol) of zinc chloride, and 500 mg of Molecular Shieves 4A were added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 182 mg (42.3%).
Melting point: 162.3 to 164.2° C
IR ($cm^{-1}$ KBr) ; ν CO 1695
ν $NO_2$ 1525, 1350

| Mass Analysis for $C_{25}H_{25}N_3O_4$ | |
|---|---|
| Calculated: | 431.18446 |
| Found: | 431.18439 |

NMR (δ, $CDCl_3$); 1.74(3H,d,J=7Hz), 1.87(3H,s), 2.39(3H,s), 4.47(1H,dd,J=12,7Hz), 4.56(1H,dd,J=12,7Hz), 4.79(1H,s), 5.55(1H,dt,J=13,7Hz), 5.56(1H,s), 5.67(1H,dq,J=14,7Hz), 6.00(1H,dd,J=14,10Hz), 6.11-(1H,dd,J=13,10Hz), 7.20(1H,dd,J=8,5Hz) 7.28(1H,dt,J=5,2Hz), 7.34(1H,t,J=8Hz), 7.48(1H,d,J=8Hz), 8.00-(1H,d,J=8Hz), 8.06(1H,s), 8.23(1H,d,J=2Hz), 8.42(1H,dd,J=5,2Hz)

Example 13

Synthesis of (E)-3-phenyl-2-propene-1-yl 1,4-dihydro-2,6-dimethyl-4-(3-nitrophenyl)-5-pyridylpyridine-3-carboxylate:

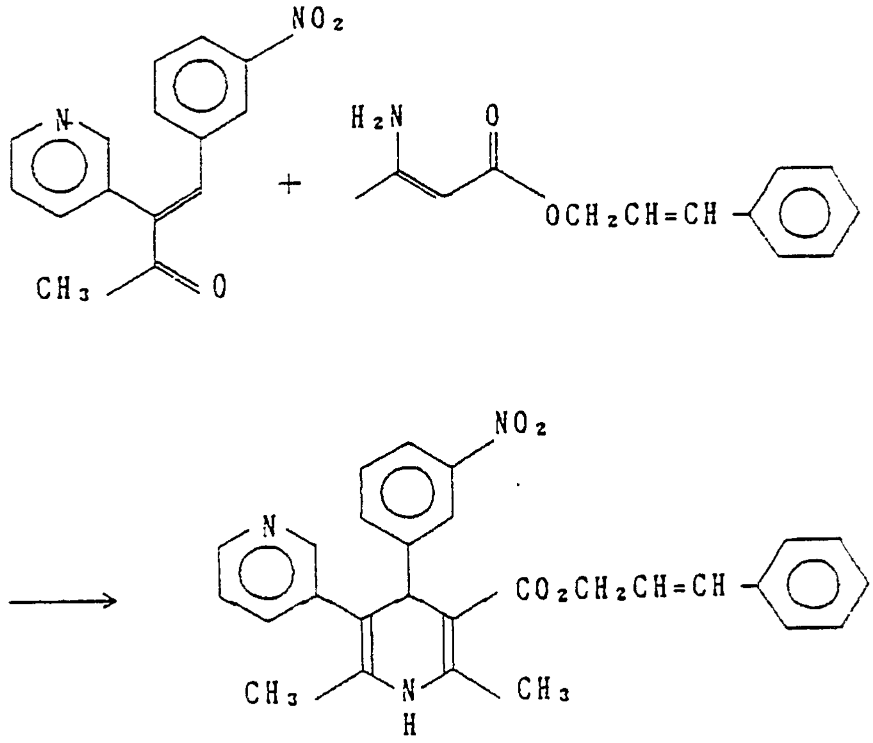

268 mg (1 mmol) of 4-(3-nitrophenyl)-3-pyridyl-3-butene-2-one, 1.08 g (5 mmol) of (E)-3-phenyl-2-propene-1-yl 3-aminocrotonate, 273 mg (2 mmol) of zinc chloride, and 500 mg of Molecular Shieves 4A were added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 315 mg (66.9%).
Melting point: 146.0 to 147.1 °C
IR ($cm^{-1}$, KBr) ; $\nu$ CO 1700
$\nu$ $NO_2$ 1530, 1350

| Mass Analysis for $C_{28}H_{25}N_3O_4$ | |
|---|---|
| Calculated: | 467.18446 |
| Found: | 467.18283 |

NMR ($\delta$, $CDCl_3$); 1.88(3H,s), 2.42(3H,s), 4.64(1H,dd,J=12,7Hz), 4.72(1H,dd,J=12,7Hz), 4.82(1H,s), 5.65-(1H,s), 6.20(1H,dt,J=15,6Hz), 6.52(1H,d,J=15Hz), 7.24(1H,dd,J=8,5Hz), 7.26~7.35(7H,m), 7.48-(1H,d,J=8Hz), 7.99(1H,d,J=8Hz), 8.08(1H,s), 8.24(1H,s), 8.42(1H,d,J=5Hz)

Example 14

Synthesis of (Z)-3-phenyl-2-propene-1-yl 1,4-dihydro-2,6-dimethyl-4-(3-nitrophenyl)-5-pyridylpyridine-3-carboxylate:

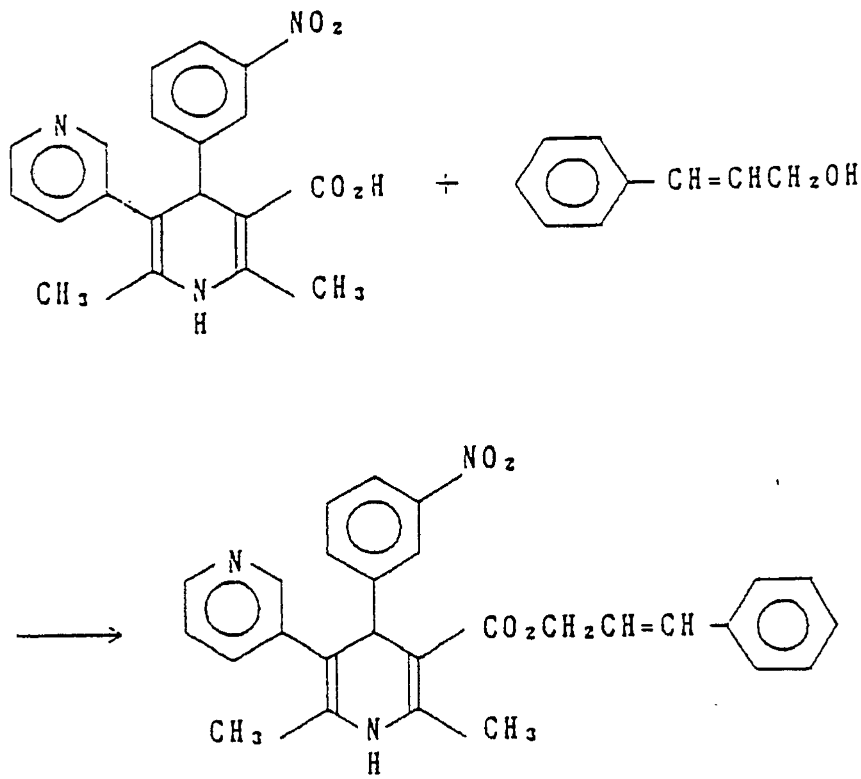

350 mg (1 mmol) of 1,4-dihydro-2,6-dimethyl-4-(3-nitrophenyl)-5-pyridylpyridine-3-carboxylic acid, 135 mg (1 mmol) of (Z)-3-phenyl-2-propene-1-ol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 467 mg (100%).

Melting point: 129.9 to 130.6° C

IR ($cm^{-1}$, KBr) ; $\nu$ CO 1698

$\nu$ $NO_2$ 1530, 1348

| Mass Analysis for $C_{28}H_{25}N_3O_4$ | |
|---|---|
| Calculated: | 467.18446 |
| Found: | 467.18401 |

NMR ($\delta$, $CDC\ell_3$); 1.90(3H,s), 2.42(3H,s), 4.73~4.88(2H,m), 4.79(1H,s), 5.70(1H,s), 5.73,(1H,dt,J=12Hz,6Hz), 6.62(1H,d,J=12Hz), 7.15(2H,d,J=4Hz), 7.23~7.41 (5H,m), 7.46~7.54(2H,m), 8.01(1H,d,J=8Hz), 8.06(1H,s), 8.28(1H,s), 8.46(1H,d,J=4.6Hz)

Example 15

Synthesis of 3-phenyl-2-propyne-1-yl 1,4-dihydro-2,6-dimethyl-4-(3-nitrophenyl)-5-pyridylpyridine-3-carboxylate:

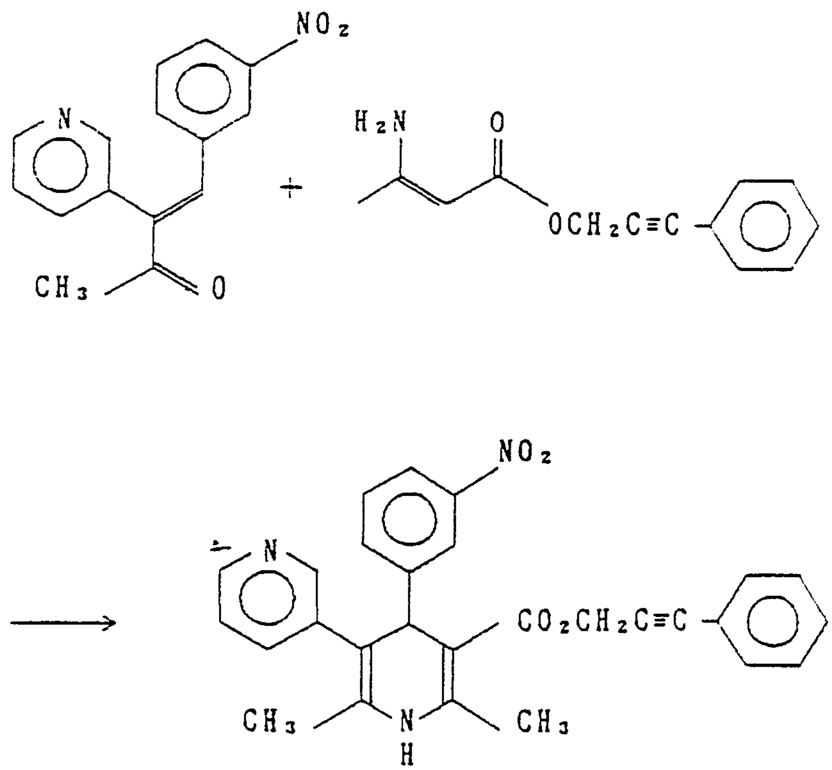

0.268 g (1 mmol) of 4-(3-nitrophenyl)-3-pyridyl-3-butene-2-one, 1.075 g (5 mmol) of 3-phenyl-2-propyne-1-yl 3-aminocrotonate, 0.273 g (2 mmol) of zinc chloride, 0.5 g of Molecular Shieves 4A were added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 0.33 g (70.9%).

Melting point: 156° C (dec.)

IR ($cm^{-1}$, KBr) ; ν CO 1700

ν $NO_2$ 1530, 1350

| Mass Analysis for $C_{28}H_{23}N_3O_4$ | |
|---|---|
| Calculated:<br>Found: | 465.16881<br>465.17159 |

NMR (δ, $CDCl_3$); 1.89(3H,s), 2.43(3H,s), 4.82(1H,d,J=15Hz), 4.84(1H,s), 4.99(1H,d,J=15Hz), 5.68(1H,s), 7.20~7.42(9H,m), 7.54(1H,d,J=8Hz), 8.00(1H,d,J=8Hz), 8.11(1H,s), 8.26(1H,s), 8.43(1H,d,J=5Hz)

Example 16

Synthesis of (2E)-5-phenyl-penta-2-ene-4-yne-1-yl 1,4-dihydro-2,6-dimethyl-4-(3-nitrophenyl)-5-pyridylpyridine-3-carboxylate:

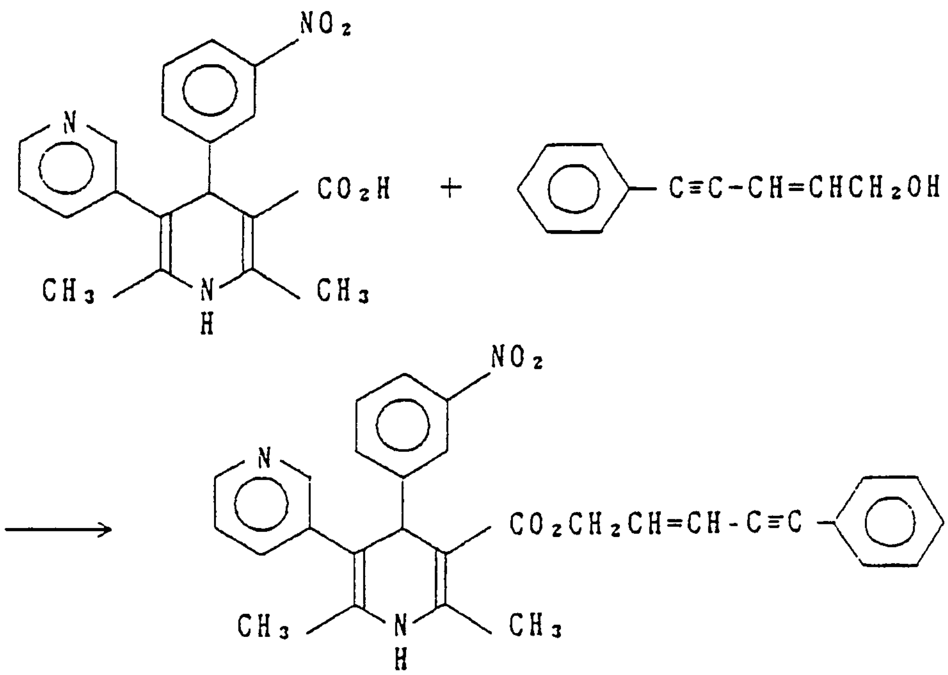

350 mg (1 mmol) of 1,4-dihydro-2,6-dimethyl-4-(3-nitrophenyl)-5-pyridylpyridine-3-carboxylic acid, 158 mg (1 mmol) of (2E)-5-phenyl-penta-2-ene-4-yne-1-ol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 475 mg (97%).

Melting point: 189.9 to 191.1 °C

IR ($cm^{-1}$, KBr) ; $\nu$ CO 1678

$\nu$ $NO_2$ 1528, 1350

| Mass Analysis for $C_{30}H_{25}N_3O_4$ | |
|---|---|
| Calculated:<br>Found: | 491.18446<br>491.18594 |

NMR (δ, $CDCl_3$); 1.91(3H,s), 2.43(3H,s), 4.58(1H,dd,J=15Hz5.4Hz), 4.65(1H,dd, J=15Hz,5.4Hz), 4.82(1H,s), 5.72(1H,s), 5.80(1H,d,J=15hz), 6.18(1H,dt,J=15hz,5.4Hz), 7.28~7.53(9H,m), 8.03(1H,d,J=8Hz), 8.08(1H,s), 8.28(1H,d,J=2.1Hz), 8.46(1H,dd,J=5.7Hz, 2.1Hz)

Example 17

Synthesis of (2E,4E)-5-phenyl-2,4-pentadiene-1-yl 1,4-dihydro-2,6-dimethyl-4-(3-nitrophenyl)-5-pyridylpyridine-3-carboxylate:

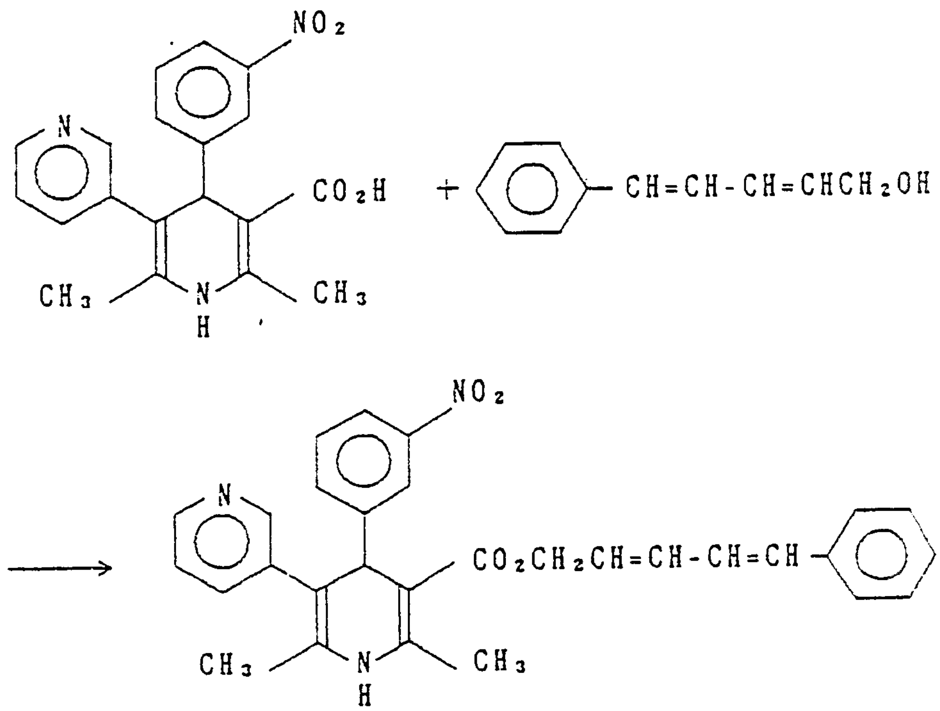

350 mg (1 mmol) of 1,4-dihydro-2,6-dimethyl-4-(3-nitrophenyl)-5-pyridylpyridine-3-carboxylic acid, 160 mg (1 mmol) of (2E,4E)-5-phenyl-2,4-pentadiene-1-ol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 377 mg (76%).

Melting point: 182° C (dec.)

IR ($cm^{-1}$, KBr) ; $\nu$ CO 1698

$\nu$ $NO_2$ 1528, 1346

Mass Analysis for $C_{30}H_{27}N_3O_4$

Calculated: 493.20011

Found: 493.19698

NMR ($\delta$, $CDCl_3$); 1.89(3H,s), 2.42(3H,s), 4.56(1H,dd,J = 14.3Hz,6.6Hz), 4.66(1H,dd,J = 14.3Hz,6.6Hz), 4.82-(1H,s), 5.65(1H,s), 5.80(1H,dt,J = 15Hz,6Hz), 6.29(1H,dd,J = 15Hz, 10.2Hz), 6.49(1H,dd,J = 15.5Hz, 10.2Hz), 6.72(1H,d,J = 15.5Hz), 7.18~7.43(8H,m), 7.50(1H,d,J = 8.3Hz), 8.02(1H,d,J = 8.3Hz), 8.09(1H,s) 8.26-(1H,d,J = 2.4Hz), 8.44(1H,dd,J = 4.8Hz, 2.4Hz)

Example 18

Synthesis of (E)-3-[4-(1-imidazolylmethyl)phenyl]-2-propene-1-yl 1,4-dihydro-2,6-dimethyl-4-(3-nitrophenyl)-5-pyridylpyridine-3-carboxylate:

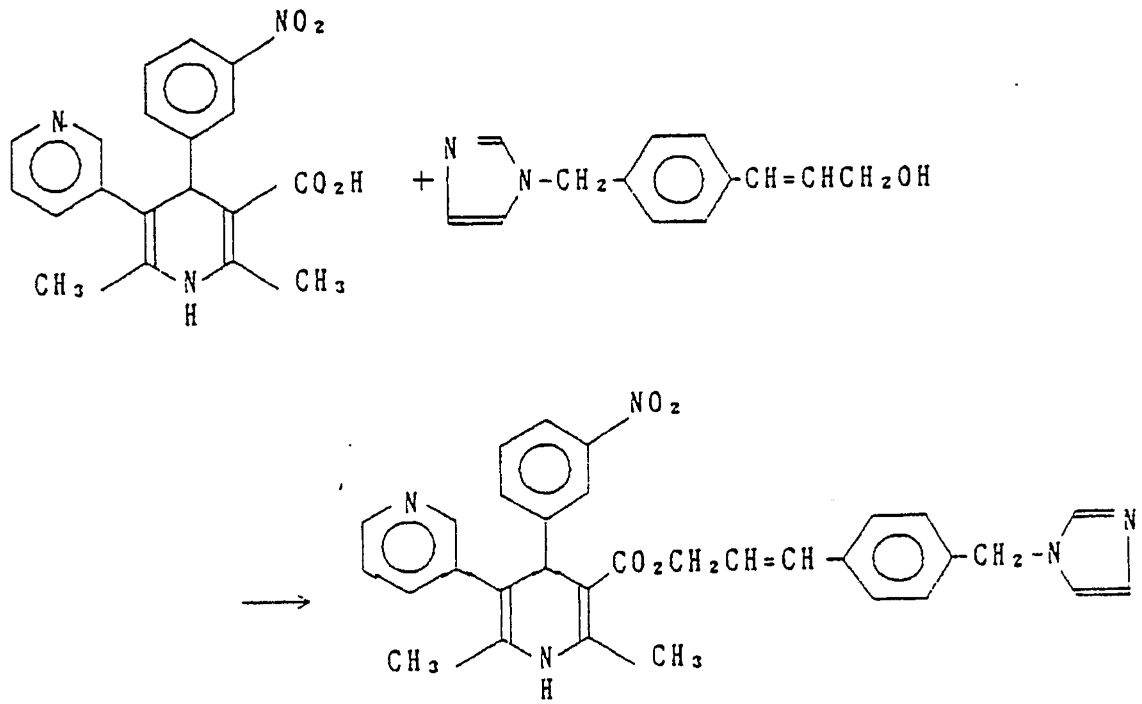

350 mg (1 mmol) of 1,4-dihydro-2,6-dimethyl-4-(3-nitrophenyl)-5-pyridylpyridine-3-carboxylic acid, 214 mg (1 mmol) of (E)-3-[4-(1-imidazolylmethyl)phenyl]-2-propene-1-ol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 530 mg (97%).

Melting point: oil

IR (cm$^{-1}$, KBr) ; $\nu$ CO 1692

$\nu$ $NO_2$ 1528, 1350

| Mass Analysis for $C_{32}H_{29}N_5O_4$ | |
|---|---|
| Calculated:<br>Found: | 547.22190<br>547.2213 |

NMR ($\delta$, $CDCl_3$); 1.86(3H,s), 2.43(3H,s), 4.61(1H,dd,J=12.6Hz,6Hz), 4.74(1H,dd,J=12.6Hz,6Hz), 4.81(1H,s), 5.19(2H,s), 5.63(1H,s), 6.20(1H,dt,J=16.2Hz,6.3Hz), 6.47(1H,d,J=16.2Hz), 6.98(1H,s), 7.32~7.36(8H,m), 7.50(1H,d,J=7.8Hz), 7.97(1H,d,J=7.8Hz), 8.05(2H,s), 8.22(1H,d,J=2.1Hz), 8.41(1H,dd,J=5.1Hz,2.1Hz)

Example 19

Synthesis of (Z)-3-[4-(1-imidazolylmethyl)phenyl]-2-propene-1-yl 1,4-dihydro-2,6-dimethyl-4-(3-nitrophenyl)-5-pyridylpyridine-3-carboxylate:

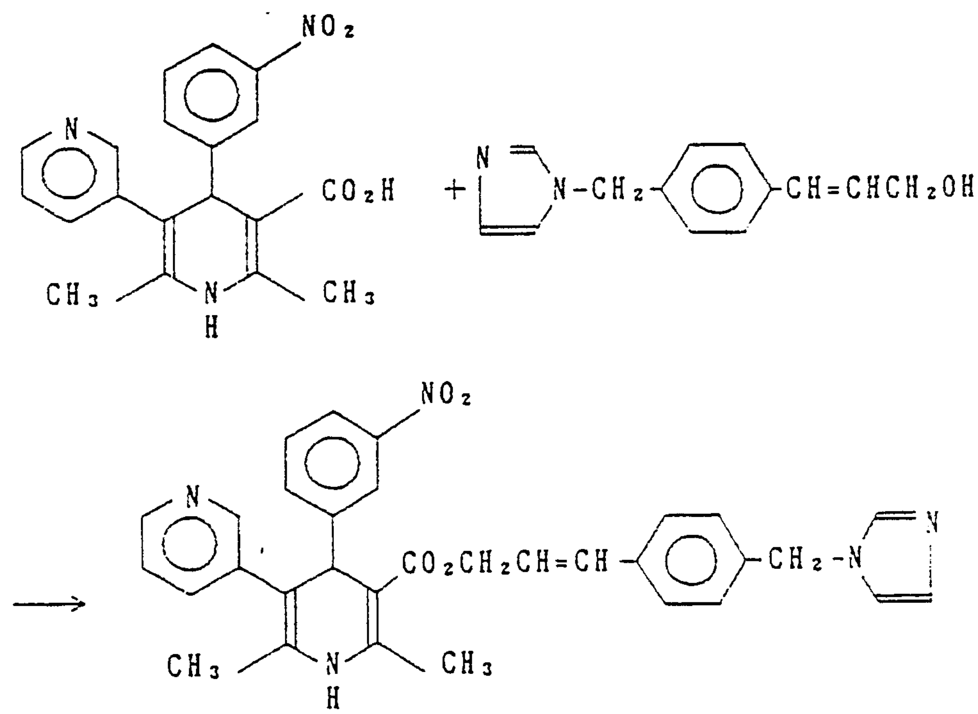

210 mg (0.6 mmol) of 1,4-dihydro-2,6-dimethyl-4-(3-nitrophenyl)-5-pyridylpyridine-3-carboxylic acid, 107 mg (0.5 mmol) of (Z)-3-[4-(1-imidazolylmethyl)phenyl]-2-propene-1-ol, 155 mg (0.75 mmol) of N,N-dicyclohexylcarbodiimide, 67 mg (0.55 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 273 mg (100%).
Melting point: oil
IR ($cm^{-1}$, KBr) ; $\nu$ CO 1692
$\nu$ $NO_2$ 1532, 1350

| Mass Analysis for $C_{32}H_{29}N_5O_4$ | |
|---|---|
| Calculated:<br>Found: | 547.22190<br>547.2246 |

NMR ($\delta$, $CDC\ell_3$); 1.86(3H,s), 2.38(3H,s), 4.74(1H,d,J=6Hz), 4.75(1H,d,J=6Hz), 4.77(1H,s), 5.20(2H,s), 5.80-(1H,dt,J=11.7Hz,6Hz), 6.57(1H,d,J=11.7Hz), 7.00(1H,s), 7.07~7.40(7H,m), 7.33(1H,t,J=7.8Hz), 7.48-(1H,d,J=7.8Hz), 7.97(1H,d,J=7.8Hz), 8.02(1H,s), 8.06(1H,s), 8.21(1H,s), 8.42(1H,d,J= 2.7Hz)

Example 20

Synthesis of (E)-3-[(5-imidazolylmethyl)thiophene-2-yl]-2-propene-yl 1,4-dihydro-2,6-dimethyl-4-(3-nitrophenyl)-5-pyridylpyridine-3-carboxylate:

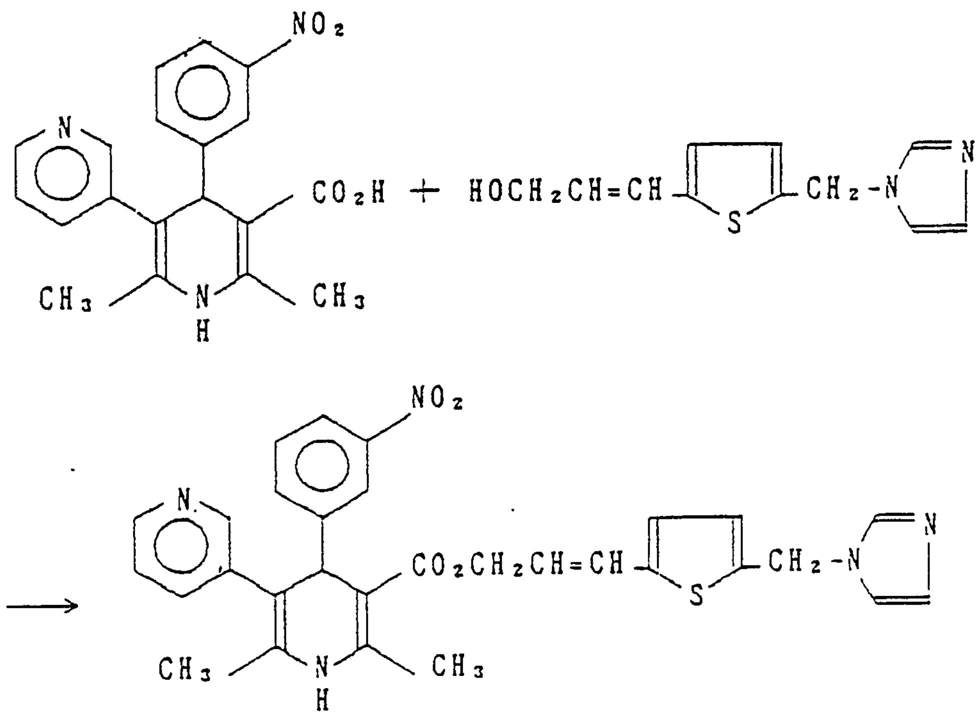

350 mg (1 mmol) of 1,4-dihydro-2,6-dimethyl-4-(3-nitrophenyl)-5-pyridylpyridine-3-carboxylic acid, 220 mg (1 mmol) of (E)-3-[(5-imidazolylmethyl)thiophene-2-yl]-2-propene-1-ol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 400 mg (72.4%).
Melting point: 200.4 to 201.7 °C
IR ($cm^{-1}$, KBr) ; ν CO 1698
ν $NO_2$ 1530, 1350

| Mass Analysis for $C_{30}H_{27}N_5O_4S$ | |
|---|---|
| Calculated: | 553.17832 |
| Found: | 553.17860 |

NMR (δ, $CDCl_3$); 1.84(3H,s), 2.41(3H,s), 4.54(1H,dd,J=12,3Hz), 4.67(1H,dd,J=12,3Hz), 5.20(2H,s), 5.74-(1H,s), 5.91(1H,dt,J=15,6Hz), 6.49(1H,d,J=15Hz), 6.74(1H,d,J=4Hz), 6.81(1H,d,J=4Hz), 6.96(1H,s), 7.08-(1H,s), 7.15(1H,dd,J=7,5Hz), 7.24(1H,dt,J=7.2Hz), 7.31(1H,t,J=8Hz), 7.46(1H,d,J=8Hz), 7.56(1H,s), 7.97-(1H,d,J=8Hz), 8.05(1H,s), 8.20(1H,s), 8.41 (1H,dd,J=5.2Hz)

Example 21

Synthesis of 2-[4-(diphenylmethyl)piperazinyl]ethyl 1,4-dihydro-2,6-dimethyl-4-(3-nitrophenyl)-5-pyridylpyridine-3-carboxylate:

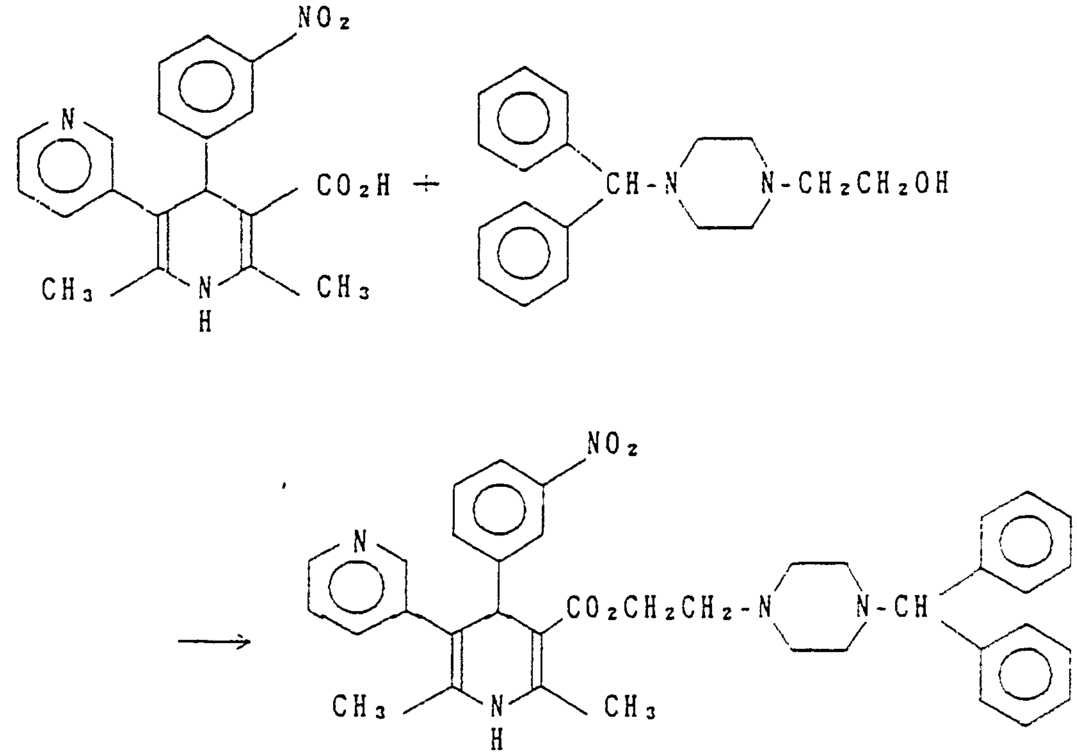

350 mg (1 mmol) of 1,4-dihydro-2,6-dimethyl-4-(3-nitrophenyl)-5-pyridylpyridine-3-carboxylic acid, 327 mg (1.1 mmol) of 2-[4-(diphenylmethyl)piperazinyl]ethanol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 563 mg (89.4%).

Melting point: 171.8 to 173.3 °C

IR (cm$^{-1}$, KBr) ; $\nu$ CO 1695

$\nu$ $NO_2$ 1530, 1350

| Mass Analysis for $C_{38}H_{39}N_5O_4$ | |
|---|---|
| Calculated: | 629.30015 |
| Found: | 629.29894 |

NMR ($\delta$, $CDC\ell_3$); 1.85(3H,s), 2.30~2.48(6H,m), 2.39(3H,s), 2.53~2.60(4H,m), 4.05~4.21(2H,m), 4.17(1H,s), 4.75(1H,s), 5.55(1H,s), 7.13(1H,dd,J=8,5Hz), 7.17~7.29(11H,m), 7.39(1H,d,J=8Hz), 7.45(1H,d,J=8Hz), 7.96-(1H,d,J=8Hz), 8.03(1H,s), 8.20(1H,s), 8.42(1H,d,J=5Hz)

Example 22

Synthesis of 2-(4-phenylpiperazinyl)ethyl 1,4-dihydro-2,6-dimethyl-4-(3-nitrophenyl)-5-pyridylpyridine-3-carboxylate:

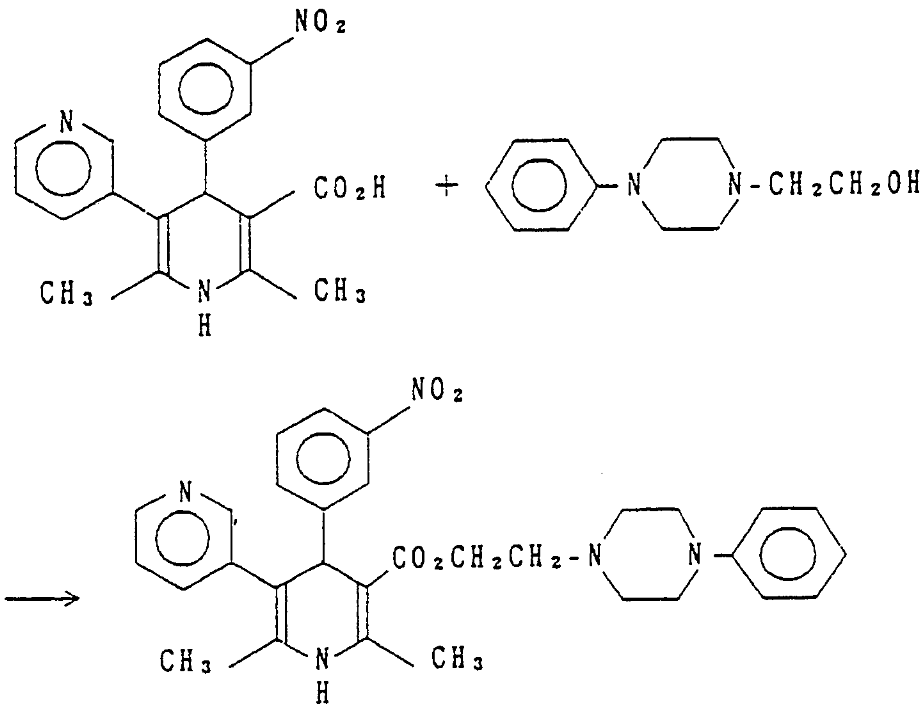

350 mg (1 mmol) of 1,4-dihydro-2,6-dimethyl-4-(3-nitrophenyl)-5-pyridylpyridine-3-carboxylic acid, 206 mg (1 mmol) of 2-(4-phenylpiperazinyl)ethanol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 369 mg (68.5%).

Melting point: oil

IR ($cm^{-1}$, KBr) ; $\nu$ CO 1695

$\nu$ $NO_2$ 1530, 1350

| Mass Analysis for $C_{31}H_{33}N_5O_4$ | |
|---|---|
| Calculated: | 539.25320 |
| Found: | 539.25105 |

NMR ($\delta$, $CDCl_3$); 1.86(3H,s), 2.41(3H,s), 2.56~2.63(6H,m) 3.12(4H,t,J=5Hz), 4.15(1H,dt,J=11,6Hz), 4.22-(1H,dt,J=11,6Hz), 4.79(1H,s), 5.53(1H,s), 6.85(1H,t,J=8Hz), 6.90(2H,d,J=8Hz), 7.14(1H,dd,J=8,5Hz), 7.22~7.29(3H,m), 7.34(1H,t,J=8Hz), 7.50(1H,d,J=8Hz), 8.00(1H,d,J=8Hz), 8.06(1H,s), 8.22(1H,d,J=2Hz), 8.42(1H,dd,J=5,2Hz)

Example 23

Synthesis of 2-(4-phenylpiperidino)ethyl 1,4-dihydro-2,6-dimethyl-4-(3-nitrophenyl)-5-pyridylpyridine-3-carboxylate:

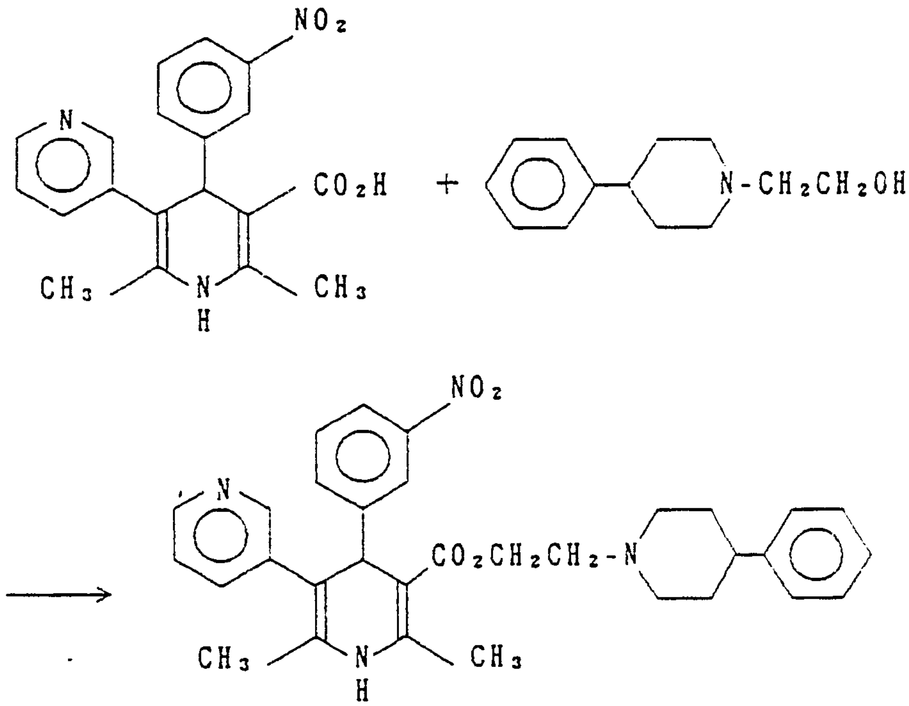

350 mg (1 mmol) of 1,4-dihydro-2,6-dimethyl-4-(3-nitrophenyl)-5-pyridylpyridine-3-carboxylic acid, 205 mg (1 mmol) of 2-(4-phenylpiperidino)ethanol, 309 mg (1 mmol) of N,N-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column; whereby the captioned compound was obtained. The yield was 220 mg (41%).
Melting point: oil
IR ($cm^{-1}$, KBr) ; $\nu$ CO 1696
$\nu$ $NO_2$ 1530, 1350

| Mass Analysis for $C_{32}H_{34}N_4O_4$ | |
|---|---|
| Calculated:<br>Found: | 538.25795<br>538.25683 |

NMR ($\delta$, $CDCl_3$); 1.60~1.82(4H,m), 1.86(3H,s), 2.06~2.16(2H,m) 2.42(3H,s), 2.45~2.50(1H,m), 2.55~2.64-(2H,m), 2.92~3.02(2H,m), 4.09~4.26(2H,m), 4.80(1H,s), 5.58(1H,s), 7.06(1H,dd,J=8,5Hz), 7.18~7.29(6H,m), 7.35 (1H,t, J=9Hz), 7.39(1H,d,J=9Hz), 8.01(1H,d,J=9Hz), 8.06(1H,s), 8.22(1H,d,J=2Hz), 8.42-(1H,dd,J=5,2Hz)

Example 24

Synthesis of 2-(N-benzyl-N-methylamino)ethyl 1,4-dihydro-2,6-dimethyl-4-(3-nitrophenyl)-5-pyridylpyridine-3-carboxylate:

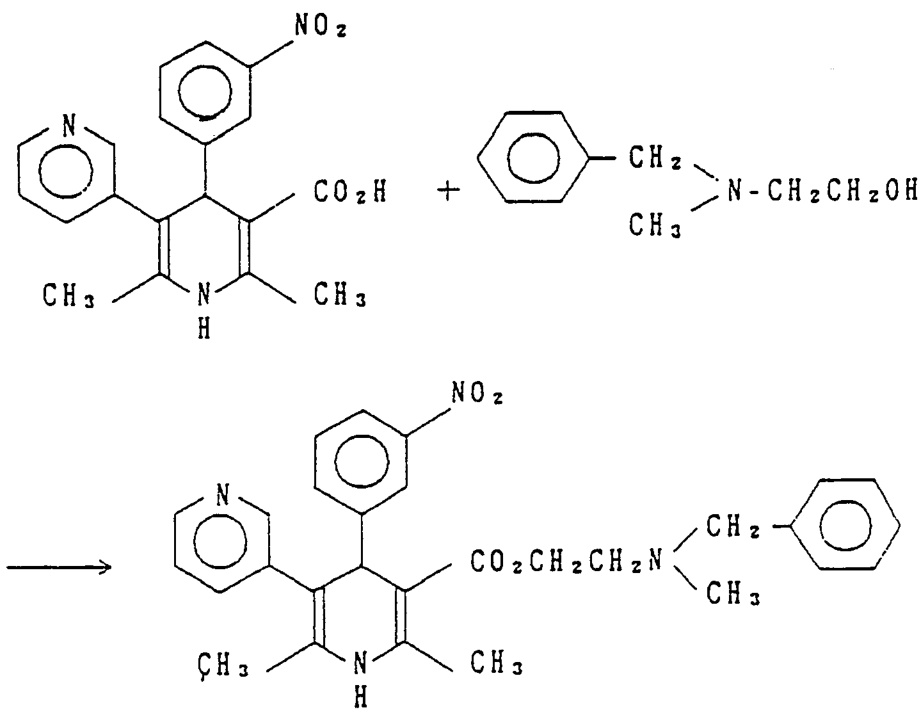

350 mg (1 mmol) of 1,4-dihydro-2,6-dimethyl-4-(3-nitrophenyl)-5-pyridylpyridine-3-carboxylic acid, 182 mg (1.1 mmol) of 2-(N-benzyl-N-methylamino)ethanol, 309 mg (1 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate.
The solvents contained in the reaction mixture were then distilled off and the residue was chromatographed on a silica gel column, whereby the captioned compound was obtained. The yield was 403 mg (80.9%).
Melting point: oil
IR ($cm^{-1}$, KBr) ; $\nu$ CO 1695
$\nu$ $NO_2$ 1530, 1350

| Mass Analysis for $C_{29}H_{30}N_4O_4$ | |
|---|---|
| Calculated: | 498.22666 |
| Found: | 498.22511 |

NMR ($\delta$, $CDC\ell_3$); 1.86(3H,s), 2.19(3H,s), 2.38(3H,s), 2.55(1H,dd,J=15,6Hz), 3.47(2H,s), 4.13(2H,t,J=6Hz), 4.78(1H,s), 5.55(1H,s), 7.16(1H,dd,J=7,5Hz); 7.22~7.30(7H,m), 7.44(1H,d,J=7Hz), 7.98(1H,d,J=8Hz), 8.05-(1H,s), 8.22(1H,s), 8.43(1H,d,J=5Hz)

Example 25

Synthesis of 2-(1,2,3,4-tetrahydroisoquinoline-2-yl)ethyl 1,4-dihydro-2,6-dimethyl-4-(3-nitrophenyl)-5-pyridylpyridine-3-carboxylate:

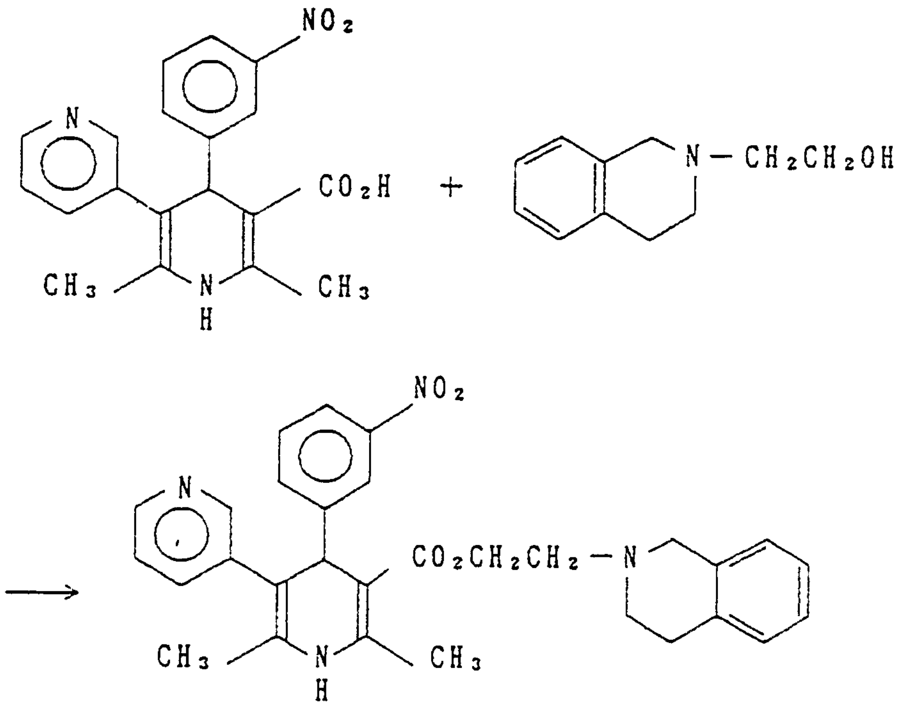

350 mg (1 mmol) of 1,4-dihydro-2,6-dimethyl-4-(3-nitrophenyl)-5-pyridylpyridine-3-carboxylic acid, 177 mg (1 mmol) of 2-(1,2,3,4-tetrahydroisoquinoline-2-yl)ethanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 378 mg (74%).
Melting point: 166° C (decomposed)
IR ($cm^{-1}$, KBr) ; ν CO 1692
ν $NO_2$ 1532, 1348

| Mass Analysis for $C_{30}H_{30}N_4O_4$ | |
|---|---|
| Calculated:<br>Found: | 510.22666<br>510.22467 |

NMR (δ, $CDCl_3$); 1.86(3H,s), 2.41(3H,s), 2.74~2.92(6H,m), 3.64~3.73(2H,m), 4.24(1H,dt,J=12Hz,6Hz), 4.29-(1H,dt,J=12Hz,6Hz), 4.81(1H,s) , 5.63(1H,s), 6.95(1H,d,J=7.5Hz), 7.06~7.30(5H,m), 7.19(1H,t,J=7Hz), 7.50-(1H,d,J=7Hz), 7.94(1H,d,J=7Hz), 8.04(1H,s), 8.21(1H,d,J=2.4Hz), 8.42(1H,dd,J=5Hz,2.4Hz)

Example 26

Synthesis of 2-(3,7-dihydro-3,7-dimethyl-1H-purine-2,6-dione-1-yl)ethyl 1,4-dihydro-2,6-dimethyl-4-(3-nitrophenyl)-5-pyridylpyridine-3-carboxylate:

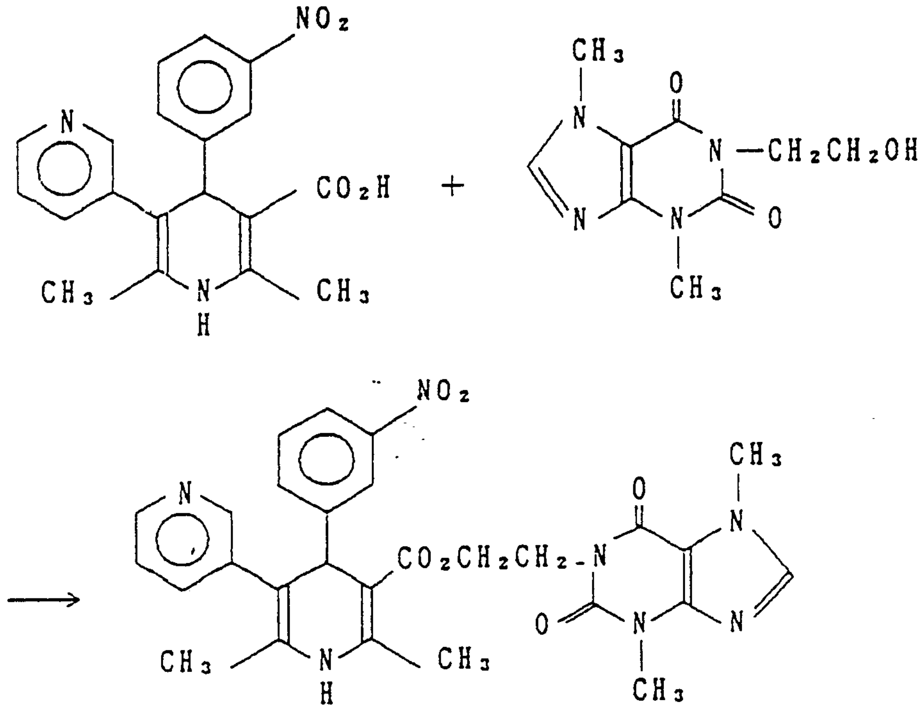

350 mg (1 mmol) of 1,4-dihydro-2,6-dimethyl-4-(3-nitrophenyl)-5-pyridylpyridine-3-carboxylic acid, 224 mg (1 mmol) of 2-(3,7-dihydro-3,7-dimethyl-1H-purine-2,6-dione-1-yl)ethanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 529 mg (95%).
Melting point: 198.1 to 199.9 °C
IR ($cm^{-1}$, KBr) ; ν CO 1708,1704,1668
ν $NO_2$ 1530, 1352

| Mass Analysis for $C_{28}H_{27}N_7O_6$ | |
|---|---|
| Calculated:<br>Found: | 557.20223<br>557.20372 |

NMR (δ, $CDCl_3$); 1.89(3H,s), 2.37(3H,s), 3.48(3H,s), 3.87(3H,s), 4.12~4.27(2H,m), 4.33~4.49(2H,m), 4.80(1H,s), 5 66(1H,s), 7.22(1H,dd,J=7Hz,2Hz), 7.28(1H,t,J=7.5Hz), 7.37(1H,d,J=7.5Hz), 7.48(1H,s), 7.52(1H,d,J=7Hz), 7.95(1H,d,J=7.5Hz), 8.07(1H,s), 8.27(1H,s), 8.43(1H,d,J=2Hz)

Example 27

Synthesis of 2-(1,2,3,6-tetrahydro-1,3-dimethyl-2,6-dioxo-7H-purine-7-yl)ethyl 1,4-dihydro-2,6-dimethyl-4-(3-nitrophenyl)-5-pyridylpyridine-3-carboxylate:

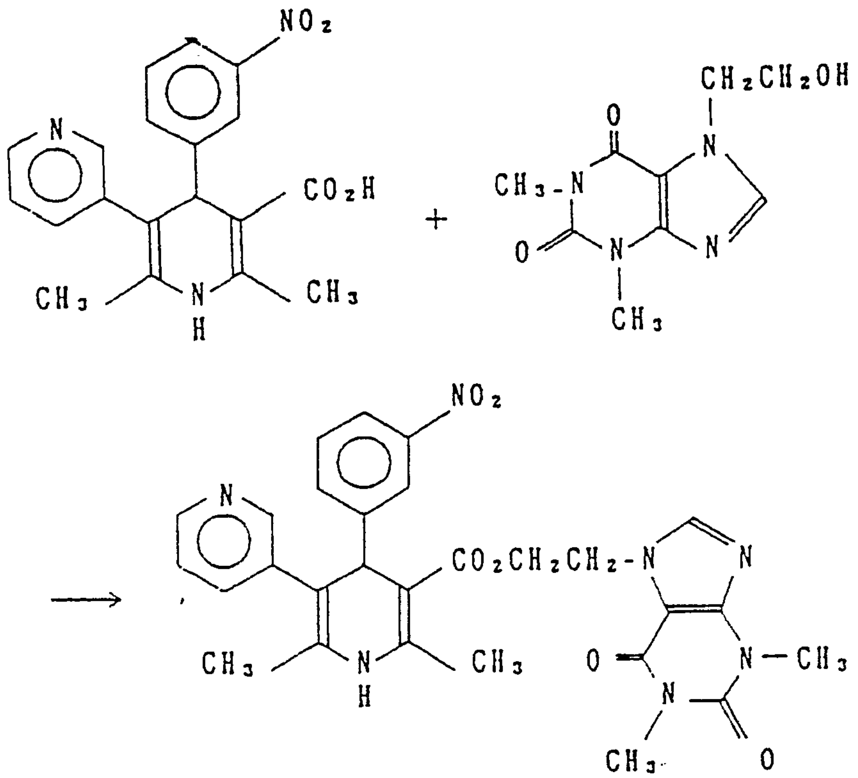

350 mg (1 mmol) of 1,4-dihydro-2,6-dimethyl-4-(3-nitrophenyl)-5-pyridylpyridine-3-carboxylic acid, 224 mg (1 mmol) of 2-(1,2,3,6-tetrahydro-1,3-dimethyl-2,6-dioxo-7H-purine-7-yl)ethanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 514 mg (92%).

Melting point: 198.6 to 200.4° C

IR ($cm^{-1}$, KBr) ; ν CO 1700, 1652

ν $NO_2$ 1528, 1350

Mass Analysis for $C_{28}H_{27}N_7O_6$

Calculated: 557.20223

Found: 557.20254

NMR (δ, $CDCl_3$); 1.87(3H,s), 2.34(3H,s), 3.38(3H,s), 3.56(3H,s), 4.35~4.58 (4H,m), 4.69(1H,s), 5.74(1H,s), 7.11(1H,s), 7.35~7.51(4H,m), 7.89(1H,s), 8.04(1H,d,J=6Hz), 8.25(1H,s), 8.47(1H,d,J=5Hz)

Example 28

Synthesis of methyl 4-(2,3-dichlorophenyl)-1,4-dihydro-2,6-dimethyl-5-pyridylpyridine-3-carboxylate:

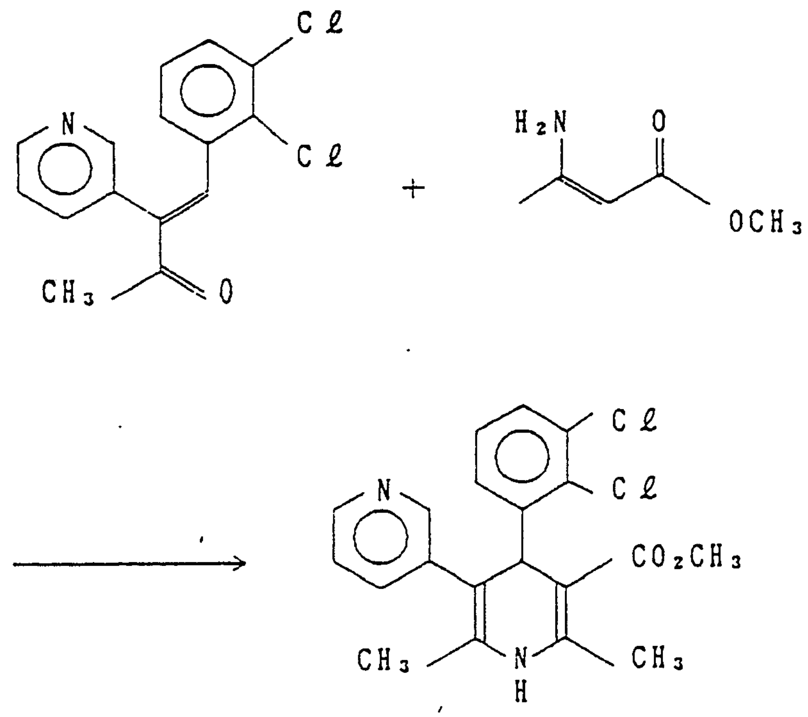

A mixuture of 292 mg (1 mmol) of 4-(2,3-dichlorophenyl)-3-pyridyl-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 668 mg (85.9%).
Melting point: 158.0 to 159.3$^\circ$ C
IR ($cm^{-1}$, KBr) ; $\nu$ CO 1700

| Mass Analysis for $C_{20}H_{18}N_2O_2C\ell_2$ | |
|---|---|
| Calculated: | 388.07450 |
| Found: | 388.0756 |

NMR ($\delta$, $CDC\ell_3$); 1.69(3H,s), 2.41(3H,s), 3.53(3H,s), 5.30(1H,s), 5.41(1H,s), 7.10~7.26(4H,m), 7.39-(1H,dd,J=8,2Hz), 8.12(1H,s), 8.41(1H,d,J=4Hz),

Example 29

Synthesis of methyl 1,4-dihydro-2,6-dimethyl-5-pyridyl-4-(3-trifluoromethylphenyl)pyridine-3-carboxylate:

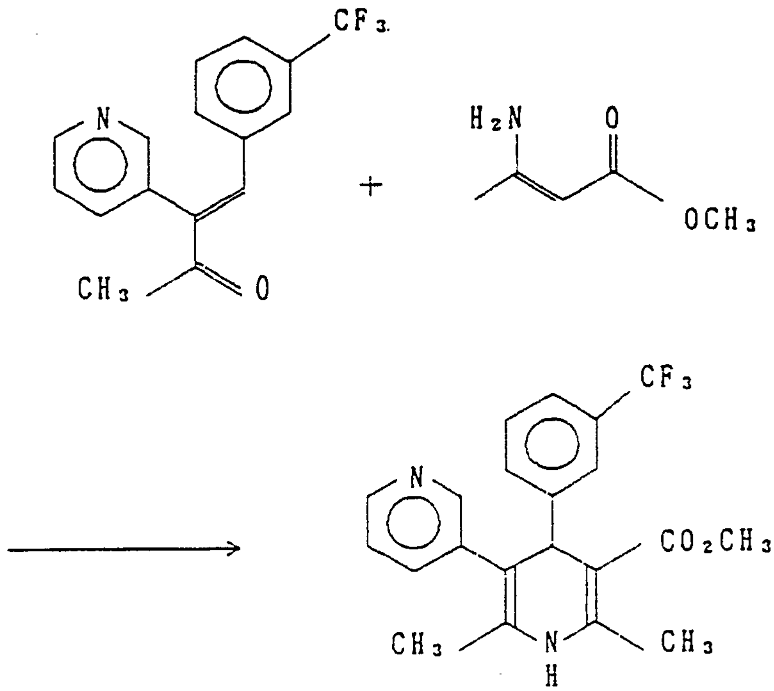

A mixuture of 291 mg (1 mmol) of 3-pyridyl-4-(3-trifluoromethylphenyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 361 mg (93.2%).
Melting point: 139 to 140.5 °C
IR ($cm^{-1}$, KBr) ; ν CO 1700

| Mass Analysis for $C_{21}H_{19}F_3N_2O_2$ | |
|---|---|
| Calculated:<br>Found: | 388.13981<br>388.14156 |

NMR (δ, $CDCl_3$); 1.85(3H,s), 2.38(3H,s), 3.61(3H,s), 4.70(1H,s), 5.53(1H,s), 7.15(1H,dd,J=8,5Hz), 7.24 (1H,dt,J=8,2Hz), 7.32~7.41 (4H,m), 8.23(1H,d,J=2Hz), 8.41(1H,dd,J=5,2Hz)

Example 30

Synthesis of methyl 4-(2-cyanophenyl)-1,4-dihydro-2,6-dimethyl-5-pyridylpyridine-3-carboxylate:

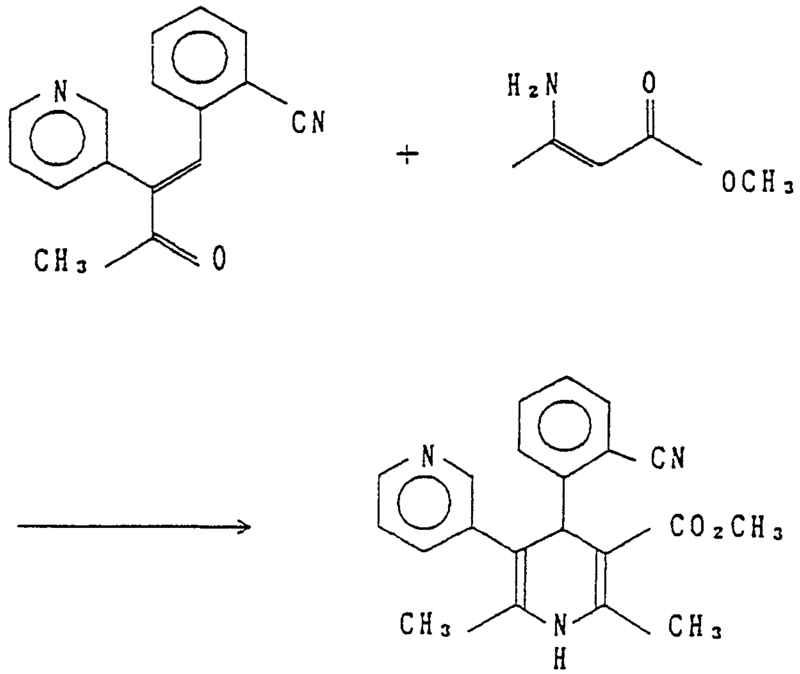

A mixuture of 248 mg (1 mmol) of 4-(2-cyanophenyl)-3-pyridyl-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained: The yield was 245 mg (71.2%).
Melting point: 142.8 to 143.9° C
IR ($cm^{-1}$, KBr) ; ν CN 2225
ν CO 1700

| Mass Analysis for $C_{20}H_{19}N_3O_2$ | |
|---|---|
| Calculated: | 345.14768 |
| Found: | 345.14727 |

NMR (δ, $CDCl_3$); 1.74(3H,s), 2.41(3H,s), 3.55(3H,s), 5.12(1H,s), 5.52(1H,s), 7.16~7.21(2H,m), 7.38-(1H,dt,J=8Hz, 2Hz), 7.41~7.50(3H,m), 8.01(1H,d,J=2Hz), 8.40(1H,dd,J=5,2Hz)

Example 31

Synthesis of methyl 1,4-dihydro-2,6-dimethyl-4-(2-furyl)-5-pyridylpyridine-3-carboxylate:

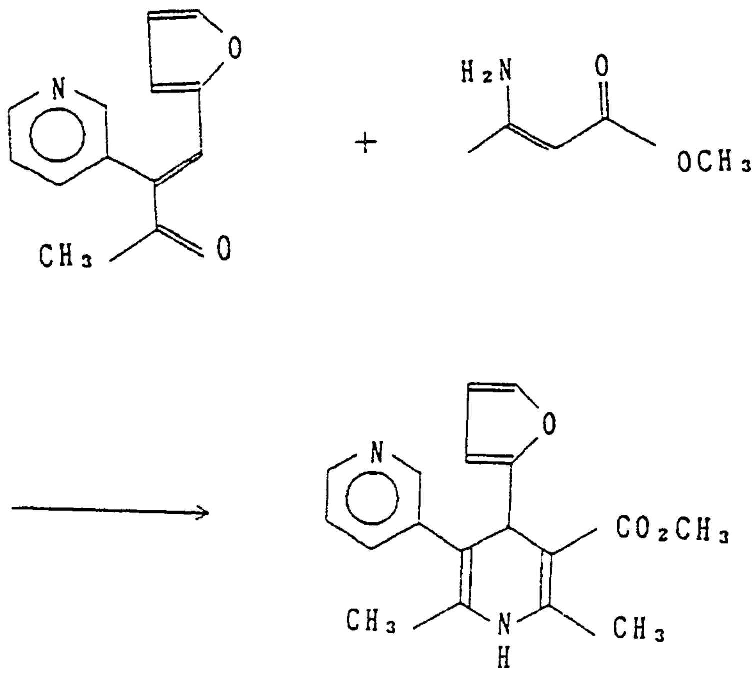

A mixuture of 213 mg (1 mmol) of 4-(2-furyl)-3-pyridyl-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 230 mg (74.6%).
Melting point: 161.5 to 162.8° C
IR ($cm^{-1}$, KBr) ; ν CO 1700

| Mass Analysis for $C_{18}H_{18}N_2O_3$ | |
|---|---|
| Calculated:<br>Found: | 310.13171<br>310.13160 |

NMR (δ, $CDCl_3$); 1.87(3H,s), 2.38(3H,s), 3.66(3H,s), 4.78(1H,s), 5.69(1H,s), 5.93(1H,d,J=3Hz), 6.22-(1H,dd,J=2,3Hz), 7.20(1H,dd,J=8,4Hz), 7.26(1H,d,J=2Hz), 7.42(1H,d,J=8Hz), 8.37(1H,s), 8.43-(1H,d,J=4Hz)

Example 32

Synthesis of methyl 1,4-dihydro-2,6-dimethyl-5-pyridyl-4-thienylpyridine-3-carboxylate:

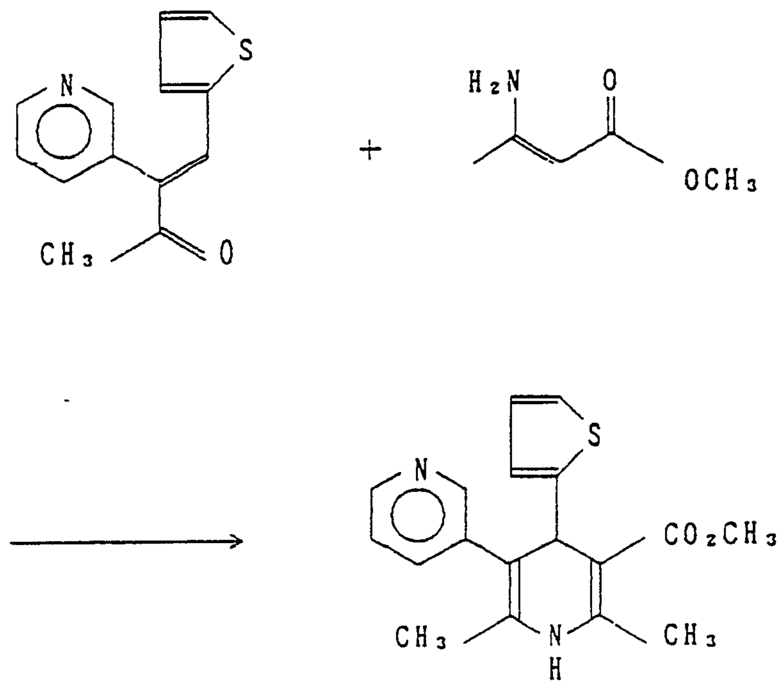

A mixuture of 229 mg (1 mmol) of 3-pyridyl-4-thienyl-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 173 mg (53%).

Melting point: 198.0 to 199.0° C

IR ($cm^{-1}$, KBr) ; $\nu$ CO 1670

| Mass Analysis for $C_{18}H_{18}N_2O_2S$ | |
|---|---|
| Calculated: | 326.10886 |
| Found: | 326.10847 |

NMR ($\delta$, CDC$\ell_3$); 1.93(3H,s), 2.35(3H,s), 3.69(3H,s), 4.69(1H,s), 5.66(1H,s), 6.75(1H,d,J=4Hz), 6.85-(1H,dd,J=6,4Hz), 7.08(1H,dd,J=6Hz), 7.18(1H,dd,J=7,5Hz), 7.43(1H,d,J=7Hz) 8.41(1H,s), 8.43-(1H,d,J=5Hz)

Example 33

Synthesis of methyl 1,4-dihydro-2,6-dimethyl-4,5-dipyridylpyridine-3-carboxylate:

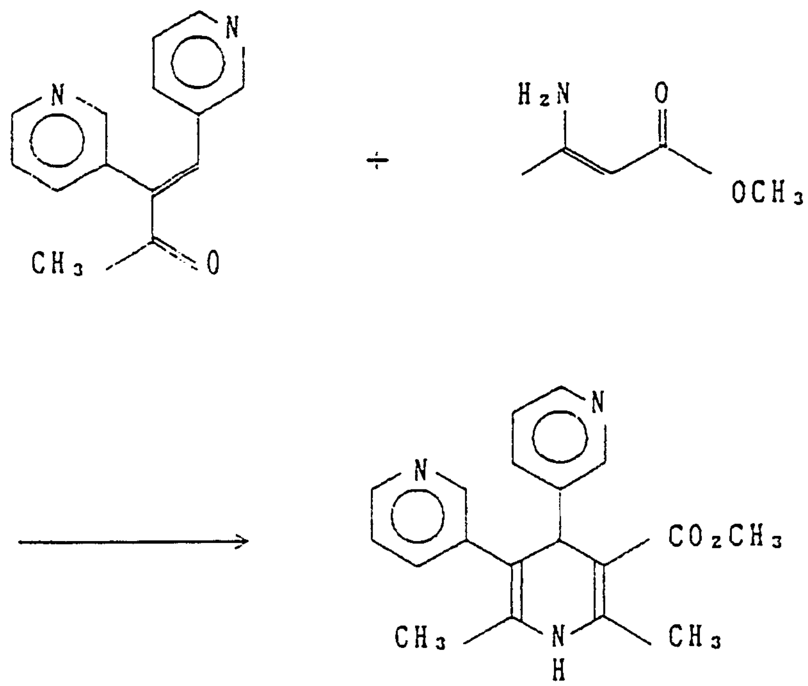

A mixuture of 224 mg (1 mmol) of 3,4-dipyridyl-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 269 mg (83.8%).
Melting point: 189.7 to 190.8° C
IR ($cm^{-1}$, KBr) ; ν CO 1700

| Mass Analysis for $C_{19}H_{19}N_3O_2$ | |
| --- | --- |
| Calculated: | 321.14768 |
| Found: | 321.14711 |

NMR (δ, $CDCl_3$); 1.84(3H,s), 2.37(3H,s), 3.61(3H,s), 4.65(1H,s), 5.94(1H,s), 7.14(1H,t,J=7Hz), 7.15-(1H,dd,J=8,6Hz), 7.26(1H,d,J=7Hz), 7.49(1H,d,J=8Hz), 8.26(1H,s), 8.39(1H,d,J=6Hz), 8.41(1H,d,J=7Hz), 8.44(1H,s)

Example 34

Synthesis of methyl 1,4-dihydro-2,6-dimethyl-5-pyridyl-4-(4-quinolyl)pyridine-3-carboxylate:

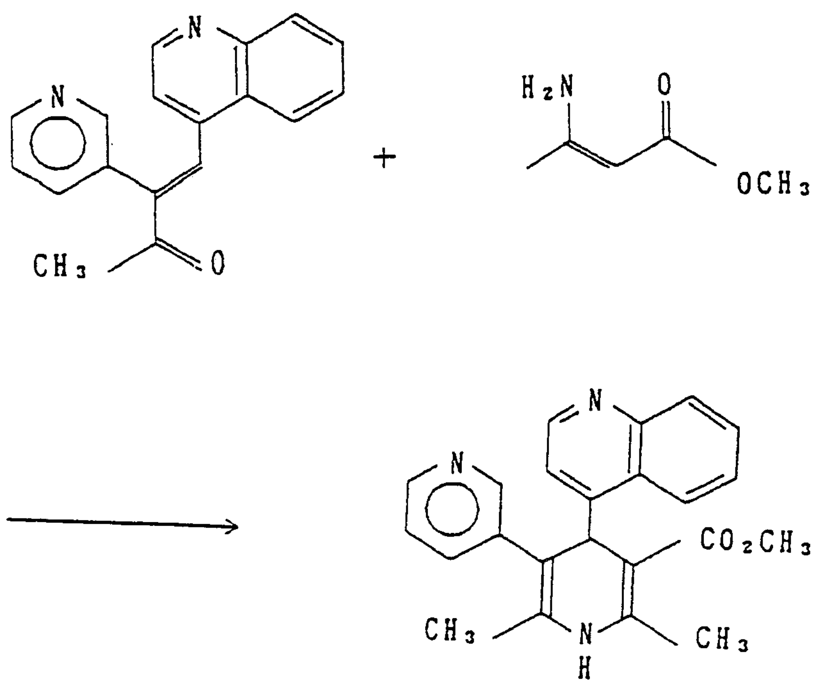

A mixuture of 274 mg (1 mmol) of 3-pyridyl-4-(4-quinolyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 324 mg (87.5%).
Melting point: 189 °C (decomposed)
IR ($cm^{-1}$, KBr) ; $\nu$ CO 1695

| Mass Analysis for $C_{23}H_{21}N_3O_2$ | |
|---|---|
| Calculated:<br>Found: | 371.16333<br>371.16350 |

NMR ($\delta$, $CDCl_3$); 1.76(3H,s) 2.46(3H,s), 3.39(3H,s), 5.51(1H,s), 5.63(1H,s), 6.94(1H,dd,J=7,5Hz), 6.99-(1H,dt,J=7,2Hz), 7.31(1H,t,J=8Hz), 7.49(1H,d,J=5Hz), 7.56(1H,t,J=8Hz), 7.89(1H,d,J=8Hz), 8.00-(1H,d,J=8Hz), 8.17(1H,s), 8.30(1H,dd,J=5,2Hz), 8.84(1H,d,J=5Hz)

Example 35

Synthesis of 3-cyano-1,4-dihydro-2,6-dimethyl-4-(3-nitrophenyl)-5-pyridylpyridine:

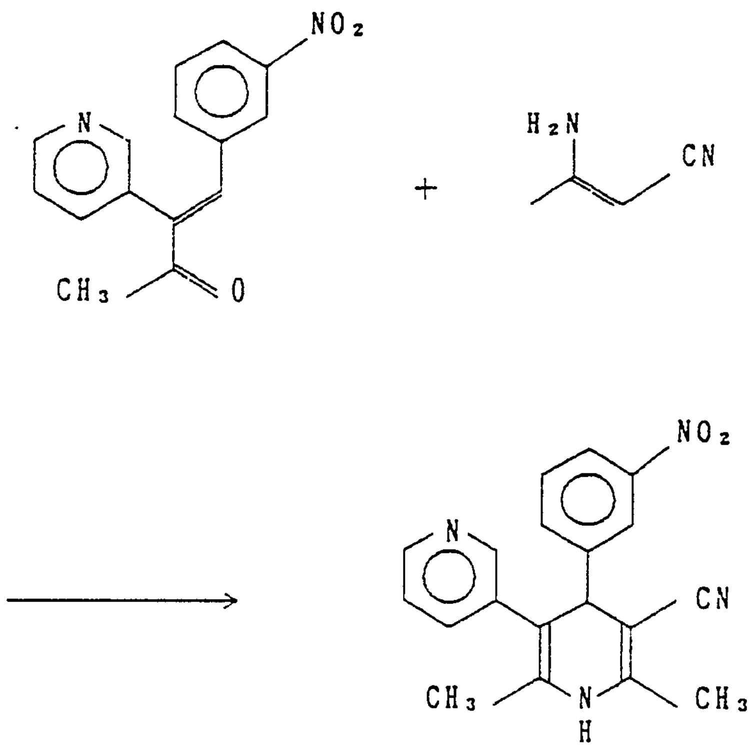

A mixuture of 0.536 g (2 mmol) of 4-(3-nitrophenyl)-3-pyridyl-3-butene-2-one, 0.821 g (10 mmol) of 3-aminocrotonitrile, 0.546 g (4 mmol) of zinc chloride, and 1.0 g of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 0.59 g (84.6%).
Melting point: 202.8 to 204.3 ° C
IR ($cm^{-1}$, KBr) ; ν CN 2192
ν $NO_2$ 1530, 1350

| Mass Analysis for $C_{19}H_{16}N_4O_2$ | |
|---|---|
| Calculated:<br>Found: | 332.12729<br>332.12685 |

NMR (δ,$CDCl_3$) ; 1.84(3H,s), 2.17(3H,s), 4.53(1H,s), 5.81(1H,s), 7.19(1H,dd,J=8,5Hz), 7.28(1H,dt,J=8,2Hz), 7.42(1H,d,J=2Hz), 7.44(1H,s), 8.07(1H,t,J=2Hz), 8.09(1H,d,J=2Hz), 8.22(1H,d,J=2Hz), 8.42-(1H,dd,J=5,2Hz)

Example 36

Synthesis of 3-acetyl-1,4dihydro-2,6-dimethyl-4-(3-nitrophenyl)-5-pyridylpyridine:

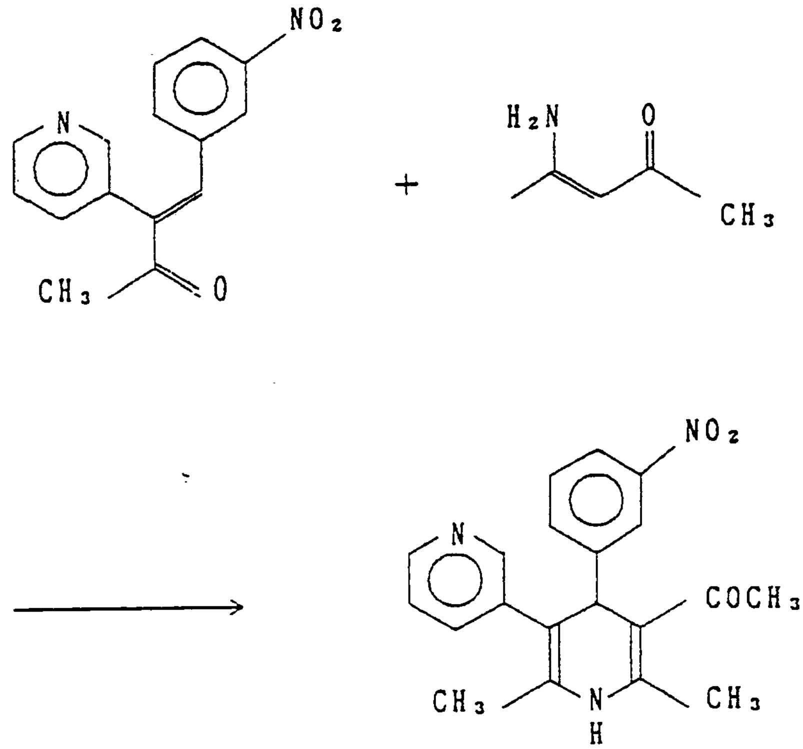

A mixuture of 268 mg (1 mmol) of 4-(3-nitrophenyl)-3-pyridyl-3-butene-2-one, 495 mg (5 mmol) of 4-amino-3-pentene-2-one, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 121 mg (34.7%).

Melting point: 160.7 to 161.9 °C

IR ($cm^{-1}$, KBr) ; $\nu$ $NO_2$ 1530, 1350

| Mass Analysis for $C_{20}H_{19}N_3O_3$ | |
|---|---|
| Calculated: | 349.14260 |
| Found: | 349.14312 |

NMR ($\delta$, $CDC\ell_3$); 1.86(3H,s), 1.98(3H,s), 2.42(3H,s), 4.86(1H,s), 5.96(1H,s), 7.22(1H,dd,J=8,5Hz), 7.31-(1H,d,J=8Hz), 7.35(1H, t,J=8Hz), 7.43(1H,d,J=8Hz), 8.00(1H,s), 8.01(1H,d,J=8Hz), 8.23(1H,s), 8.45-(1H,d,J=5Hz)

Example 37

Synthesis of 3-benzoyl-1,4-dihydro-2,6-dimethyl-4-(3-nitrophenyl)-5-pyridylpyridine:

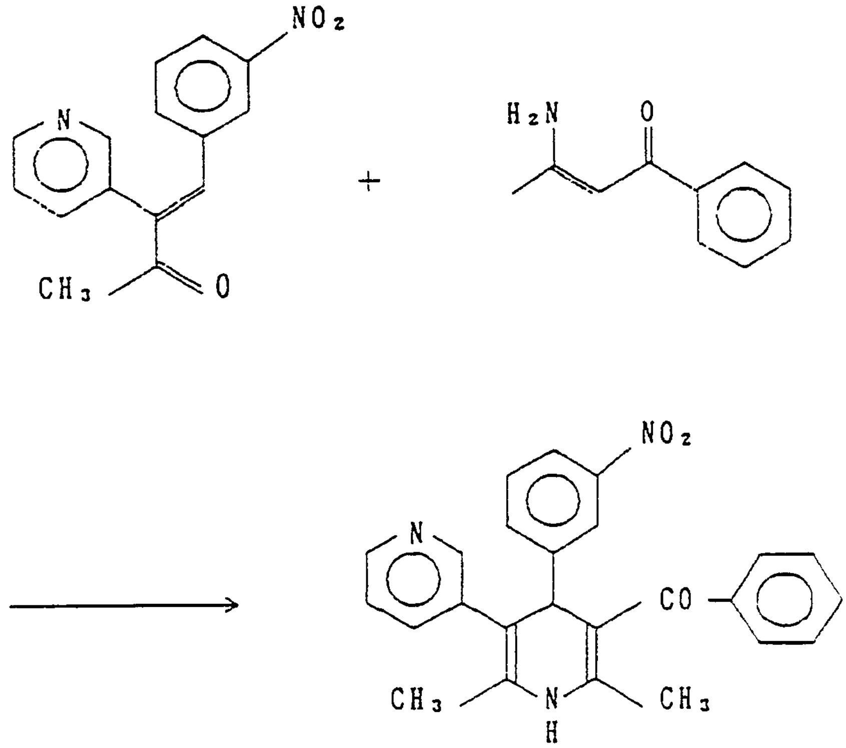

A mixuture of 0.536 g (2 mmol) of 4-(3-nitrophenyl)-3-pyridyl-3-butene-2-one, 0.161 g (10 mmol) of 3-amino-1-phenyl-2-butene-1-one, 0.546 g (4 mmol) of zinc chloride, and 1 g of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 0.424 g (51.7%).
Melting point: 172.9 to 173.8 °C
IR ($cm^{-1}$, KBr) ; $\nu$ $NO_2$ 1530, 1350

| Mass Analysis for $C_{25}H_{21}N_3O_3$ | |
|---|---|
| Calculated:<br>Found: | 411.15825<br>411.15588 |

NMR ($\delta$, $CDCl_3$); 1.85(3H,s), 1.98(3H,s), 4.99(1H,s), 5.71(1H,s), 7.25~7.47(8H,m), 7.48(1H,d,J=8Hz), 8.00-(1H,d,J=8Hz), 8.06(1H,s), 8.32(1H,s), 8.43(1H,d,J=5Hz)

Example 38

Synthesis of methyl 5-(6-chloropyridyl)-1,4-dihydro-2,6-dimethyl-4-(3-nitrophenyl)pyridine-3-carboxylate:

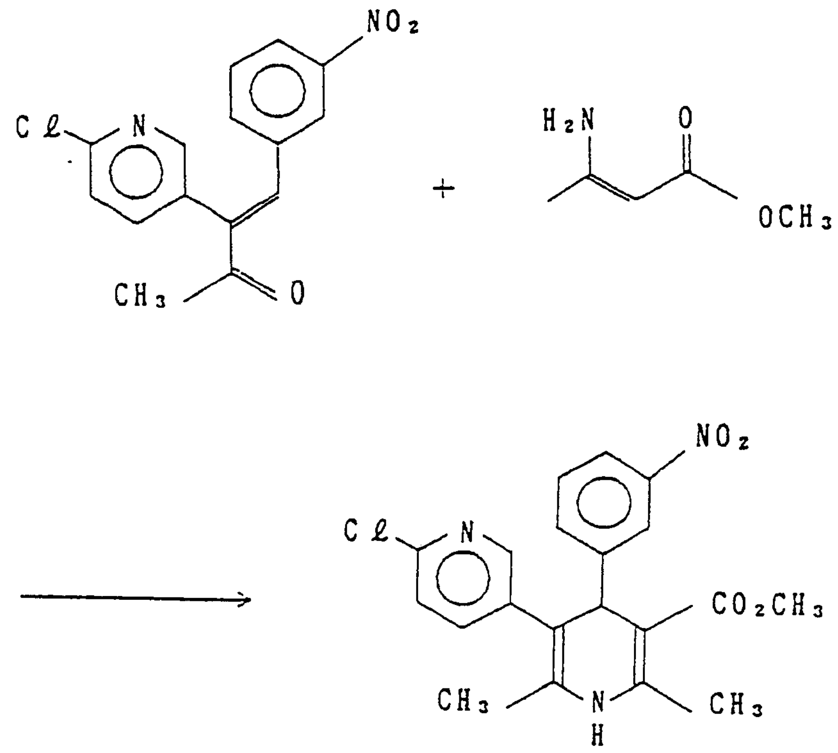

A mixuture of 303 mg (1 mmol) of 3-(6-chloropyridyl)-4-(3-nitrophenyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 229 mg (57.5%).

Melting point: 122.5 to 124.0° C

IR ($cm^{-1}$, KBr) ; $\nu$ CO 1680

$\nu$ $NO_2$ 1530, 1350

| Mass Analysis for $C_{20}H_{18}C\ell N_3O_4$ | |
|---|---|
| Calculated:<br>Found: | 399.09854<br>399.09642 |

NMR ($\delta$, $CDC\ell_3$); 1.86(3H,s), 2.39(3H,s), 3.62(3H,s), 4.74(1H,s), 5.58(1H,s), 7.19(1H,d,J=7Hz), 7.22-(1H,d,J=7Hz), 7.36(1H,t,J=8Hz), 7.45(1H,d,J=8Hz), 7.99(1H,s), 8.02(1H,d,J=8Hz) 8.06(1H,s)

Example 39

Synthesis of methyl 5-(2-chloropyridyl)-1,4-dihydro-2,6-dimethyl-4-(3-nitrophenyl)pyridine-3-carboxylate:

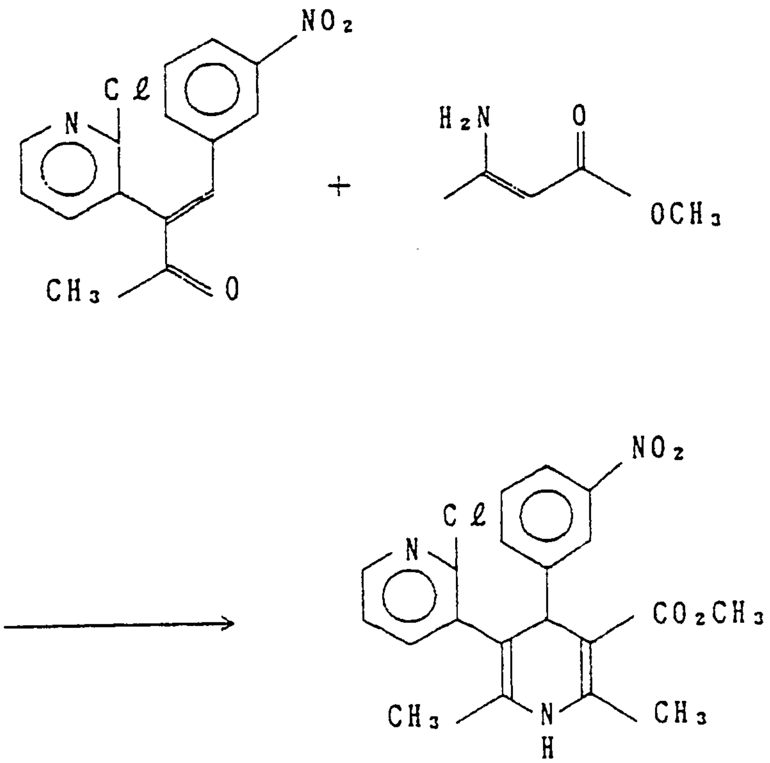

A mixuture of 303 mg (1 mmol) of 3-(2-chloropyridyl)-4-(3-nitrophenyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 204 mg (51%).

Melting point: oil

IR ($cm^{-1}$, KBr) ; $\nu$ CO 1698

$\nu$ $NO_2$ 1532, 1350

| Mass Analysis for $C_{20}H_{18}C\ell N_3O_4$ | |
|---|---|
| Calculated: | 399.09547 |
| Found: | 399.10099 |

NMR ($\delta$, $CDC\ell_3$): measured at a temperature of 100°C, using cyclosilan-$d_{18}$ ($\delta$ = -0.327) as an internal standard.

1.58(3H,s), 2.31(3H,s), 3.43(3H,s), 4.73(1H,s), 6.90~7.33(2H,m), 7.33~7.48(2H,m), 7.75(1H,s), 7.91-(1H,d,J=8Hz), 8.19(2H,m)

Example 40

Synthesis of methyl 1,4-dihydro-2,6-dimethyl-5-(6-methoxypyridyl)-4-(3-nitrophenyl)pyridine-3-carboxylate:

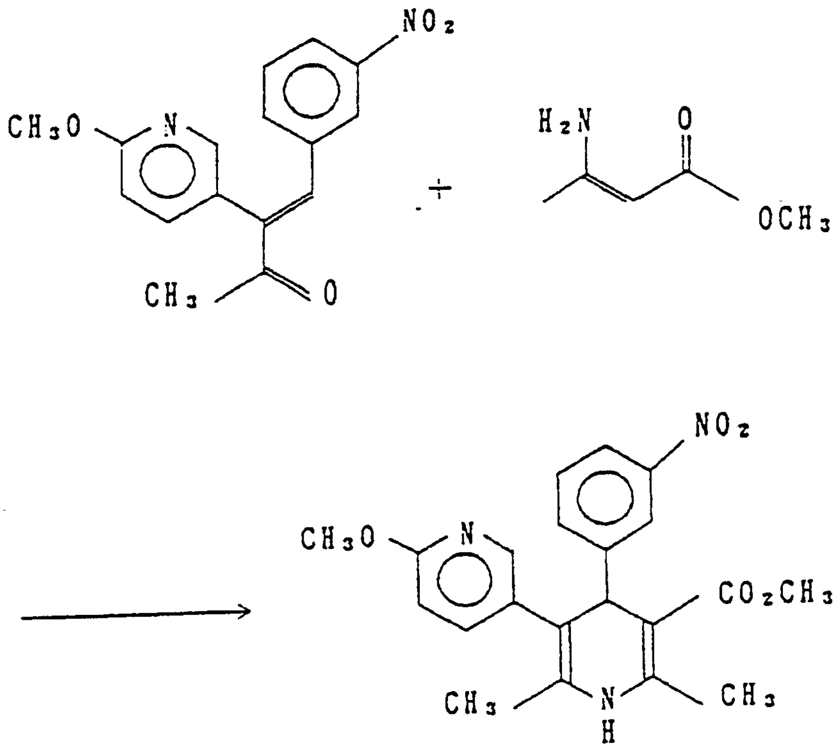

A mixuture of 298 mg (1 mmol) of 3-(6-methoxypyridyl)-4-(3-nitrophenyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 290 mg (74.5%).

Melting point: oil

IR ($cm^{-1}$, KBr) ; ν CO 1695

ν $NO_2$ 1530, 1350

| Mass Analysis for $C_{21}H_{21}N_3O_5$ | |
|---|---|
| Calculated: | 395.14808 |
| Found: | 395.14987 |

NMR (δ, $CDCl_3$); 1.85(3H,s), 2.38(3H,s), 3.60(3H,s), 3.88(3H,s), 4.72(1H,s), 5.51(1H,s), 6.62(1H,d,J=8Hz), 7.16(1H,dd,J=8,2Hz), 7.34(1H,t,J=8Hz), 7.48(1H,d,J=8Hz), 7.71(1H,d,J=2Hz), 8.00(1H,d,J=8Hz), 8.06-(1H,s)

Example 41

Synthesis of methyl 1,4-dihydro-2,6-dimethyl-5-(2-methoxyphenyl)-4-(3-nitrophenyl) pyridine-3-carboxylate:

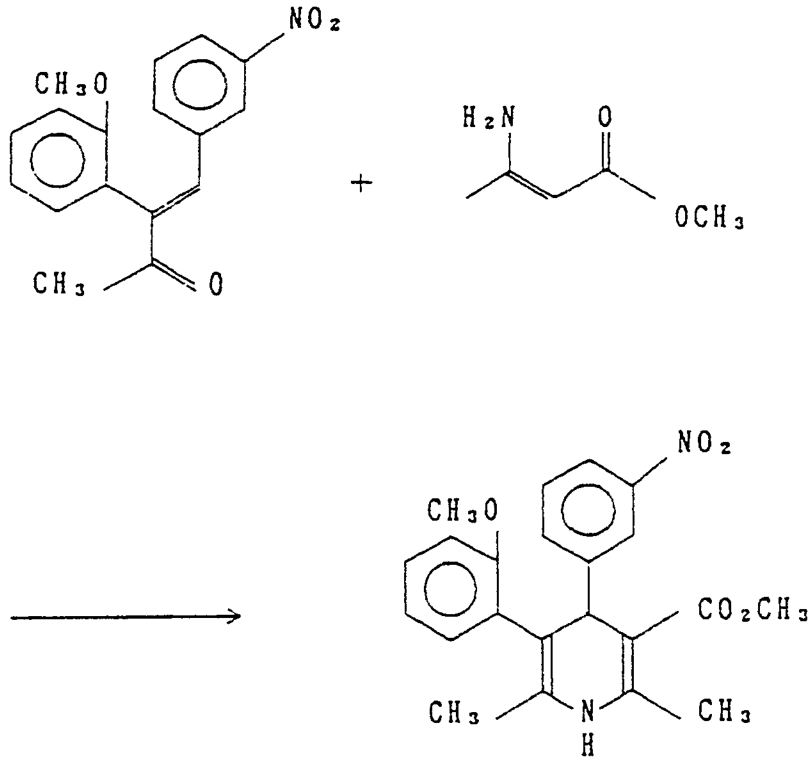

A mixuture of 297 mg (1 mmol) of 3-(2-methoxyphenyl)-4-(3-nitrophenyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained: The yield was 216 mg (55%).

Melting point: 176.4 to 177.7 °C

IR (cm$^{-1}$, KBr) ; $\nu$ NH 3370, CO 1705

$\nu$ $NO_2$ 1535, 1345

| Mass Analysis for $C_{22}H_{22}N_2O_5$ | |
|---|---|
| Calculated:<br>Found: | 394.15283<br>394.15187 |

NMR ($\delta$, $CDCl_3$); 1.70(3H,bs), 2.40(3H,s), 3.57(3H,s), 3.74~3.97(3H,b), 4.82(1H,bs), 5.42(1H,bs), 6.60~6.92-(4H,b), 7.16(1H,td,J = 8Hz,2Hz), 7.25(1H,t,J = 8Hz), 7.36(1H,d,J = 8Hz), 7.57(1H,d,J = 8Hz), 8.01(1H,s)

Example 42

Synthesis of methyl 1,4-dihydro-2,6-dimethyl-5-(2-fluorophenyl)-4-(3-nitrophenyl)pyridine-3-carboxylate:

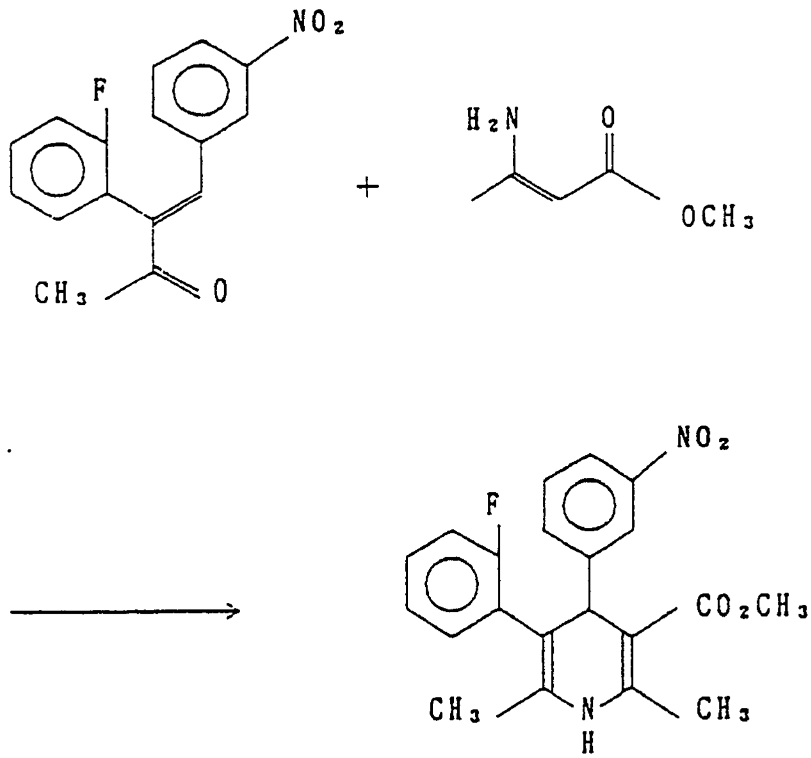

A mixuture of 285 mg (1 mmol) of 3-(2-fluorophenyl)-4-(3-nitrophenyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 242 mg (63%).
Melting point: 153.0 to 153.1 °C
IR ($cm^{-1}$, KBr) ; ν NH 3360, CO 1690
ν $NO_2$ 1535, 1345

| Mass Analysis for $C_{21}H_{19}FN_2O_4$ | |
|---|---|
| Calculated:<br>Found: | 382.13284<br>382.13492 |

NMR (δ, $CDCl_3$); 1.73(3H,s), 2.39(3H,s), 3.58(3H,s), 4.79(1H,s), 5.40(1H,bs), 6.81~6.90(1H,b), 6.92~7.02-(2H,m), 7.13~7.22(1H,m), 7.27(1H,t,J=8Hz), 7.41(1H,d,J=8Hz), 7.94(1H,ddd,J=8Hz,2Hz,1Hz), 8.00-(1H,t,J=2Hz)

Example 43

Synthesis of methyl 1,4-dihydro-2,6-dimethyl-4-(3-nitrophenyl)-5-thienylpyridine-3-carboxylate:

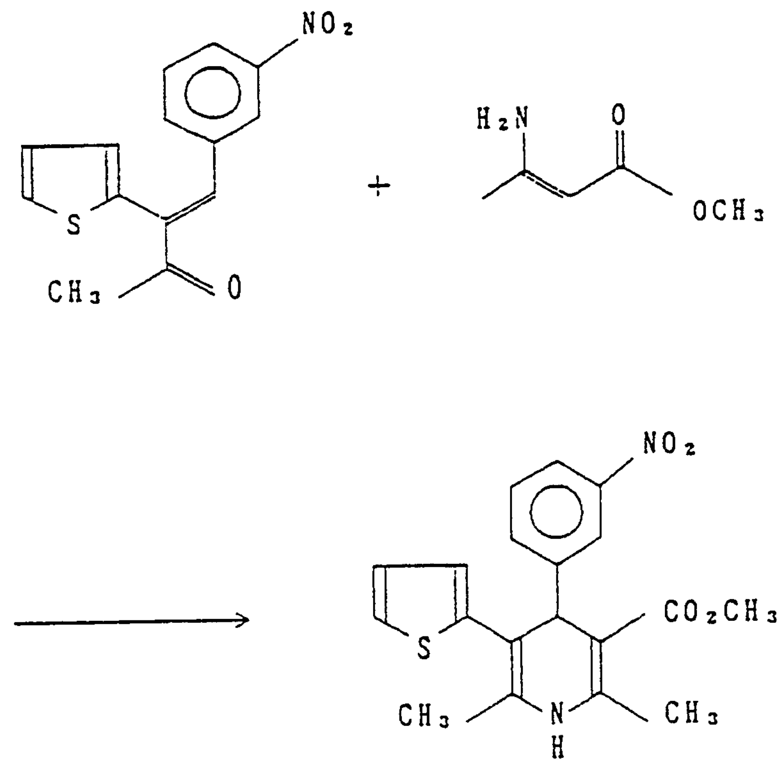

A mixuture of 273 mg (1 mmol) of 4-(3-nitrophenyl)-3-thienyl-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 206 mg (61%).

Melting point: 144.2 to 144.4 °C

IR ($cm^{-1}$, KBr) ; ν NH 3370, CO 1650

ν $NO_2$ 1550, 1355

| Mass Analysis for $C_{19}H_{18}N_2O_2S$ | |
|---|---|
| Calculated: | 338.10886 |
| Found: | 338.10655 |

NMR (δ, $CDCl_3$); 2.11(3H,s), 2.37(3H,s), 3.65(3H,s), 4.88(1H,s), 5.57(1H,bs), 6.66(1H,dd,J = 4Hz,1Hz), 6.88-(1H,dd,J = 5Hz,4Hz), 7.12(1H,dd,J = 5Hz,1Hz), 7.38(1H,t,J = 8Hz), 7.62(1H,dt,J = 8Hz,1Hz), 8.02-(1H,ddd,J = 8Hz,2Hz,1Hz), 8.14(1H,t,J = 2Hz)

Example 44

Synthesis of methyl 1,4-dihydro-2,6-dimethyl-5-(2-furyl)-4-(3-nitrophenyl)pyridine-3-carboxylate:

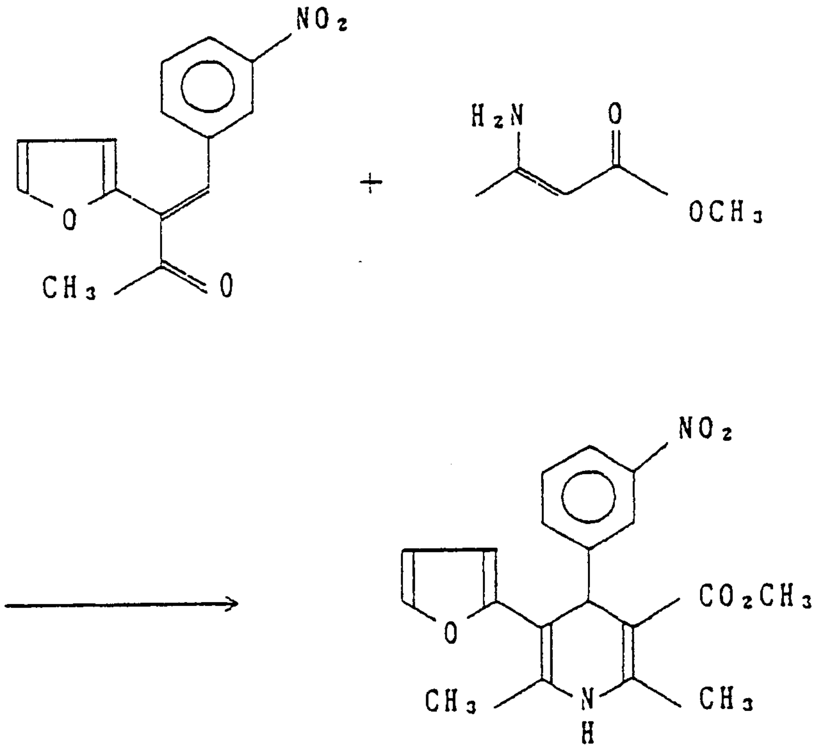

A mixuture of 257 mg (1 mmol) of 3-(2-furyl)-4-(3-nitrophenyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 289 mg (82%).

Melting point: 173.2 to 173.9 °C

IR ($cm^{-1}$, KBr) ; $\nu$ NH 3370, CO 1650

$\nu$ $NO_2$ 1530, 1350

| Mass Analysis for $C_{19}H_{18}N_2O_5$ | |
|---|---|
| Calculated: | 354.12154 |
| Found: | 354.12038 |

NMR ($\delta$, $CDCl_3$); 2.27(3H,s), 2.36(3H,s), 3.68(3H,s), 5.02(1H,s), 5.59(1H,bs), 6.07(1H,d,J=3Hz), 6.31-(1H,dd,J=3Hz,2Hz), 7.31(1H,d,J=2Hz), 7.37(1H,t,J=8Hz), 7.99(1H,ddd,J=8Hz,2Hz,1Hz), 8.15(1H,t,J=2Hz)

Example 45

Synthesis of methyl 1,4-dihydro-2,6-dimethyl-4-(3-nitrophenyl)-5-pyridazinylpyridine-3-carboxylate:

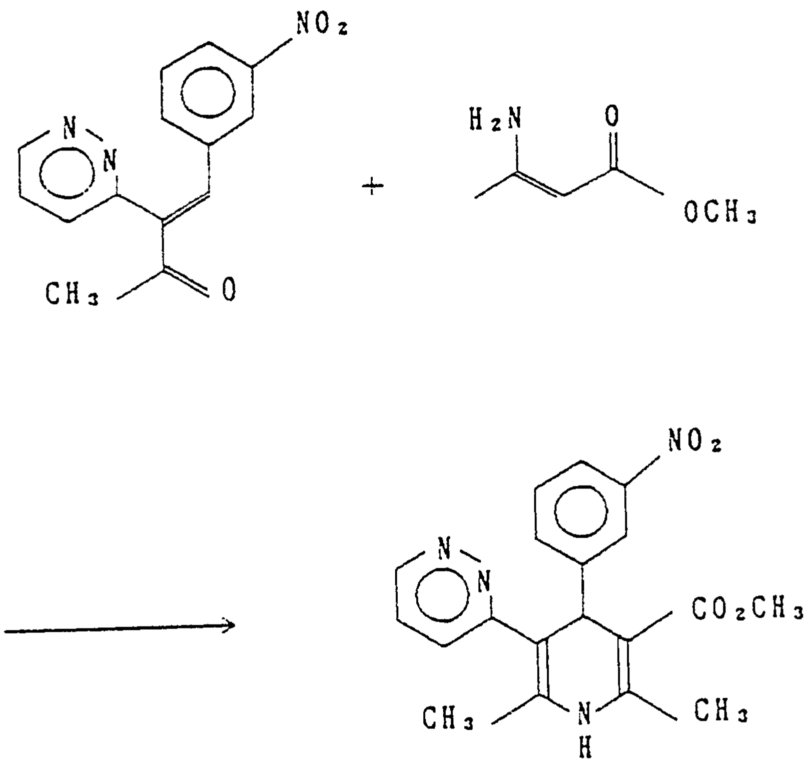

A mixuture of 213 mg (0.84 mmol) of 4-(3-nitrophenyl)-3-pyridazinyl-3-butene-2-one, 484 mg (4.2 mmol) of methyl 3-aminocrotonate, 343 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform The solvents contained in the extract were distilled off and the residue was chromatographed on a silica gel column, whereby the captioned compound was obtained. The yield was 280 mg (91%).
Melting point: 94.8° C
IR $\nu$ ($cm^{-1}$, KBr) ; $\nu$ CO 1680
$\nu$ $NO_2$ 1528, 1348

| Mass Analysis for $C_{19}H_{18}N_4O_4$ | |
|---|---|
| Calculated: | 366.13277 |
| Found: | 366.13126 |

NMR ($\delta$, $CDCl_3$); 2.18(3H,s), 2.40(3H,s), 3.66(3H,s), 5.25(1H,s), 6.04(1H,s), 7.23(1H,dd,J=8.7Hz,2.4Hz), 7.34(1H,dd,J=8.7Hz,5.1Hz), 7.35(1H,t,J=7.8Hz), 7.61(1H,d,J=7.8Hz), 7.98(1H,d,J=7.8Hz), 8.12(1H,s), 8.98-(1H,dd,J=5,1Hz,1.5Hz)

Example 46

Synthesis of methyl 1,4-dihydro-2,6-dimethyl-4-(3-nitrophenyl)-5-(3-quinolyl)pyridine-3-carboxylate:

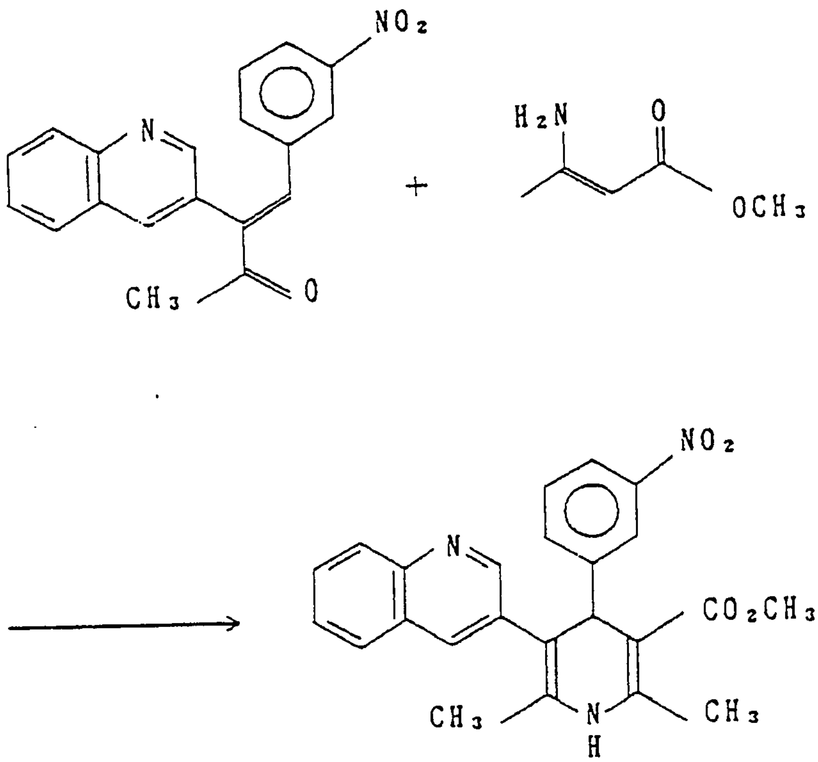

A mixuture of 302 mg (1 mmol) of 4-(3-nitrophenyl)-3-(3-quinolyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 300 mg (80.8%).

Melting point: 115°C

IR ($cm^{-1}$, KBr) ; $\nu$ CO 1695

$\nu$ $NO_2$ 1530, 1350

| Mass Analysis for $C_{24}H_{21}N_3O_4$ | |
|---|---|
| Calculated: | 415.15316 |
| Found: | 415.15194 |

NMR ($\delta$, $CDC\ell_3$); 1.93(3H,s), 2.43(3H,s), 3.63(3H,s), 4.92(1H,s), 5.64(1H,s), 7.33(1H,t, J=8Hz), 7.47-(1H,d,J=8Hz), 7.52(1H,t,J=8Hz), 7.67~7.74(3H,m), 8.01(1H,d,J=8Hz), 8.09(1H,d,J=8Hz), 8.12(1H,s), 8.57-(1H,d,J=2Hz)

Example 47

Synthesis of methyl 1,4-dihydro-2,6-dimethyl-4-(3-nitrophenyl)-5-pyrazinylpyridine-3-carboxylate:

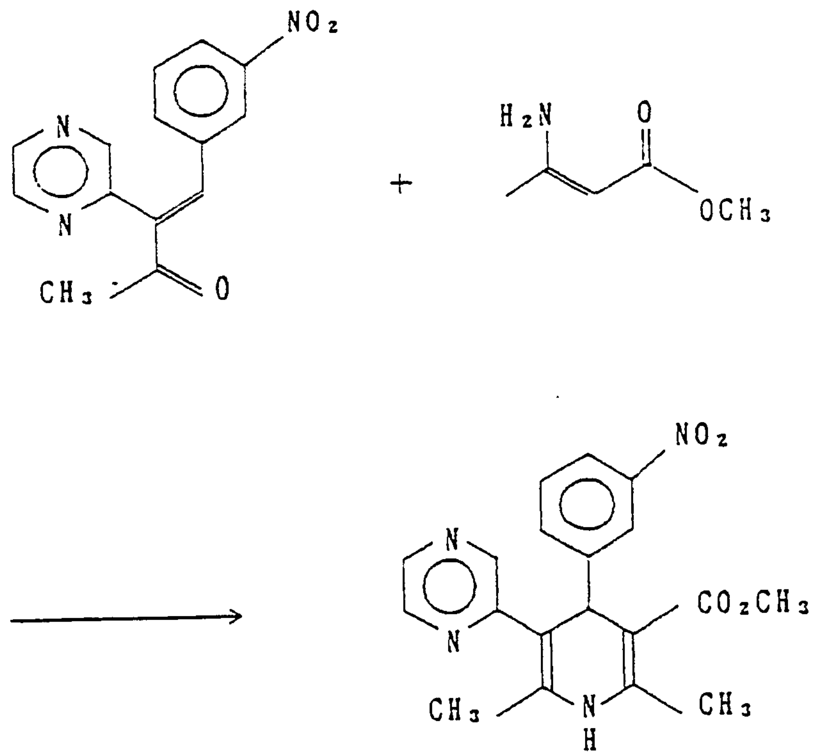

A mixuture of 253 mg (1 mmol) of 4-(3-nitrophenyl)-3-pyrazinyl-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 271 mg (77.3%).
Melting point: 134.9 to 136.1 °C
IR ($cm^{-1}$, KBr) ; $\nu$ CO 1670
$\nu$ $NO_2$ 1530, 1350

| Mass Analysis for $C_{19}H_{18}N_4O_4$ | |
|---|---|
| Calculated:<br>Found: | 366.13277<br>366.13473 |

NMR ($\delta$,$CDCl_3$); 2.18(3H,s), 2.40(3H,s), 3.66(3H,s), 5.25(1H,s), 5.68(1H,s), 7.35(1H,t,J=8Hz), 7.59-(1H,d,J=8Hz), 7.97(1H,d,J=8Hz), 8.10(1H,s), 8.30(1H,d,J=3Hz), 8.34(1H,d,J=2Hz), 8.51(1H,dd,J=3,2Hz)

Example 48

Synthesis of methyl 1,4-dihydro-2,6-dimethyl-4-(3-nitrophenyl)-5-(2-pyridyl)pyridine-3-carboxylate:

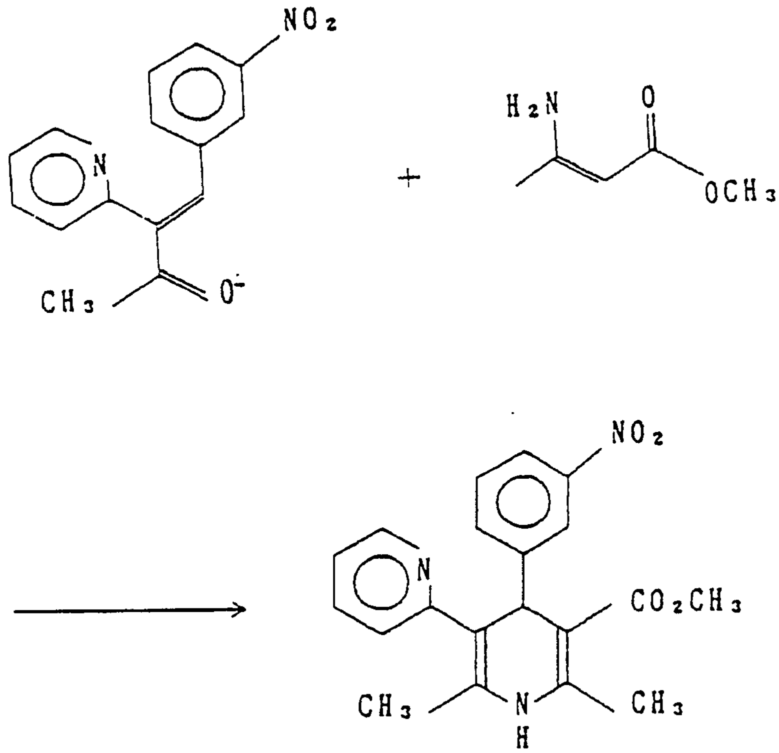

A mixuture of 268 mg (1 mmol) of 4-(3-nitrophenyl)-3-(2-pyridyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 292 mg (80%).
Melting point: 156.6 to 158.1 °C
IR ($cm^{-1}$, KBr) ; $\nu$ NH 3340, CO 1665
$\nu$ $NO_2$ 1535, 1345

| Mass Analysis for $C_{20}H_{19}N_3O_4$ | |
|---|---|
| Calculated: | 365.13751 |
| Found: | 365.13973 |

NMR ($\delta$, $CDCl_3$); 2.07(3H,s), 2.39(3H,s), 3.63(3H,s), 5.23(1H,s), 5.54(1H,s), 6.97(1H,d,J=8Hz), 7.05-(1H,dd.J=8Hz,5Hz), 7.31(1H,t,J=8Hz), 7.50(1H,td,J=8Hz,2Hz), 7.53(1H,d,J=8Hz), 7.95(1H,dt,J=8Hz,2Hz), 8.07(1H,t,J=2Hz), 8.55(1H,d,J=5Hz)

Example 49

Synthesis of methyl 1,4-dihydro-2,6-dimethyl-4-(3-nitrophenyl)-5-(4-pyridyl)pyridine-3-carboxylate:

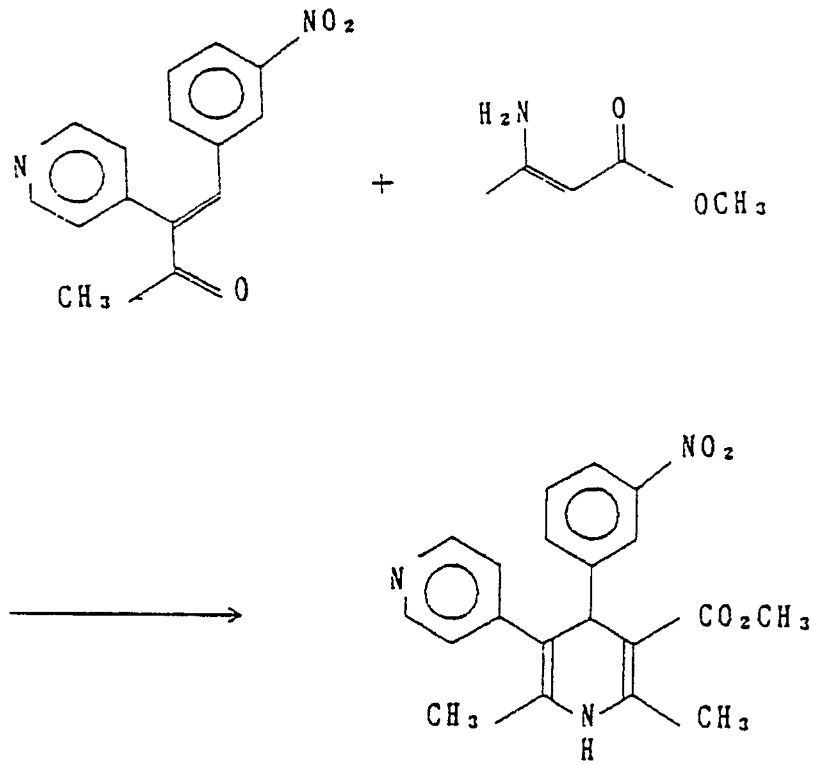

A mixuture of 268 mg (1 mmol) of 4-(3-nitrophenyl)-3-(4-pyridyl)-3-butene-2-one, 575 mg (5 mmol)) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained, The yield was 300 mg (82%).

Melting point: 165 ° C (decomposed)

IR ($cm^{-1}$, KBr) ; ν CO 1680

ν $NO_2$ 1530, 1350

| Mass Analysis for $C_{20}H_{19}N_3O_4$ | |
|---|---|
| Calculated:<br>Found: | 365.13751<br>365.13897 |

NMR (δ, $CDCl_3$); 1.95(3H,s) 2.39(3H,s), 3.64(3H,s), 4.86(1H,s), 5.61(1H,s), 6.93(2H,dd,J = 5Hz,2Hz), 7.34-(1H,t,J = 8Hz), 7.47(1H,d,J = 8Hz), 8.00(1H,ddd,J = 8Hz,2Hz,1Hz), 8.07(1H,t,J = 2Hz), 8.45(2H,dd,J = 5Hz,2Hz)

Example 50

Synthesis of 2-(N-benzyl-N-methylamino)ethyl 1,4-dihydro-2,6-dimethyl-4-(3-nitrophenyl)-5-(4-pyridyl)-pyridine-3-carboxylate:

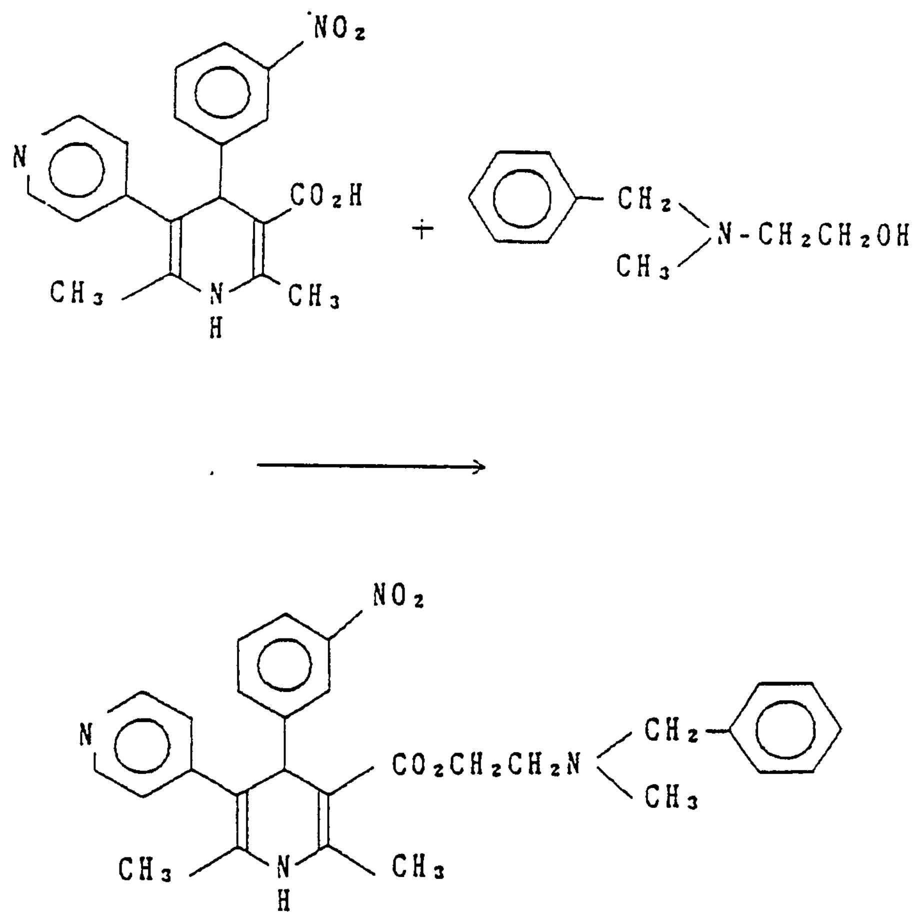

350 mg (1 mmol) of 1,4-dihydro-2,6-dimethyl-4-(3-nitrophenyl)-5-(4-pyridyl)pyridine-3-carboxylic acid, 165 mg (1 mmol) of 2-(N-benzyl-N-methylamino)ethanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 502 mg (100%).

Melting point: oil

IR ($cm^{-1}$, KBr) ; $\nu$ CO 1695

$\nu$ $NO_2$ 1530, 1350

| Mass Analysis for $C_{29}H_{30}N_4O_4$ | |
|---|---|
| Calculated: | 498.22666 |
| Found: | 498.22850 |

NMR ($\delta$, $CDCl_3$);. 1.94(3H,s), 2.20(3H,s), 2.38(3H,s), 2.58(1H,dd,J=9,6Hz), 2.65(1H,dd,J=9,6Hz), 3.49-(2H,s), 4.16(2H,t,J=6Hz), 4.85(3H,s), 5.65(1H,s), 6.91(2H,d,J=7Hz), 7.15~7.30(6H,m), 7.46(1H,d,J=8Hz), 7.97(1H,d,J=8Hz), 8.07(1H,s), 8.45(2H,d,J=7Hz)

Example 51

Synthesis of (E)-3-phenyl-2-propene-1-yl 1,4-dihydro-2,6-dimethyl-4-(3-nitrophenyl)-5-(4-pyridyl)pyridine-3-carboxylate:

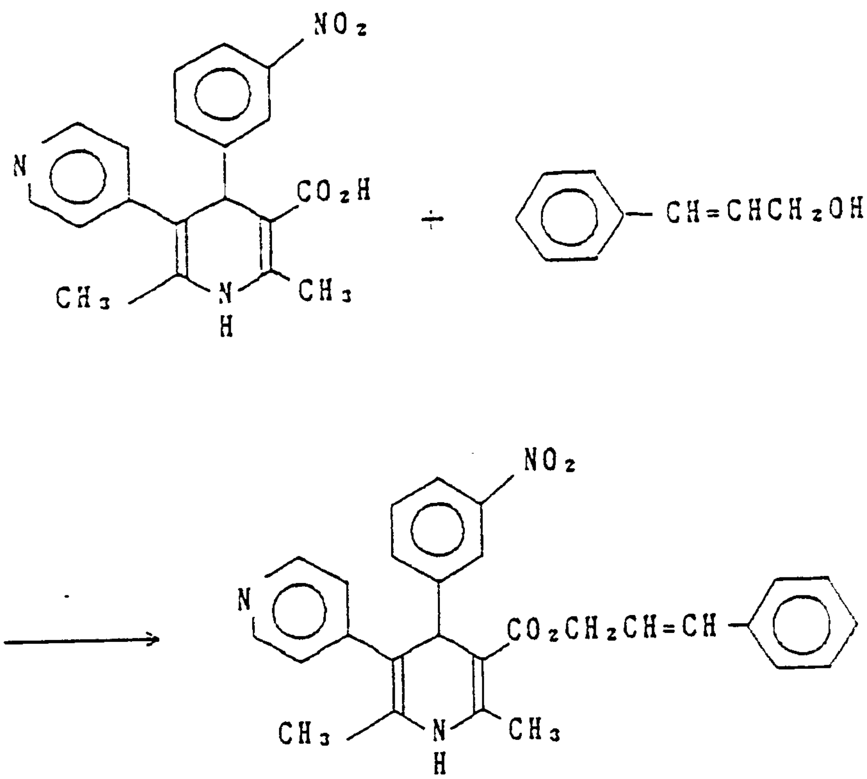

700 mg (2 mmol) of 1,4-dihydro-2,6-dimethyl-4-(3-nitrophenyl)-5-(4-pyridyl)pyridine-3-carboxylic acid was suspended in 10 ml of anhydrous tetrahydrofuran in an atmosphere of an inert gas (Ar), to which was added 220 mg (2.2 mmol) of triethylamine. To the resulting mixture, 228 mg (2.1 mmol) of ethylchloroformate was added dropwise while cooling with ice. After stirring the mixture for 30 minutes, 268 mg (2 mmol) of (E)-3-phenyl-2-propene-1-ol was added to the mixture while cooling with ice, followed by agitation for 2.5 hours. The reaction mixture was then washed with a saturated saline solution and dried over anhydrous sodium sulfate. Tetrahydrofluran contained in the reaction mixture was then distilled off and the residue was chromatographed on a silica gel column, whereby the captioned compound was obtained. The yield was 280 mg (30%).

Melting point: 158.0 to 159.5° C

IR ($cm^{-1}$, KBr) ; $\nu$ CO 1698

$\nu$ $NO_2$ 1530, 1350

| Mass Analysis for $C_{28}H_{25}N_3O_4$ | |
|---|---|
| Calculated: | 467.18446 |
| Found: | 467.18453 |

NMR ($\delta$, $CDCl_3$); 1.94(3H,s), 2.41(3H,s), 4.67(1H,dd,J=12,7Hz), 4.75(1H,dd,J=12,7Hz), 4.89(1H,s), 5.69-(1H,s), 6.23(1H,dt,J=15,5Hz), 6.54(1H,d,J=15Hz), 6.91(2H,d,J=7Hz), 7.25~7.36(6H,m), 7.49(1H,d,J=8Hz), 8.00(1H,d,J=8Hz), 8.10(1H,s), 8.44(2H,d,J=7Hz)

Example 52

Synthesis of 2-(4-phenylpiperazinyl)ethyl 1,4-dihydro-2,6-dimethyl-4-(3-nitrophenyl)-5-(4-pyridyl)pyridine-3-carboxylate:

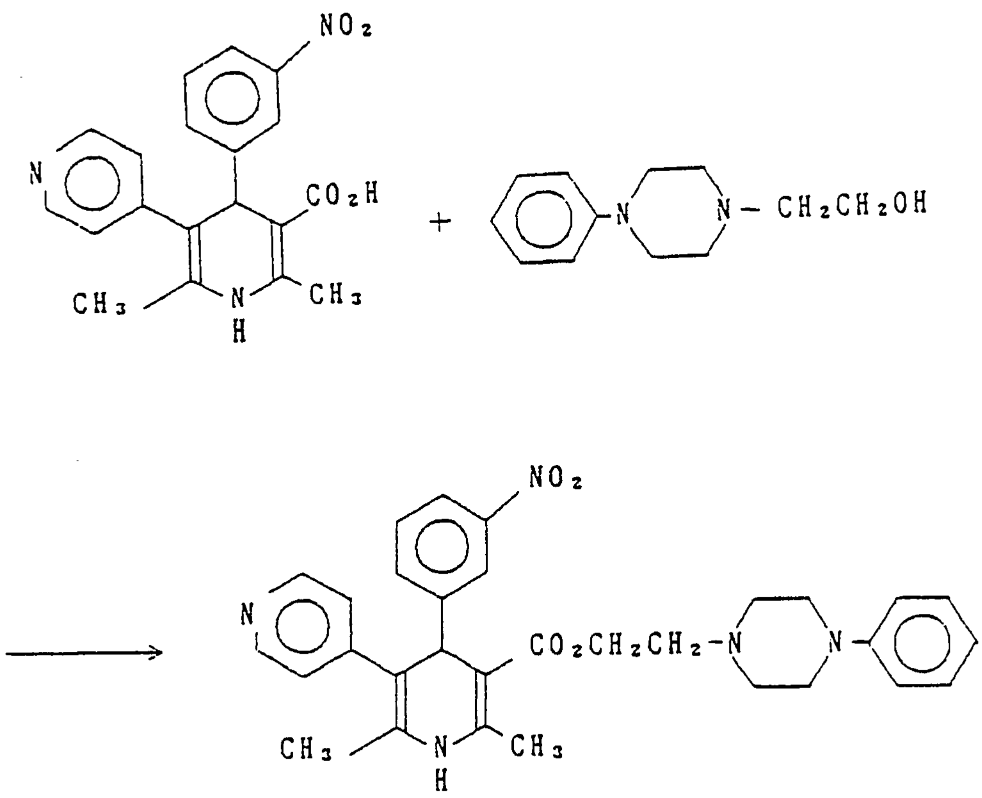

350 mg (1 mmol) of 1,4-dihydro-2,6-dimethyl-4-(3-nitrophenyl)-5-(4-pyridyl)pyridine-3-carboxylic acid, 206 mg (1 mmol) of 2-(4-phenylpiperazinyl)ethanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 471 mg (100%).
Melting point: oil
IR ($cm^{-1}$, KBr) ; ν CO 1694
ν $NO_2$ 1532, 1350

| Mass Analysis for $C_{31}H_{33}N_5O_4$ | |
|---|---|
| Calculated: | 539.25321 |
| Found: | 539.25378 |

NMR (δ, $CDCl_3$); 1.94(3H,s), 2.41(3H,s), 2.50~2.82(6H,m), 3.10~3.27(4H,m), 4.20~4.40(2H,m), 4.88(1H,s), 5.66(1H,s), 6.85~6.98(4H,m), 7.23~7.32(5H,m), 7.34(1H,t,J=7Hz), 7.49(1H,d,J=7Hz), 8.00(1H,d,J=7Hz), 8.06(1H,s), 8.46(1H,d,J=6.3Hz)

Example 53

Synthesis of 2-propene-1-yl 1,4-dihydro-2,6-dimethyl-4-(3-nitrophenyl)-5-(4-pyridyl)pyridine-3-carboxylate:

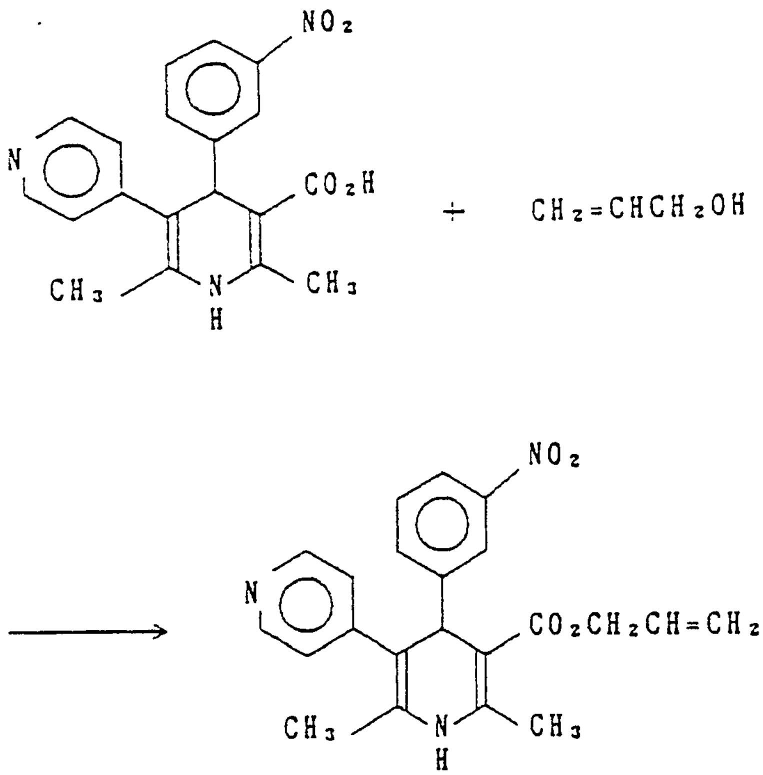

350 mg (1 mmol) of 1,4-dihydro-2,6-dimethyl-4-(3-nitrophenyl)-5-(4-pyridyl)pyridine-3-carboxylic acid, 58 mg (1 mmol) of 2-propene-1-ol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 390 mg (100%).

Melting point: 185.3 to 186.7 °C

IR ($cm^{-1}$, KBr) ; ν CO 1696

ν $NO_2$ 1526, 1348

| Mass Analysis for $C_{22}H_{21}N_3O_4$ | |
|---|---|
| Calculated: | 391.15317 |
| Found: | 391.15342 |

NMR (δ, $CDCl_3$); 2.08(3H,s), 2.41(3H,s), 4.54(1H,dd,J = 13Hz,6Hz), 4.62(1H,dd,J = 13Hz,6Hz), 4.94(1H,s), 5.20(1H,dd,J = 10Hz,2Hz), 5.22(1H,dd,J = 19Hz,2Hz), 5.82~5.97(1H,m), 5.90(1H,s), 7.17(2H,d,J = 6Hz), 7.38-(1H,t.J = 7.8Hz), 7.51(1H,d,J = 7.8Hz), 8.04(1H,d,J = 7.8Hz), 8.09(1H,s), 8.46(1H,d,J = 6Hz)

Example 54

Synthesis of 2-(phenoxy)ethyl 1,4-dihydro-2,6-dimethyl-4-(3-nitrophenyl)-5-(4-pyridyl)pyridine-3-carboxylate:

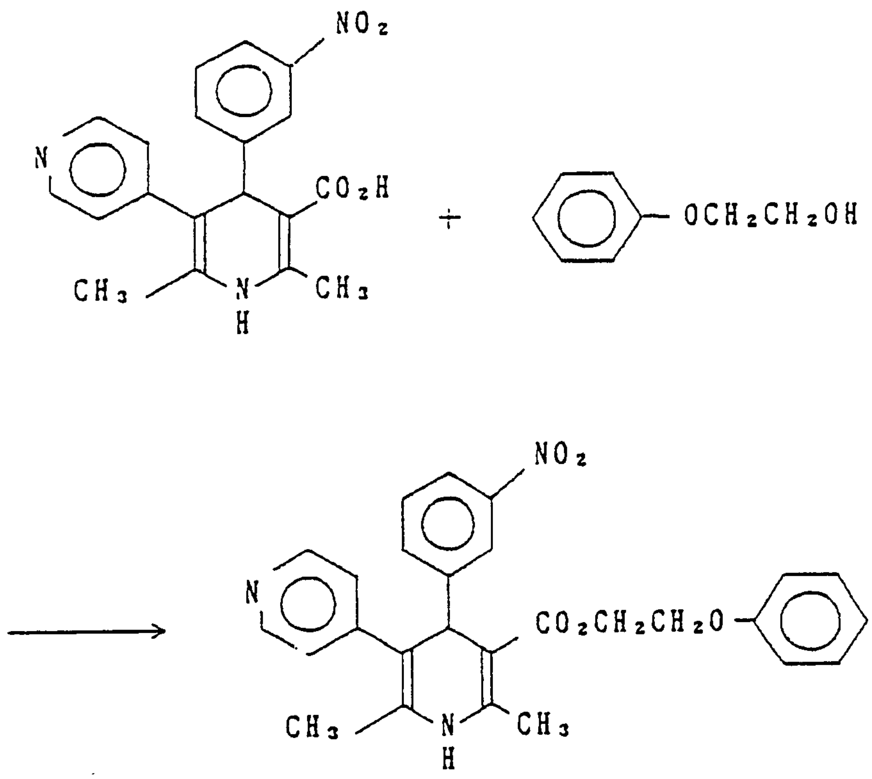

350 mg (1 mmol) of 1,4-dihydro-2,6-dimethyl-4-(3-nitrophenyl)-5-(4-pyridyl)pyridine-3-carboxylic acid, 138 mg (1 mmol) of 2-(phenoxy)ethanol, 309 mg (1.5 mmol) of N,N′-dicyclohexylcarbodiimide, 134 mg (1.1 mmol) of 4-N,N-dimethylaminopyridine were dissolved in 10 ml of toluene, with application of heat thereto. This reaction mixture was refluxed with application of heat for 1 hour, and then cooled to room temperature. To this reaction mixture, 20 ml of chloroform was added. The reaction mixture was then washed with water and dried over anhydrous sodium sulfate. The solvents contained in the reaction mixture were then distilled off and the residue was chromatographed on a silica gel column, whereby the captioned compound was obtained. The yield was 540 mg (100%).

Melting point: 80° C

IR ($cm^{-1}$, KBr) ; $\nu$ CO 1698

$\nu$ $NO_2$ 1528, 1350

| Mass Analysis for $C_{27}H_{25}N_3O_5$ | |
|---|---|
| Calculated: | 471.17938 |
| Found: | 471.18037 |

NMR ($\delta$, $CDCl_3$); 1.97(3H,s), 2.40(3H,s), 4.03~4.18(2H,m), 4.30~4.39(1H,m), 4.42~4.52(1H,m), 4.87(1H,s), 5.78(1H,s), 6.85(2H,d,J=7Hz), 6.93~7.00(1H,m), 6.95(2H,d,J=6.5Hz), 7.19(1H,t,J=8.2Hz), 7.28-(2H,t,J=7Hz), 7.46(1H,d,J=8.2Hz), 7.94(1H,s), 8.44(2H,d,J=6.5Hz)

Example 55

Synthesis of (E)-3-[4-(1-imidazolylmethyl)phenyl]-2-propene-1-yl 1,4-dihydro-2,6-dimethyl-5-(2-furyl)-4-(3-nitrophenyl)pyridine-3-carboxylate:

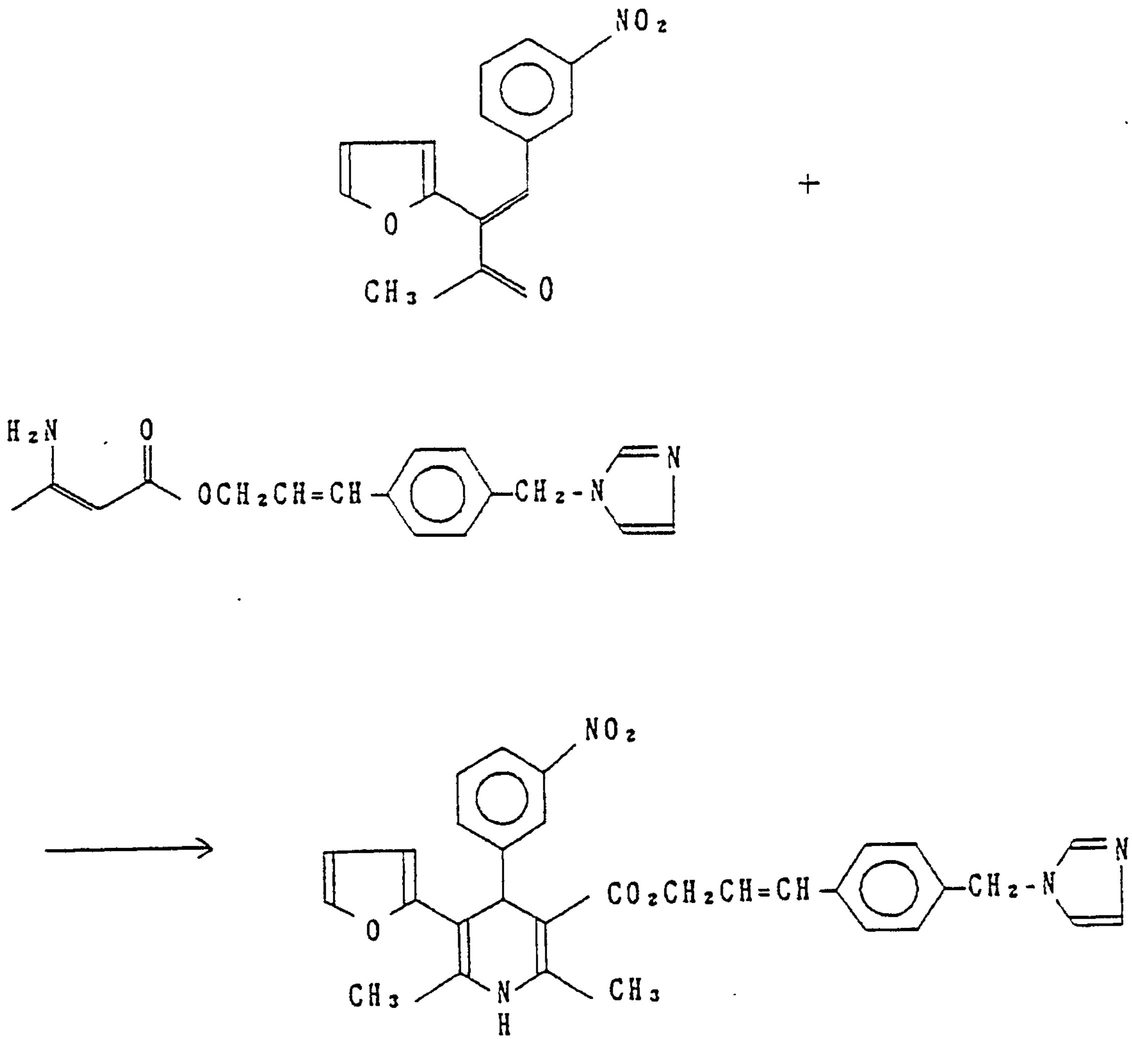

A mixuture of 257 mg (1 mmol) of 3-(2-furyl)-4-(3-nitrophenyl)-3-butene-2-one, 885 mg (3 mmol) of (E)-3-[4-(1-imidazolylmethyl)phenyl]-2-propene-1-yl 3-aminocrotonate, 409 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 210 mg (39%).

Melting point: 81 °C (decomposed)

IR ($cm^{-1}$, KBr) ; ν CO 1690

ν $NO_2$ 1528, 1350

| Mass Analysis for $C_{31}H_{28}N_4O_5$ | |
|---|---|
| Calculated:<br>Found: | 536.20593<br>536.20635 |

NMR (δ, $CDCl_3$); 2.27(3H,s), 2.39(3H,s), 4.68(1H,dd,J = 12.5Hz,6Hz), 4.80(1H,dd,J = 12.5Hz,6Hz), 5.05(1H,s), 5.22(1H,s), 5.66(1H,s), 6.05(1H,d,J = 3.2Hz), 6.25~6.35(2H,m), 6.54(1H,d,J = 16Hz), 7.00(1H,s), 7.19-(2H,d,J = 9Hz), 7.24(1H,s), 7.30(1H,d,J = 2Hz), 7.35(1H,t,J = 6.4Hz), 7.38(2H,d,J = 9Hz), 7.68(1H,d,J = 6.4Hz), 7.97(1H,d,J = 6.4Hz), 8.16(1H,s), 8.18(1H,s)

Example 56

Synthesis of 3-cyano-1,4-dihydro-2,6-dimethyl-4-(3-nitrophenyl)-5-(2-pyridyl)pyridine:

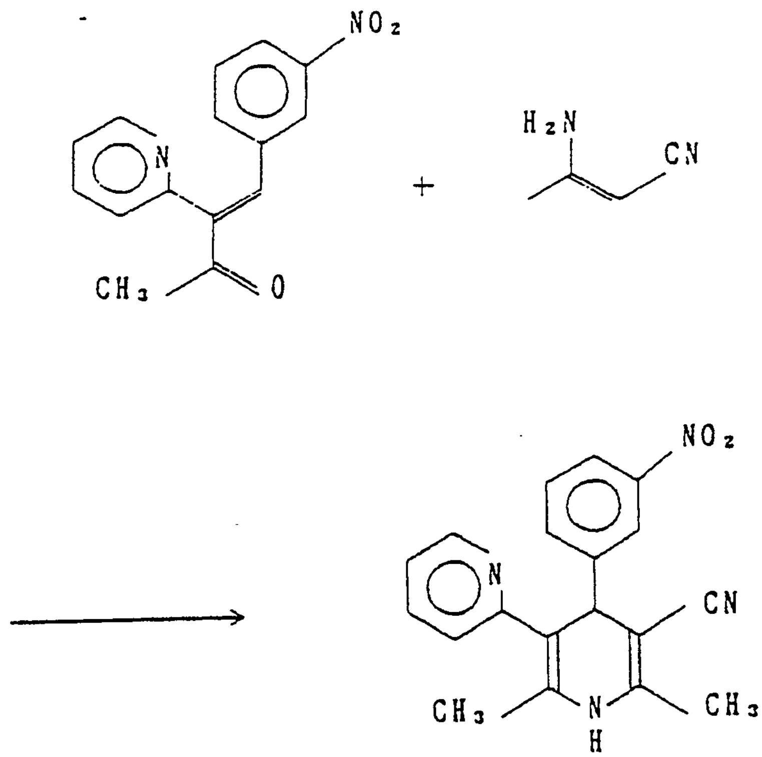

A mixuture of 268 mg (1 mmol) of 4-(3-nitrophenyl)-3-(2-pyridyl)-3-butene-2-one, 410 mg (5 mmol) of 3-aminocrotonitrile, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 160 mg (48%).
Melting point: 137.3 to 138.5 °C
IR ($cm^{-1}$, KBr) ; $\nu$ NH 3390 CN 2190
$\nu$ $NO_2$ 1530, 1355

| Mass Analysis for $C_{19}H_{16}N_4O_2$ | |
|---|---|
| Calculated:<br>Found: | 332.12730<br>332.12778 |

NMR (δ, $CDCl_3$); 2.03(3H,s), 2.17(3H,s), 5.03(1H,s), 5.56(1H,s) 6.94(1H,d,J=8Hz), 7.05(1H,dd,J=8Hz,5Hz), 7.39(1H,t,J=8Hz), 7.50(1H,td,J=8Hz,2Hz), 7.52(1H,d,J=8Hz), 8.02(1H,dt,J=8Hz,2Hz), 8.09(1H,t,J=2Hz), 8.52(1H,d,J=5Hz)

Example 57

Synthesis of N,N-diethyl-1,4-dihydro-2,6-dimethyl-4-(3-nitrophenyl)-5-(2-pyridyl)pyridine-3-carboxamide:

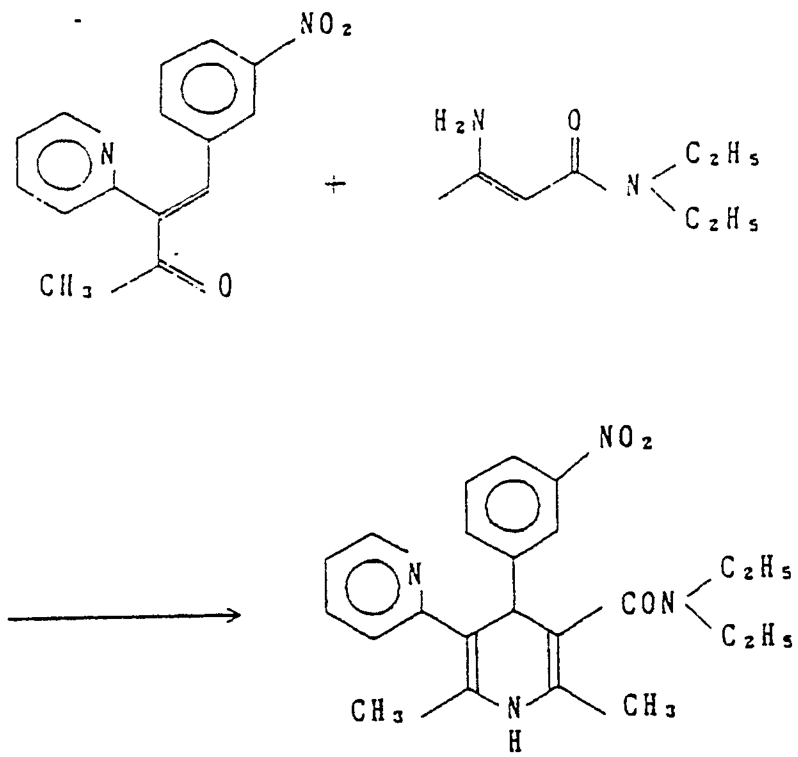

A mixuture of 268 mg (1 mmol) of 4-(3-nitrophenyl)-3-(2-pyridyl)-3-butene-2-one, 180 mg (5 mmol) of N,N-diethyl 3-amino-2-buteneamide, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained, The yield was 259 mg (64%).

Melting point: oil

IR ($cm^{-1}$, KBr) ; $\nu$ CO 1685

$\nu$ $NO_2$ 1530, 1350

| Mass Analysis for $C_{31}H_{33}N_5O_4$ | |
|---|---|
| Calculated: | 539.25321 |
| Found: | 539.25378 |

NMR ($\delta$, $CDCl_3$); 0.68~1.13(6H,m), 1.85(3H,s) 2.09(3H,s), 3.14~3.49(4H,m), 4.97(1H,s), 5.10(1H,bs), 6.96-(1H,dd,J=8Hz,5Hz), 7.02(1H,d,J=8Hz), 7.33(1H,t,J=8Hz), 7.45(1H,td,J=8Hz,2Hz), 7.57(1H,d,J=8Hz), 7.94-(1H,ddd,J=8Hz,2Hz,1Hz), 8.07(1H,t,J=2Hz), 8.46(1H,d,J=5Hz)

Example 58

Synthesis of 3-benzoyl-1,4-dihydro-2,6-dimethyl-4-(3-nitrophenyl)-5-(2-pyridyl)pyridine:

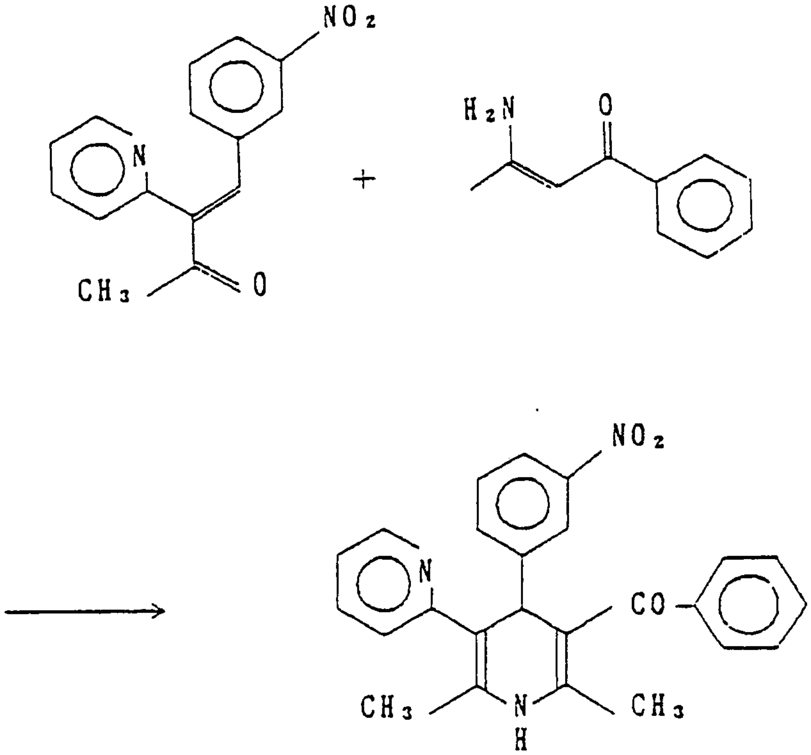

A mixuture of 268 mg (1 mmol) of 4-(3-nitrophenyl)-3-(2-pyridyl)-3-butene-2-one, 805 mg (5 mmol) of 3-amino-1-phenyl-2-butene-1-one, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 288 mg (70%).
Melting point: 202.3 to 206.8° C
IR ($cm^{-1}$, KBr) ; ν NH 3310 CO 1680
ν $NO_2$ 1525, 1345

| Mass Analysis for $C_{25}H_{21}N_3O_3$ | |
|---|---|
| Calculated: | 411.15827 |
| Found: | 411.15832 |

NMR (δ, $CDCl_3$); 1.86(3H,s), 2.21(3H,s), 5.32(1H,s) 5.64(1H,bs), 7.02(1H,d,J=8Hz) 7.05-(1H,ddd,J=8Hz,5Hz,1Hz), 7.27~7.58(8H,m), 7.96(1H,ddd,J=8Hz,2Hz,1Hz), 8.05(1H,t,J=2Hz), 8.54-(1H,d,J=5Hz)

Example 59

Synthesis of 3-acetyl-1,4-dihydro-2,6-dimethyl-4-(3-nitrophenyl)-5-(2-pyridyl)pyridine:

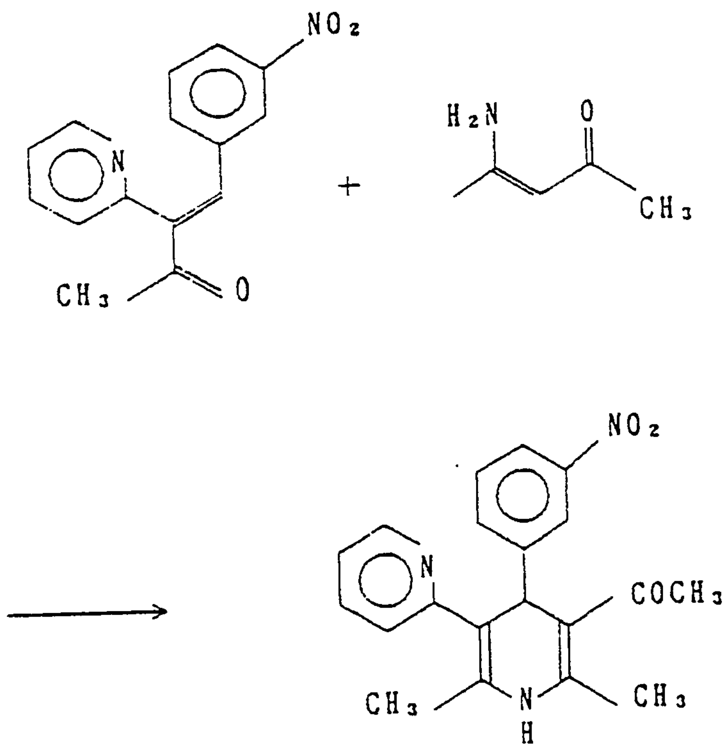

A mixuture of 268 mg (1 mmol) of 4-(3-nitrophenyl)-3-(2-pyridyl)-3-butene-2-one, 495 mg (5 mmol) of 4-amino-3-pentene-2-one, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 49 mg (14%).
Melting point: 192.5 to 193.1 °C
IR ($cm^{-1}$, KBr) ; ν NH 3320
ν $NO_2$ 1530, 1340

| Mass Analysis for $C_{20}H_{19}N_3O_3$ | |
|---|---|
| Calculated:<br>Found: | 349.14260<br>349.14319 |

NMR (δ, $CDCl_3$); 2.04(3H,s), 2.18(3H,s), 2.42(3H,s), 5.34(1H,s), 5.60(1H,bs), 6.96(1H,dd,J = 8Hz,1Hz), 7.09-(1H,dd,J = 8Hz,5Hz), 7.31(1H,t,J = 8Hz), 7.48(1H,d,J = 8Hz), 7.53(1H,td,J = 8Hz,2Hz), 7.96(1H,d,J = 8Hz), 7.99-(1H,t,J = 2Hz), 8.59(1H,d,J = 5Hz)

Example 60

Synthesis of 2,2,2-trifluoroethyl 1,4-dihydro-2,6-dimethyl-4-(3-nitrophenyl)-5-(2-pyridyl)pyridine-3-carboxylate:

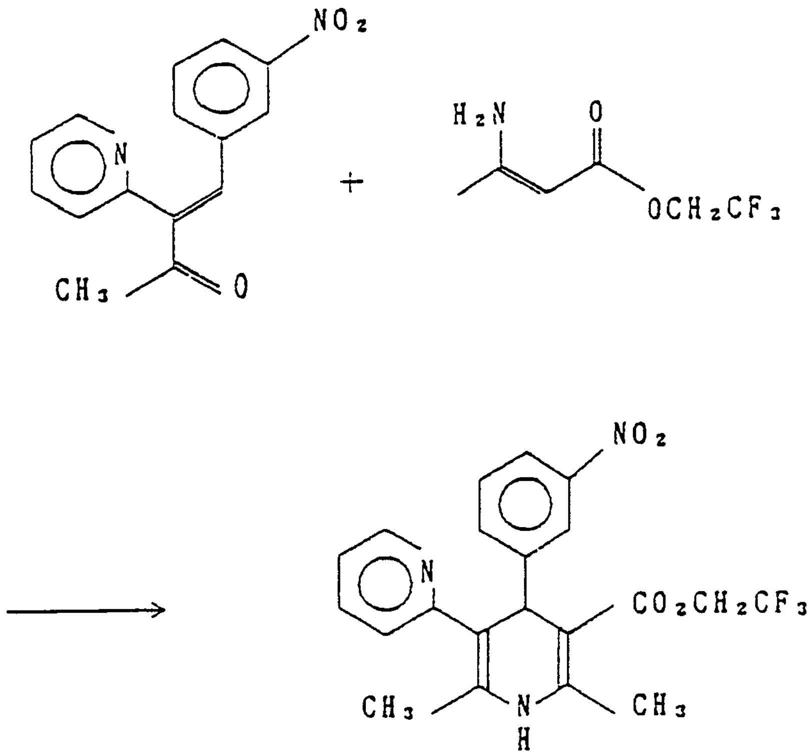

A mixuture of 268 mg (1 mmol) of 4-(3-nitrophenyl)-3-(2-pyridyl)-3-butene-2-one, 915 mg (5 mmol) of 2,2,2-trifluoroethyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 101 mg (23%).
Melting point: 132.1 to 134.4 °C
IR ($cm^{-1}$, KBr) ; $\nu$ NH 3390
$\nu$ CO 1960 $NO_2$ 1525, 1350

| Mass Analysis for $C_{21}H_{18}N_3O_4$ | |
|---|---|
| Calculated:<br>Found: | 433.12491<br>433.12453 |

NMR ($\delta$, $CDCl_3$); 2.05(3H,s), 2.41(3H,s), 4.24~4.53(2H,m), 5.21(1H,s), 5.67(1H,bs), 6.93(1H,d,J=8Hz), 7.07-(1H,ddd,J=8Hz,5Hz,1Hz), 7.31(1H,t,J=8Hz), 7.49(1H,d,J=8Hz), 7.51(1H,td,J=8Hz,2Hz), 7.97-(1H,dt,J=8Hz,2Hz), 8.07(1H,t,J=2Hz), 8.56(1H,d,J=5Hz)

Example 61

Synthesis of 1,4-dihydro-2,6-dimethyl-3-nitro-4-(3-nitrophenyl)-5-(2-pyridyl)pyridine:

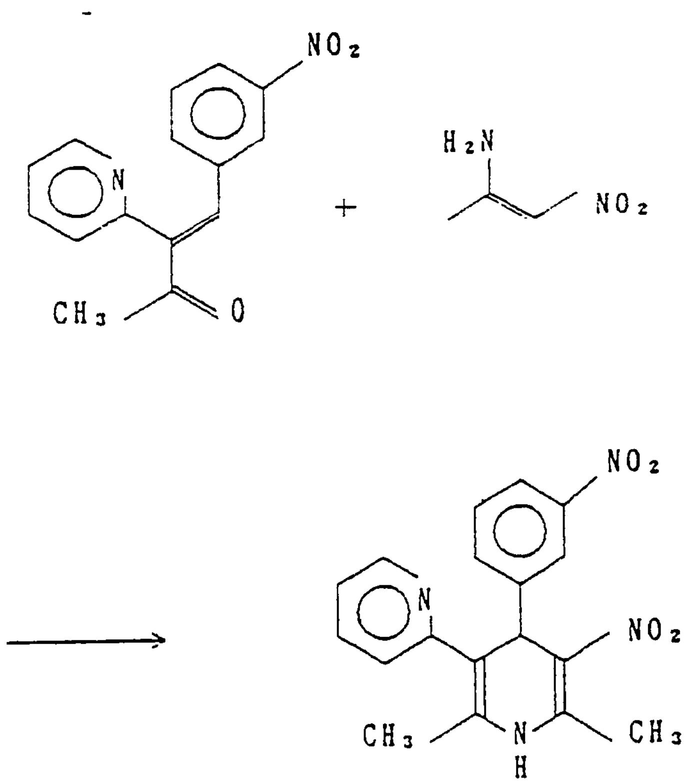

A mixuture of 268 mg (1 mmol) of 4-(3-nitrophenyl)-3-(2-pyridyl)-3-butene-2-one, 510 mg (5 mmol) of 2-amino-1-nitro-1-propene, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 98 mg (28%).
Melting point: 195.6 to 199.2°C
IR ($cm^{-1}$, KBr) ; $\nu$ $NO_2$ 1530, 1350

| Mass Analysis for $C_{18}H_{16}N_4O_4$ | |
|---|---|
| Calculated:<br>Found: | 352.11712<br>352.11891 |

NMR ($\delta$, $CDCl_3$); 2.06(3H,s), 2.64(3H,s), 5.70(1H,s), 5.98(1H,bs), 6.95(1H,d,J=8Hz), 7.11-(1H,ddd,J=8Hz,5Hz,1Hz), 7.33(1H,t,J=8Hz), 7.54(1H,td,J=8Hz,2Hz), 7.55(1H,d,J=8Hz), 7.98-(1H,ddd,J=8Hz,2Hz,1Hz), 8.08(1H,t,J=2Hz), 8.58(1H,d,J=8Hz)

Example 62

Synthesis of methyl 1,4-dihydro-2,6-dimethyl-4-(3-nitrophenyl)-5-pyridylpyridine-3-sulfinate:

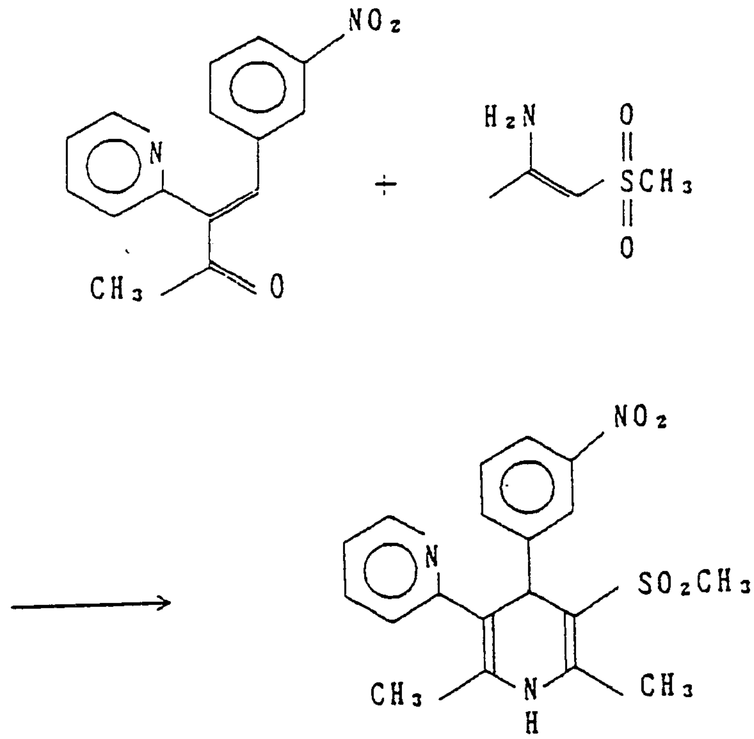

A mixuture of 268 mg (1 mmol) of 4-(3-nitrophenyl)-3-(2-pyridyl)-3-butene-2-one, 675 mg (5 mmol) of methyl 3-amino-2-propenesulfinate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 150 mg (39%).
Melting point: 210.8 to 211.5 °C
IR ($cm^{-1}$, KBr) ; ν NH 3350
ν $NO_2$ 1530, 1350

| Mass Analysis for $C_{19}H_{19}N_3O_4S$ | |
| --- | --- |
| Calculated:<br>Found: | 385.10960<br>385.10962 |

NMR (δ, $CDCl_3$); 2.07(3H,s), 2.39(3H,s), 2.68(3H,s), 5.30(1H,s), 6.05(1H,bs), 7.00(1H,d,J=8Hz), 7.07-(1H,dd,J=8Hz,5Hz), 7.35(1H,t,J=8Hz), 7.53(1H,td,J=8Hz,2Hz), 7.62(1H,d,J=8Hz), 8.01(1H,d,J=8Hz), 8.15-(1H,t,J=2Hz), 8.53(1H,d,J=5Hz)

Example 63

Synthesis of methyl 1,4-dihydro-2,6-dimethyl-4-(3-methoxyphenyl)-5-(2-pyridyl)pyridine-3-carboxylate:

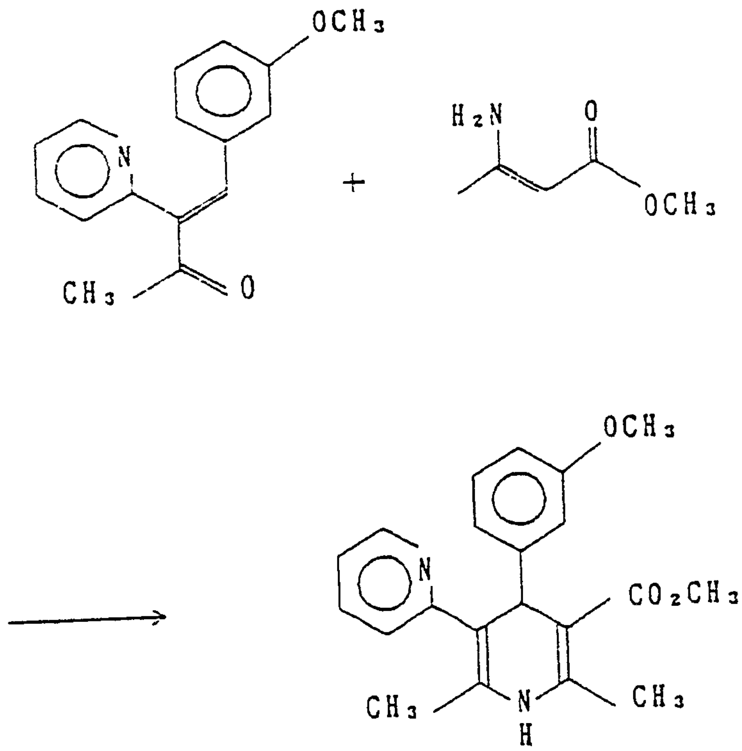

A mixuture of 268 mg (1 mmol) of 4-(3-methoxyphenyl)-3-(2-pyridyl)-3-butene-2-one, 575 mg (5 mmol) of methyl 3-aminocrotonate, 273 mg (2 mmol) of zinc chloride, and 500 mg of Molecular Shieves 4A was added to 1,4-dioxane. This reaction mixture was refluxed with application of heat in an inert atmoshpere for 5 hours. The reaction mixture was then cooled to room temperature and neutralized with a saturated aqueous solution of sodium hydrogencarbonate, followed by extraction with chloroform. The solvents contained in the extract were distilled off and the residue was chromatographed on a silica gel column, whereby the captioned compound was obtained. The yield was 210 mg (60%).
Melting point: 164.2 to 164.6 °C
IR ($cm^{-1}$, KBr) ; $\nu$ CO 1690

| Mass Analysis for $C_{21}H_{22}N_2O_3$ | |
|---|---|
| Calculated:<br>Found: | 350.16302<br>350.16082 |

NMR ($\delta$, $CDC\ell_3$); 2.07(3H,s), 2.35(3H,s), 3.63(3H,s), 3.71(3H,s), 5.02(1H,s), 5.45(1H,bs), 6.66-(1H,dd,J=8Hz,2Hz), 6.79(1H,t,J=2Hz), 6.83(1H,d,J=8Hz), 6.97(1H,d,J=8Hz), 7.03-(1H,ddd,J=8Hz,5Hz,1Hz), 7.11(1H,t,J=8Hz), 7.48(1H,td,J=8Hz,2Hz), 8.54(1H,d,J=5Hz)

**1. Test for blood platelet aggregation inhibitory activity of rabbit**

The compound to be tested was dissolved in a 50%HCO-60/ethanol solvent and the solution was diluted with physiological sodium chloride solution in such a fashion that the maximum concentration of the solvents contained therein did not exceed 5%, whereby a test solution for the compound was prepared.

A blood of a rabbit (Japanese white; male; 2.3 - 3.6 kg) was exsanguinated from a carotid of the rabbit, and nine parts of the blood were mixed with one part of a 3.8% aqueous solution of sodium citrate. The mixture was centrifuged under 200 xg at 20 °C for 15 minutes. The upper layer is a platelet rich plasma (PRP), and the lower layer was further centrifuged under 1500 xg at 20 °C for 10 minutes, so that a platelet poor plasma (PPP) was obtained.

10 $\mu l$ of the test solution was added to the 200 $\mu l$ of PRP, and the mixture was subjected to incubation at 37°C for 10 minutes. To this mixture was added 10 $\mu l$ of a sodium arachidonate solution (22 mM) for causing the aggregation. The extent of the aggregation was investigated by measuring the transparency of the test sample by Agricometer (NKK, PAT-4A). The blood platelet aggregation inhibitory activity of the test sample was obtained as a relative value with the transparency of PPP being 100%.

The results are shown in table below.

**2. Test for hypotensive activity**

Male spontaneously hypertensive rats (SHR) with arterial blood pressures higher than 140 mmHg were used for the experiments. Arterial blood pressure was measured through the catheterized artery with a pressure transducer (MPU-05 manufactured by Nihon Kohden Kabushiki Kaisha). The catheter was inserted into the abdominal aorta via the median coccygeal artery of SHR under light ether anesthesia. Heart rate (HR) was simultaneously recorded by a heart rate counter (AT-601G manufactured by Nihon Kohden) triggered by the blood pressure pulse. The recordings were made on a rectigraph (manufactured by Sanei Sokki Kabushik Kaisha). The compounds to be tested were administered at least two hours after the operation with the catheter, which had been previously inserted into the coccygeal vein. SHR were restrained with a wire mesh cage immediately after the operation.

The results are shown in table below.

T A B L E

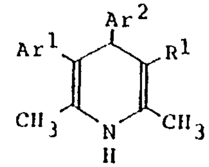

| Examples | $Ar^1$ | $Ar^2$ | $R^1$ | AA Control $10^{-4}$M(%) | SHR - ΔMAX β300 μg/kg |
|---|---|---|---|---|---|
| 1 | 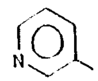 | 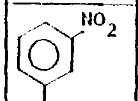 | $CO_2Me$ | 46.2 | 40 |
| 2 | 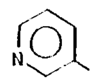 | 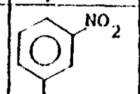 | $CO_2Et$ | 42.1 | 45 |
| 3 | 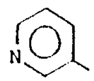 | 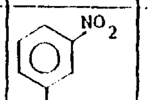 | $CO_2i$-Pr | 34.2 | 60 |
| 4 | 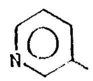 | 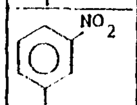 | $CO_2n$-Hex | 30.9 | 35 |
| 5 | 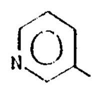 | 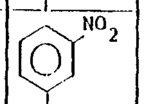 | $CO_2c$-Hex | 20.0 | 40 |
| 6 | 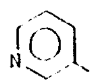 | 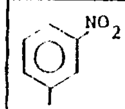 | 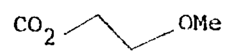 | 29.5 | 35 |
| 7 | 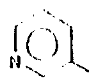 | 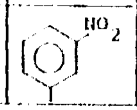 | 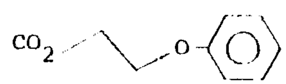 | 28.4 | 50 |

T A B L E (Cont'd)

| Examples | $Ar^1$ | $Ar^2$ | $R^1$ | AA Control $10^{-4}M(\%)$ | SHR - ΔMAX 300 μg/kg |
|---|---|---|---|---|---|
| 8 | 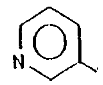 | 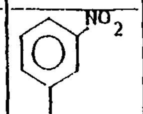 | 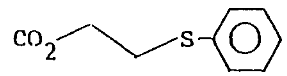 | 40.7 | 40 |
| 9 | 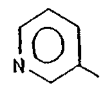 | 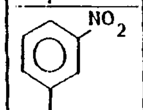 | $CO_2CH_2CF_3$ | 51.3 | 50 |
| 10 | 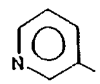 | 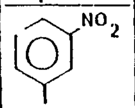 | 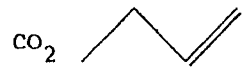 | 42.3 | 45 |
| 11 | 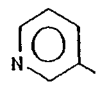 | 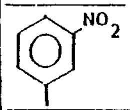 | 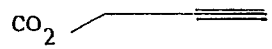 | 43.0 | 30 |
| 12 | 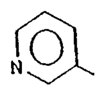 | 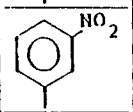 | 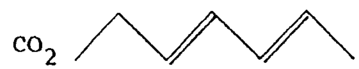 | 36.0 | 25 |
| 13 | 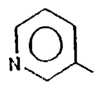 | 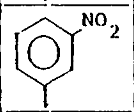 | 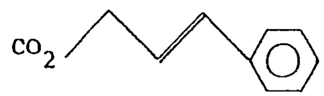 | 30.1 | 35 |
| 14 | 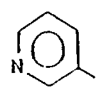 | 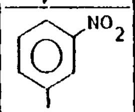 | 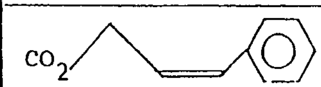 | 24.2 | 25 |
| 15 | 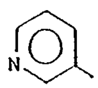 | 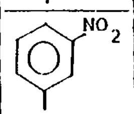 | 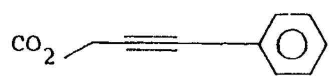 | 32.2 | 15 |
| 16 | 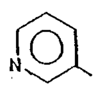 | 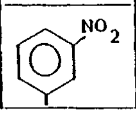 | 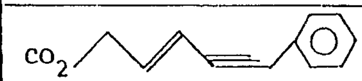 | 42.1 | 25 |

T A B L E (Cont'd)

| Examples | $Ar^1$ | $Ar^2$ | $R^1$ | AA Control $10^{-4}$M(%) | SHR - ΔMAX 300 μg/kg |
|---|---|---|---|---|---|
| 17 | 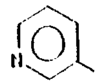 | 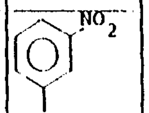 | 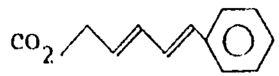 | 33.8 | 55 |
| 18 | 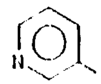 | 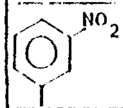 | 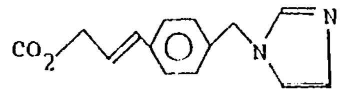 | 30.3 | 10 |
| 19 | 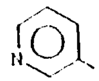 | 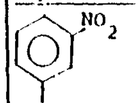 | 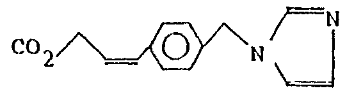 | 49.5 | 25 |
| 21 | 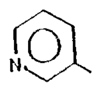 | 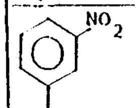 | 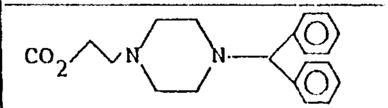 | 39.0 | 40 |
| 22 | 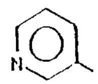 | 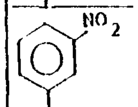 | 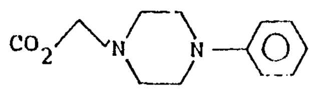 | 42.5 | 80 |
| 23 | 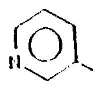 | 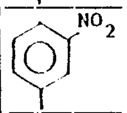 | 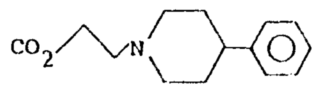 | 28.5 | 83 |
| 24 | 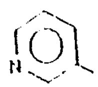 | 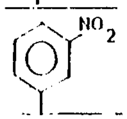 | 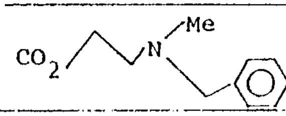 | 27.0 | 35 |
| 25 | 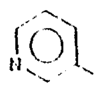 | 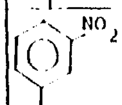 | 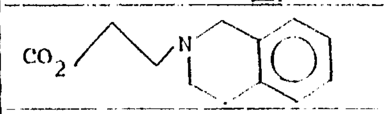 | 29.4 | 65 |
| 30 | 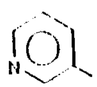 | 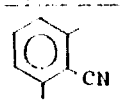 | $CO_2Me$ | 21.5 | 35 |

T A B L E (Cont'd)

| Examples | $Ar^1$ | $Ar^2$ | $R^1$ | AA Control $10^{-4}M$(%) | SHR - ΔMAX 300 μg/kg |
|---|---|---|---|---|---|
| 47 | 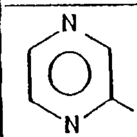 | 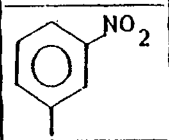 | $CO_2Me$ | 37.3 | 55 |
| 50 | 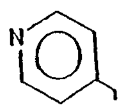 | 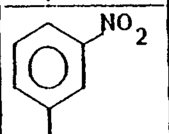 | 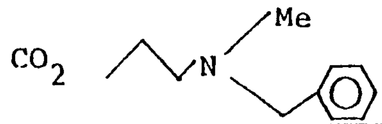 | 12.5 | 40 |
| 51 | 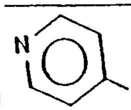 | 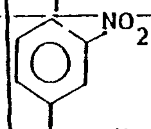 | 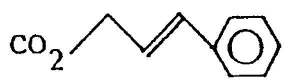 | 12.6 | 40 |
| 52 | 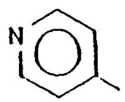 | 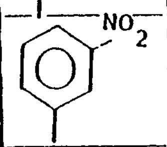 | 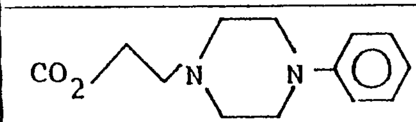 | 15.0 | 70 |
| 53 | 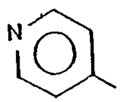 | 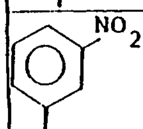 | 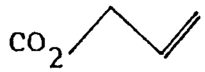 | 17.0 | 35 |
| 54 | 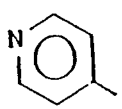 | 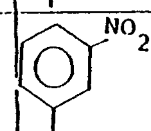 | 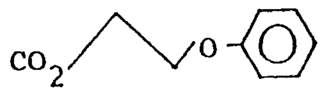 | 16.8 | 35 |
| 55 | 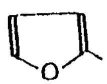 | 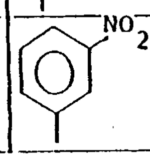 | 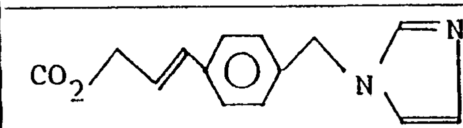 | 38.3 | 20 |

## Claims

1. 1,4-Dihydropyridine derivatives of formula (I):

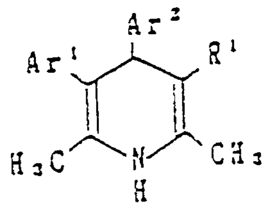

( I )

wherein $Ar^1$ and $Ar^2$ each represent an aromatic hydrocarbon group which may have a substitutent, or an aromatic heterocyclic group which may have a substituent; $R^1$ represents a group selected from the group consisting of $-CO_2R^2$, $-SO_2R^3$, $-COR^4$, $-CON(R^5)_2$, -CN or $-NO_2$; $R^2$ represents (i) hydrogen, (ii) a straight chain, branched chain or cyclic saturated hydrocarbon group having 2 to 10 carbon atoms, which may have a substituent selected from the group consisting of an alkyl group having 1 to 4 carbon atoms, an alkoxyl group having 1 to 4 carbon atoms, a phenoxy group which may have a substituent, a phenylthio group which may have a substituent, a substituted amino group, a cyclic amino group which may have a substituent, a phenyl group which may have a substituent, an aromatic heterocyclic group which may have a substituent, and a trihalomethyl group, or (iii) a straight chain, branched chain or cyclic unsaturated hydrocarbon group having 2 to 10 carbon atoms, which may have a substituent selected from the group consisting of an alkyl group having 1 to 4 carbon atoms, an alkoxyl group having 1 to 4 carbon atoms, a phenoxy group which may have a substituent, a phenylthio group which may have a substituent, a substituted amino group, a cyclic amino group which may have a substituent, a phenyl group which may have a substituent, an aromatic heterocyclic group which may have a substituent, and a trihalomethyl group; $R^3$ represents an alkyl group having 1 to 4 carbon atoms; $R^4$ represents an alkyl group having 1 to 4 carbon atoms, or a phenyl group; and $R^5$ represents an alkyl group having 1 to 4 carbon atoms.

2. The 1,4-dihydropyridine derivatives as claimed in Claim 1, wherein $Ar^1$ is an aromatic heterocyclic group which may have a substituent.

3. The 1,4-dihydropyridine derivatives as claimed in Claim 1, wherein said aromatic heterocyclic group represented by $Ar^1$ is selected from the group consisting of pyridyl group, quinolyl group, isoquinolyl group, furyl group, thienyl group, benzoxazolyl group, benzothiazolyl group, pyridazinyl group, pyrazinyl group, pyrimidinyl group, indolyl group, benzoxadiazolyl group, benzothiadiazolyl group, which may have a substituent.

4. The 1,4-dihydropyridine derivatives as claimed in Claim 3, wherein the substituent of the aromatic heterocyclic group represented by $Ar^1$ is selected from the group consisting of a halogen, cyano group, nitro group, trifluoromethyl group, trichloromethyl group, azide group, amide group, an alkyl group having 1 to 4 carbon atoms, an alkoxyl group having 1 to 4 carbon atoms, benzoyl group, an alkylthio group having 1 to 4 carbon atoms, phenylthio group, phenoxy group, a lower alkoxycarbonyl group, a lower acyl group, benzyloxy group, hydroxy group, and cinnamyloxy group.

5. The 1,4-dihydropyridine derivatives as claimed in Claim 1, wherein $Ar^1$ and $Ar^2$ each represent an aromatic hydrocarbon group selected from the group consisting of phenyl group and naphthyl group, which may have a substituent.

6. The 1,4-dihydropyridine derivatives as claimed in Claim 1, wherein $Ar^1$ and $Ar^2$ each represent an aromatic heterocyclic group selected from the group consisting of pyridyl group, quinolyl group, isoquinolyl group, furyl group, thienyl group, benzoxazolyl group, benzothiazolyl group, pyridazinyl group, pyrazinyl group, pyrimidinyl group, indolyl group, benzoxadiazolyl group, benzothiadiazolyl group, which may have a substituent.

7. The 1,4-dihydropyridine derivatives as claimed in Claim 5, wherein the substituent of the aromatic hydrocarbon group represented by either $Ar^1$ or $Ar^2$ is selected from the group consisting of a halogen, cyano group, nitro group, trifluoromethyl group, trichloromethyl group, azide group, amide group, an alkyl group having 1 to 4 carbon atoms, an alkoxyl group having 1 to 4 carbon atoms, a benzoyl group, an alkylthio group having 1 to 4 carbon atoms, phenylthio group, phenoxy group, a lower alkoxycarbonyl group, a lower acyl group, benzyloxy group, hydroxy group, and cinnamyloxy group.

8. The 1,4-dihydropyridine derivatives as claimed in Claim 6, wherein the substituent of the aromatic heterocyclic group represented by either $Ar^1$ or $Ar^2$ is selected from the group consisting of a halogen, cyano group, nitro group, trifluoromethyl group, trichloromethyl group, azide group, amide group, an alkyl group having 1 to 4 carbon atoms, an alkoxyl group having 1 to 4 carbon atoms, benzoyl group, an alkylthio group having 1 to 4 carbon atoms, phenylthio group, phenoxy group, a lower alkoxycarbonyl group, a lower acyl group, benzyloxy group, hydroxy group, and cinnamyloxy group.

9. The 1,4-dihydropyridine derivatives as claimed in Claim 1, wherein $Ar^1$ and $Ar^2$ are selected from the group consisting of phenyl group, 2-nitrophenyl group, 3-nitrophenyl group, 4-nitrophenyl group, 2-cyanophenyl group, 2-bromophenyl group, 3-bromophenyl group, 4-bromophenyl group, 2-chlorophenyl group, 3-chlorophenyl group, 4-chlorophenyl group, 2-iodophenyl group, 3-iodophenyl group, 4-iodophenyl group, 3-cyanophenyl group, 4-cyanophenyl group, 2,3-dichlorophenyl group, 2,6-dichlorophenyl group, 3,5-dichlorophenyl group, 2-furyl group, furyl group, thienyl group, 3-thienyl group, 1-naphthyl group, naphthyl group, 3-azidophenyl group, 2-trifluoromethylphenyl group, 3-trifluoromethylphenyl group, 4-trifluoromethylphenyl group, 2-methylphenyl group, 3-methylphenyl group, 4-methylphenyl group, 2-hydroxyphenyl group, 3-hydroxyphenyl group, 4-hydroxyphenyl group, 2-benzyloxyphenyl group, 3-benzyloxyphenyl group, 2-cinnamyloxyphenyl group, 3-cinnamyloxyphenyl group, 3-benzoylphenyl group, 2-methoxyphenyl group, 3-methoxyphenyl group, 4-methoxyphenyl group, 4-methylthiophenyl group, 3-trichloromethylphenyl group, 2-pyridyl group, pyridyl group, 4-pyridyl group, quinolyl group, 3-quinolyl group, 4-quinolyl group, 1-isoquinolyl group, quinoxalyl group, 2-chloropyridyl group, 6-chloropyridyl group, 2-methylpyridyl group, 6-methylpyridyl group, 2-methoxypyridyl group, 6-methoxypyridyl group, 5-bromopyridyl group, 2-amionpyridyl group, 2-mercaptopyridyl group, pyridazinyl group, 4-pyridazinyl group, pyrazinyl group, pyrimidinyl group, 5-pyrimidinyl group, 7-benzoxazolyl group, 7-benzothiazolyl group, 3-benzoxadiazolyl group, 3-benzothiazolyl group, 2-indolyl group, 3-indolyl group, and 5-indolyl group.

10. The 1,4-dihydropyridine derivatives as claimed in Claim 1, wherein $R^1$ is $-CO_2R^2$, in which $R^2$ is hydrogen.

11. The 1,4-dihydropyridine derivatives as claimed in Claim 1, wherein $R^1$ is $-CO_2R^2$, in which $R^2$ is a straight chain or branched saturated hydrocarbon group selected from the group consisting of methyl group, ethyl group, n-propyl group, n-butyl group, n-pentyl pentyl group, n-hexyl group, n-heptyl group, n-octyl group, n-nonyl group, n-decyl group, isoproyl group, and isobutyl group, which may have a substituent.

12. The 1,4-dihydropyridine derivatives as claimed in Claim 1, wherein $R^1$ is $-CO_2R^2$, in which $R^1$ is a cyclic hydrocarbon group selected from the group consisting of cyclopentyl group and cyclohexyl group, which may have a substituent.

13. The 1,4-dihydropyridine derivatives as claimed in Claim 1, wherein $R^1$ is $-CO_2R^2$, in which $R^2$ is an unsaturated hydrocarbon group selected from the group consisting of propenyl group, 2-butenyl group, 3-butenyl group, 2-pentenyl group, 2,4-hexadienyl group, 2,4-hexadiynyl group, hexa-4-en-2-yne, which may have a substituent.

14. The 1,4-dihydropyridine derivatives as claimed in Claim 11, wherein the substituent of $R^2$ is selected from the group consisting of an alkoxyl group having 1 to 6 carbon atoms, an unsubstituted or substituted phenoxy group, an unsubstituted or substituted phenylthio group, a substituted amino group, cyclic amino group, an unsubstituted or substituted phenyl group, an unsubstituted or substituted pyridyl group, an aromatic heterocyclic group, and a trihalomethyl group.

15. The 1,4-dihydropyridine derivatives as claimed in Claim 12, wherein the substituent of $R^2$ is selected from the group consisting of an alkyl group having 1 to 6 carbon atom, an alkyl group having 1 to 6 carbon atoms, an unsubstituted or substituted phenoxy group, an unsubstituted or substituted phenylthio group, a substituted amino group, a cyclic amino group, an unsubstituted or substituted phenyl group, an unsubstituted or substituted pyridyl group, an aromatic heterocyclic group, and a trihalomethyl group.

16. The 1,4-dihydropyridine derivatives as claimed in Claim 13, wherein the substituent of $R^2$ is selected from the group consisting of an alkyl group having 1 to 6 carbon atom, an alkyl group having 1 to 6 carbon atoms, an unsubstituted or substituted phenoxy group, an unsubstituted or substituted phenylthio group, a substituted amino group, cyclic amino group, an unsubstituted or substituted phenyl group, an unsubstituted or substituted pyridyl group, an aromatic heterocyclic group, and a trihalomethyl group.

17. The 1,4-dihydropyridine derivatives as claimed in Claim 14, wherein said alkoxyl group is selected from the group consisting of methoxy group, ethoxy group and propoxy group.

18. The 1,4-dihydropyridine derivatives as claimed in Claim 14, wherein said substituted amino group is selected from the group consisting of dimethy amino group, diethyl amino group, and N-benzyl-N-methylamino group.

19. The 1,4-dihydropyridine derivatives as claimed in Claim 14, wherein said cyclic amino group is

selected from the group consisting of an unsubstituted or substituted piperazinyl group and piperidinyl group.

20. The 1,4-dihydropyridine derivatives as claimed in Claim 14, wherein said aromatic heterocyclic group is selected from the group consisting of pyridyl group, quinolyl group, isoquinolyl group, furyl group, thienyl group, benzoxazolyl group, benzothiazolyl group, pyridazinyl group, pyrazinyl group, pyrimidinyl group, indolyl group, benzoxadiazolyl group, and benzothiadiazolyl group.

21. The 1,4-dihydropyridine derivatives as claimed in Claim 15, wherein said alkyl group is selected from the group consisting of methyl group, ethyl group, propyl group, and isopropyl group.

22. The 1,4-dihydropyridine derivatives as claimed in Claim 15, wherein said alkoxyl group is selected from the group consisting of methoxy group, ethoxy group and propoxy group.

23. The 1,4-dihydropyridine derivatives as claimed in Claim 15, wherein said substituted amino group is selected from the group consisting of dimethy amino group, diethyl amino group, and N-benzyl-N-methylamino group.

24. The 1,4-dihydropyridine derivatives as claimed in Claim 15, wherein said cyclic amino group is selected from the group consisting of an unsubstituted or substituted piperazinyl group and piperidinyl group.

25. The 1,4-dihydropyridine derivatives as claimed in Claim 15, wherein said aromatic heterocyclic group is selected from the group consisting of pyridyl group, quinolyl group, isoquinolyl group, furyl group, thienyl group, benzoxazolyl group, benzothiazolyl group, pyridazinyl group, pyrazinyl group, pyrimidinyl group, indolyl group, benzoxadiazolyl group, and benzothiadiazolyl group.

26. The 1,4-dihydropyridine derivatives as claimed in Claim 16, wherein said alkyl group is selected from the group consisting of methyl group, ethyl group, propyl group, and isopropyl group.

27. The 1,4-dihydropyridine derivatives as claimed in Claim 16, wherein said alkoxyl group is selected from the group consisting of methoxy group, ethoxy group and propoxy group.

28. The 1,4-dihydropyridine derivatives as claimed in Claim 16, wherein said substituted amino group is selected from the group consisting of dimethy amino group, diethyl amino group, and N-benzyl-N-methylamino group.

29. The 1,4-dihydropyridine derivatives as claimed in Claim 16, wherein said cyclic amino group is selected from the group consisting of an unsubstituted or substituted piperazinyl group and piperidinyl group.

30. The 1,4-dihydropyridine derivatives as claimed in Claim 16, wherein said aromatic heterocyclic group is selected from the group consisting of pyridyl group, quinolyl group, isoquinolyl group, furyl group, thienyl group, benzoxazolyl group, benzothiazolyl group, pyridazinyl group, pyrazinyl group, pyrimidinyl group, indolyl group, benzoxadiazolyl group, and benzothiadiazolyl group.

31. The 1,4-dihydropyridine derivatives as claimed in Claim 1, wherein $R^1$ is $-CO_2R^2$, in which $R^2$ is selected from the group consisting of hydrogen, methyl group, ethyl group, n-propyl group, n-butyl group, n-pentyl group, n-hexyl group, n-heptyl group, n-octyl group, n-nonyl group, n-decyl group, isopropyl group, isobutyl group, cyclopentyl group, cyclohexyl group, 2-propene-1-yl group, 2-propyne-1-yl group, (E)-2-butene-1-yl group, (E)-3-butene-1-yl group, (E)-2-pentene-1-yl group, (2E,4E)-2,4-hexadienyl group, 2,4-hexadiynyl group, (E)-hexa-4-en-2-yne group, (E)-3-phenyl-2-propene-1-yl group, (Z)-3-phenyl-2-propyne-1-yl group, 3-phenyl-2-propyne-1-yl group, (2E,4E)-5-phenyl-2,4-pentadiene-1-yl group, 5-phenyl-penta-2,4-diyne-1-yl group, (E)-5-phenyl-penta-2-en-4-yne-1-yl group, (E)-3-[4-(1-imidazolylmethyl)phenyl]-2-propene-1-yl group, (E)-3-[3-(1-imidazolylmethyl)phenyl]-2-propene-1-yl group, (E)-3-[2-(1-imidazolylmethyl)phenyl]-2-propene-1-yl group, (Z)-3-[4-(1-imidazolylmethyl)phenyl]-2-propene-1-yl group, (E)-3-[6-(1-imidazolylmethyl)pyridine-2-yl]-2-propene-1-yl group, (E)-3-[5-(1-imidazolylmethyl)furan-2-yl]-2-propene-1-yl group, (E)-3-[5-(1-imidazolylmethyl)thiophene-2-yl]-2-propene-1-yl group, (E)-3-phenyl-1-methyl-2-propene-1-yl group, (E)-2-fluoro-3-phenyl-2-propene-1-yl group, 2-methoxyethyl group, 3-methoxypropyl group, 3-ethoxypropyl group, 2-phenoxyethyl group, 2-phenylthioethyl group, 2-(N-methylamino)ethyl group, 2-(N,N-dimethylamino)ethyl group, 2-(N-methyl-N-phenylamino)ethyl group, 2-(N,N-diethylamino)ethyl group, 2-(N-benzyl-N-methylamino)ethyl group, 2-(1-piperazinyl)ethyl group, 4-(1-piperazinyl)butyl group, 6-(1-piperazinyl)hexyl group, 2-(1-piperidinyl)ethyl group, 2-(4-phenylpiperazine-1-yl) ethyl group, 3-(4-phenylpiperazine-1-yl)propyl group, 4-(4-phenylpiperazine-1-yl)butyl group, 6-(4-phenylpiperadine-1-yl)hexyl group, 2-(4-phenylpiperidine-1-yl)ethyl group, 3-(4-phenylpiperidine-1-yl)propyl group, 4-(4-phenylpiperidine-1-yl)butyl group, 4-(4-phenylpiperidine-1-yl)butyl group, 6-(4-phenylpiperidine-1-yl)hexyl group, 2-[4-(diphenylmethyl)piperazine-1-yl]ethyl group, 3-[4-(diphenylmethyl)piperazine-1-yl)propyl group, 4-[4-(diphenylmethyl)piperazine-1-yl)]butyl group, 6-[4-(diphenylmethyl)piperazine-1-yl)]hexyl group, 2-morpholinoethyl group, N-benzylpyrrolidine-3-yl group, N-benzylpiperidine-3-yl group, 2-(1,2,3,4-tetrahydroisoquinoline-2-yl)ethyl group, 2,2,2-trifluoroethyl group, 2-(3,7-dihydro-3,7-dimethyl-1H-purine-2,6-dione-1-yl)ethyl group, and 2-(1,2,3,6-tetrahydro-1,3-dimethyl-2,6-dioxo-7H-purine-7-yl)ethyl group.

32. The 1,4-dihydropyridine derivatives as claimed in Claim 1, wherein $R^1$ is $-SO_2R^3$, in which $R^3$ is an alkyl group having 1 to 4 carbon atoms.

33. The 1,4-dihydropyridine derivatives as claimed in Claim 32, wherein the alkyl group represented by $R^3$ is selected from the group consisting of methyl group, ethyl group, n-propyl group, and isopropyl group.

34. The 1,4-dihydropyridine derivatives as claimed in Claim 1, wherein $R^1$ is $-COR^4$, in which $R^4$ is an alkyl group having 1 to 4 carbon atoms.

35. The 1,4-dihydropyridine derivatives as claimed in Claim 34, wherein the alkyl group represented by $R^4$ is selected from the group consisting of methyl group, ethyl group, n-propyl group, and isopropyl group.

36. The 1,4-dihydropyridine derivatives as claimed in Claim 1, wherein $R^1$ is $-COR^4$, in which $R^4$ is a phenyl group.

37. The 1,4-dihydropyridine derivatives as claimed in Claim 1, wherein $R^1$ is $-CON(R^5)_2$, in which $R^5$ is an alkyl group having 1 to 4 carbon atoms.

38. The 1,4-dihydropyridine derivatives as claimed in Claim 37, wherein the alkyl group represented by $R^5$ is selected from the group consisting of methyl group, ethyl group, n-propyl group, and isopropyl group.

European Patent Office

# EUROPEAN SEARCH REPORT

Application number

EP 90110397.8

## DOCUMENTS CONSIDERED TO BE RELEVANT

| Category | Citation of document with indication, where appropriate, of relevant passages | Relevant to claim | CLASSIFICATION OF THE APPLICATION (Int Cl.5) |
|---|---|---|---|
| X | DE - A1 - 3 501 855 (BAYER) * Totality * -- | 1 | C 07 D 401/04<br>C 07 D 405/04<br>C 07 D 401/14<br>C 07 D 401/10<br>C 07 D 401/12 |
| P,X | EP - A2 - 0 325 130 (BAYER) * Claim 1; examples 52,53 * ---- | 1 | |

| TECHNICAL FIELDS SEARCHED (Int Cl.5) |
|---|
| C 07 D 401/00<br>C 07 D 405/00 |

The present search report has been drawn up for all claims

| Place of search | Date of completion of the search | Examiner |
|---|---|---|
| VIENNA | 27-08-1990 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document